(19) Europäisches Patentamt
European Patent Office
Office européen des brevets

(11) EP 0 701 554 B1

(12) **FASCICULE DE BREVET EUROPEEN**

(45) Date de publication et mention de la délivrance du brevet: **27.06.2001 Bulletin 2001/26**

(21) Numéro de dépôt: **95916723.0**

(22) Date de dépôt: **07.04.1995**

(51) Int Cl.[7]: **C07D 293/12**, C07D 293/10, C07D 421/12, A61K 31/41

(86) Numéro de dépôt international: **PCT/FR95/00447**

(87) Numéro de publication internationale: **WO 95/27706 (19.10.1995 Gazette 1995/45)**

(54) **NOUVEAUX COMPOSES DE STRUCTURE BENZISOSELEN-AZOLIN ET -AZINE, LEUR PROCEDE DE PREPARATION ET LEURS APPLICATIONS THERAPEUTIQUES**

BENZISOSELEN-AZOLINE UND-AZINE-DERIVATE, VERFAHREN ZU DEREN HERSTELLUNG UND IHRE THERAPEUTISCHE VERWENDUNG

NOVEL COMPOUNDS HAVING A BENZISOSELEN-AZOLINE AND -AZINE STRUCTURE, METHOD FOR PREPARING SAME AND THERAPEUTIC USES THEREOF

(84) Etats contractants désignés: **BE CH DE DK ES FR GB IT LI LU NL PT**

(30) Priorité: **07.04.1994 FR 9404107**

(43) Date de publication de la demande: **20.03.1996 Bulletin 1996/12**

(73) Titulaire: **OXIS Isle of Man, Limited Douglas, Isle of Man IM1 (GB)**

(72) Inventeurs:
- **ERDELMEIER, Irène F-75013 Paris (FR)**
- **CHAUDIERE, Jean F-94100 Saint-Maur-des-Fosses (FR)**
- **MOUTET, Marc F-92220 Bagneux (FR)**
- **YADAN, Jean-Claude F-75011 Paris (FR)**

(74) Mandataire: **Portal, Gérard et al Cabinet Beau de Loménie 158, rue de l'Université 75340 Paris Cédex 07 (FR)**

(56) Documents cités:
**DE-A- 3 027 074** **DE-A- 3 444 135**
**US-A- 4 711 961** **US-A- 5 246 951**

- **Progress in Drug Research, vol. 36(9), 1991**
- **Tetrahedron Letters vol. 33(27). pp. 3863-66, 1992**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 701 554 B1

## Description

La présente invention concerne l'utilisation de nouveaux composés de structure benzisosélénazoline gem-disubstitués en position 3 et benzisosélénazine gem-disubstitués en position 4 comme agents antioxydants ainsi que leur procédé de préparation et des compositions pharmaceutiques en comportant application.

**ETAT DE L'ART ANTERIEUR:**

La plupart des tissus et cellules de mammifères possèdent des enzymes dénommées glutathion peroxydases, qui leur permettent de dégrader les hydroperoxydes cytotoxiques endogènes ou exogènes.
Ces enzymes antioxydantes et cytoprotectrices jouent un rôle central dans la prévention du "stress oxydant" et de ses conséquences délétères.
Elles catalysent la réduction du peroxyde d'hydrogène ( réaction 1) ou celle des hydroperoxydes organiques ( réaction 2) par le glutathion réduit (GSH):

$$H_2O_2 + 2GSH \rightarrow 2H_2O + GSSG \qquad \text{réaction 1}$$

$$ROOH + 2GSH \rightarrow ROH + H_2O + GSSG \qquad \text{réaction 2}$$

Les glutathion peroxydases sont des enzymes à sélénium dont trois sous-familles ont été décrites:

* celle des enzymes intra-cellulaires, hydrosolubles et possèdant une structure de tétramère symètrique (voir L. Flohé; "Structures and Catalytic Mechanism of Glutathione Peroxidase" ; in Glutathione Centennial; (1989); N. Taniguchi, T. Higashi, Y. Sakamoto and A. Meister Eds.; Academic Press; pages 103-114).
* celle des enzymes intra-cellulaires, partiellement liées aux membranes et possèdant une structure monomèrique (voir F. Ursini et coll.; Biochem. Biophys Acta; (1982); 710; pages197-211).
* et enfin, la glutathion peroxydase spécifique du plasma sanguin, enzyme tétramèrique dont l'extrémité N-terminale est spécifiquement glycosylée (voir K. Takahashi et coll.; J. Biochem.; (1990); 108; pages 145-148).

Les sites actifs de ces enzymes possèdent tous un atome de sélénium essentiel; sous la forme d'un résidu sélénocystéine incorporé dans la chaîne polypeptidique.
Le caractère essentiel du sélénium au site actif est bien documenté (voir J.W. Forstrom et coll.; Biochemistry; (1978); 17; 2639-2644 et A. Wendel et coll.; Hoppe-Seyler's Z. Physiol. Chem.; (1978); 359; pages 1035-1036).
Dans les situations de carence alimentaire en sélénium, les concentrations et activités de ces glutathion peroxydases chutent progressivement (voir Y.X. Wang and J. Kiem.; Biological Trace Elements Res.; (1988); 15; page 89).
Par ailleurs, des expériences de mutagénèse dirigée ont montré que le remplacement d'un atome de sélénium du site actif par un atome de soufre entraînait une chute majeure de l'activité catalytique (voir C. Rocher et coll.; Eur. J.Biochem.; (1992); 205; pages 955-960).
Chez l'animal et chez l'homme, les sels de sélénite ou de sélénate et la L-sélénométhionine constituent trois formes naturelles d'apport en sélénium.
L'apport alimentaire en sélénium est un facteur limitant de la biosynthèse des glutathion peroxydases, mais l'augmentation de l'activité glutathion peroxydase avec cet apport en sélénium suit une courbe de saturation rapide. Au delà du plateau de saturation, une augmentation de l'apport alimentaire en sélénium se traduit par une toxicité marquée (voir O.A.Levander; Ann.Rev.Nutr.; (1987); 7; pages 227-250). L'intervalle entre les quantités requises de sélénium d'origine naturelle et leur seuil de toxicité est donc étroit.
A l'inverse, des expériences de microinjection intra-cellulaire de l'enzyme érythrocytaire ont démontré son effet protecteur trés marqué sur la viabilité de fibroblastes ou de cellules endothéliales exposées à un stress oxydant (voir C. Michiels et coll.; Experiment. Cell Res.; (1988); 179; pages 581-589).
L'utilisation d'une glutathion peroxydase d'origine naturelle dans un but thérapeutique est cependant difficile à envisager pour les raisons suivantes:

* la stabilité des enzymes purifiées est insuffisante;
* il n'existe pas de méthode efficace pour assurer leur ciblage intra-cellulaire;
* elles ne peuvent être administrées par voie orale.

Afin d'éliminer ces difficultés, un certain nombre de composés organoséléniés de faible poids moléculaire ont été synthétisés.

Les propriétés biochimiques et pharmacologiques des composés organoséléniés qui ont été synthétisés et étudiés ont été revues récemment (voir M.J. Parnham et E. Graf; Progress in Drug Res.; (1991); 36; pages 9-47).

Il est également connu par Tetrahedron Letters, Vol. 33 No. 27, pages 3863-3866, 1992, la synthèse de 2H-3-3,4-dihydro-1,2-benzoselenazin-3-on et de ses dérivés, ayant un hétérocycle à six membres et dont l'activité antioxydante n'est pas meilleure que celle des composés dont l'hétérocycle est à cinq membres tel que décrit dans le document Parnham de 1991 précité.

Les composés organoséléniés présentant une activité glutathion peroxydase produisent généralement des intermédiaires catalytiques du type selenol et/ou disélénure.

Parmi ces composés, le 2-phényl-3-one-benzisosélénazoline(2H) et certains de ses dérivés ne semblent pas avoir d'effet toxique majeur (voir A. Wendel et coll.; Biochem. Pharmacol.; (1984); 33; pages 3241-3245 et S.D. Mercurio et G.F. Combs; Biochem. Pharmacol.; (1986); 35; pages 4505-4509).

En présence d'un excès de glutathion GSH, le 2-phényl-3-one-benzisosélénazoline(2H) fournit cependant un dérivé trés peu soluble dans l'eau, ce qui limite ses applications pharmacologiques.

La toxicité des composés organoséléniés de type sélénols et/ou disélénures est en grande partie due à la réduction catalytique de l'oxygène en superoxyde et peroxyde d'hydrogène (voir J.CHAUDIERE et coll.; Arch.Biochem. Biophys.; (1992); 296; pages 328-336).

L'un des buts de la présente invention est de concevoir des composés organoséléniés possédant une activité catalytique de type glutathion peroxydase en présence de concentrations physiologiques de glutathion GSH.

Ces composés doivent pouvoir pénétrer à l'intérieur des tissus ou cellules cibles, être solubles dans l'eau aux concentrations actives et ne pas réduire efficacement l'oxygène en sous-produits toxiques.

Ces buts sont atteints grâce à l'invention qui porte sur la conception de nouveaux dérivés benzisosélén-azoline et -azine dont les activités antioxydantes et cytoprotectrices ainsi que leur procédé de préparation sont décrits.

Sur le plan chimique, un certain nombre de dérivés benzisosélénazolone ont été décrits- dans la littérature. D'une manière générale, l'accès à la plupart de ces dérivés a été réalisé essentiellement par réaction entre un dérivé aromatique lithié, généré soit par échange halogène-métal soit par déprotonation à l'aide d'une base forte, et du sélénium métal (Se°) (voir C.LAMBERT et coll.; Synthetic Comm.; (1991); 21; pages 85-98).

L'un des buts de l'invention décrite dans ce brevet est de proposer une nouvelle méthode d'introduction du sélénium sur un noyau benzènique halogéné, substitué en position 2, par réaction avec un dérivé sélénié nucléophile tel qu'un sel de sélénocyanate comme par exemple le sélénocyanate de potassium, éventuellement généré in situ, en présence de sel cuivreux ($Cu^I$).

**DESCRIPTION DE L'INVENTION:**

La présente invention a pour but:

1) de résoudre le nouveau problème technique consistant en la fourniture de nouveaux dérivés benzisosélén-azoline et -azine ayant une très bonne activité antioxydante et cytoprotectrice, permettant ainsi de constituer un principe actif précieux dans le cadre de compositions pharmaceutiques;
2) de résoudre le nouveau problème technique, énoncé ci-dessus, selon une solution fournissant également un procédé de préparation de ces composés aisé à mettre en oeuvre;

Le problème technique, énoncé ci-dessus, est résolu pour la première fois, d'une manière simultanée par la présente invention selon une solution simple, avec un procédé de préparation relativement aisé à mettre en oeuvre et fournissant de bons rendements.

Selon un premier aspect, la présente invention fournit de nouveaux composés benzisosélén-azoline et -azine répondant à la formule générale (II) suivante:

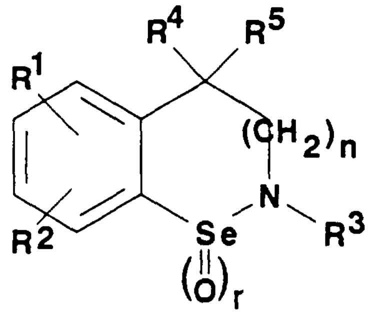

**Formule Générale II**

dans laquelle:

$R^1$ = hydrogène; alkyle inférieur; $-OR^6$; $-(CH_2)_mNR^6R^7$; $-(CH_2)_qNH_2$; $-(CH_2)_mNHSO_2(CH_2)_2NH_2$; $-NO_2$; -CN; $-SO_3H$; $-N^+(R^5)_2O^-$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^2$ = hydrogène; alkyle inférieur; $-OR^6$; $-(CH_2)_mNR^6R^7$; $-(CH_2)_qNH_2$; $-(CH_2)_mNHSO_2(CH_2)_2NH_2$; $-NO_2$; -CN; $-SO_3H$; $-N^+(R^5)_2O^-$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^3$ = hydrogène; alkyle inférieur; aralkyle; aralkyle substitué; $-(CH_2)_mCOR^8$; $-(CH_2)_qR^8$; $-CO(CH_2)_pCOR^8$; $-(CH_2)_mSO_2R^8$; $-(CH_2)_mS(O)R^8$;

$R^4$ = alkyle inférieur; aralkyle; aralkyle substitué; $-(CH_2)_pCOR^8$; $-(CH_2)_pR^8$; F;

$R^5$ = alkyle inférieur; aralkyle; aralkyle substitué;

$R^6$ = alkyle inférieur; aralkyle; aralkyle substitué; $-(CH_2)_mCOR^8$; $-(CH_2)_qR^8$;

$R^7$ = alkyle inférieur; aralkyle; aralkyle substitué; $-(CH_2)_mCOR^8$;

$R^8$ = alkyle inférieur; aralkyle; aralkyle substitué; aryle; aryle substitué; hétéroaryle; hétéroaryle substitué; hydroxyle; alkoxyle inférieur; $R^9$;

$R^9$ =

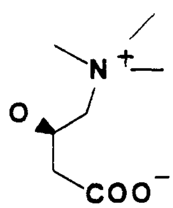

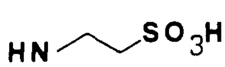

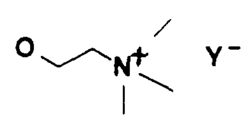

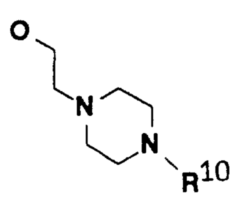

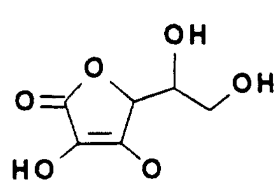

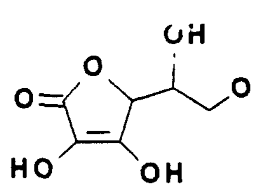

$R^{10}$ = hydrogène; alkyle inférieur; aralkyle ou aralkyle substitué; aryle ou aryle substitué;

$Y^-$ représente l'anion d'un acide pharmaceutiquement acceptable;
n = 0, 1;
m = 0, 1, 2;
p = 1, 2, 3;
q = 2, 3, 4;
r = 0, 1.

et leurs sels d'acides ou de bases pharmaceutiquement acceptables;
sachant qu'il ne peut y avoir au plus qu'un seul substituant $R^9$ au sein de chaque molécule répondant à la formule générale II.

Dans le cadre de la description et des revendications:

- par groupement alkyle ou alkoxyle inférieur, on entend des groupements linéaires ou ramifiés comprenant 1 à 6 atomes de carbone;
- le terme hétéroaryle signifie tout noyau aromatique mono ou bicyclique, chaque cycle comprenant de 5 à 6 sommets et incluant dans son squelette carboné un ou éventuellement plusieurs hétéroatomes identiques ou différents choisis parmi azote, oxygène ou soufre, et éventuellement substitué;
- le ternie substitué, affectant les groupements aryle, aralkyle ou hétéroaryle signifie que ceux-ci sont substitués sur la partie aromatique par un ou plusieurs groupements identiques ou différents choisis parmi alkyle inférieur, trifluorométhyle, alkoxyle inférieur, hydroxyle, nitro, amino, alkylamino; dialkylamino; sulfonyle; sulfonamide; sulfoalkyle; carboxyle; carbalkoxyle ou par un ou plusieurs atomes d'hydrogène;
- lorsque $R^1$ représente -COOH, $-SO_3H$, l'invention couvre également les sels d'addition à une base pharmaceutiquement acceptable;
- lorsque $R^1$ représente $-NR^6R^7$, l'invention couvre également les sels d'addition à un acide pharmaceutiquement acceptable;
- lorsque $R^8$ représente OH, l'invention couvre également les sels d'addition à une base pharmaceutiquement acceptable.

Parmi les acides pharmaceutiquement acceptables, on peut citer, à titre non limitatif, les acides chlorhydrique, bromhydrique, iodhydrique, sulfurique, tartrique, méthane-sulfonique, trifluorométhane-sulfonique, etc...

Parmi les bases pharmaceutiquement acceptables, on peut citer, à titre non limitatif, les hydroxydes de sodium, de potassium, les carbonates de métaux alcalins ou alcalino-terreux ou des bases organiques comme la triéthylamine ou l'arginine, etc...

Selon un deuxième aspect, la présente invention concerne l'utilisation de composés de type benzisosélénazoline et -azine répondant à la formule générale (I) suivante:

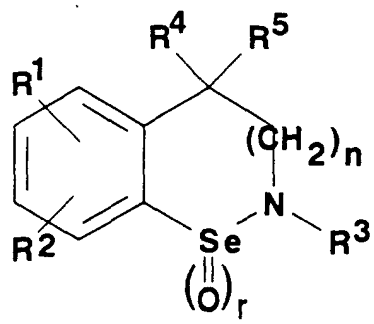

Formule Générale I

dans laquelle:

$R^1$ = hydrogène; alkyle inférieur; $-OR^6$; $-(CH_2)_mNR^6R^7$; $-(CH_2)_qNH_2$; $-(CH_2)_mNHSO_2(CH_2)_2NH_2$; -CN; $-SO_3H$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^2$ = hydrogène: alkyle inférieur; $-OR^6$; $-(CH_2)_mNR^6R^7$: $-(CH_2)_qNH_2$; $-(CH_2)_mNHSO_2(CH_2)_2NH_2$; -CN; $-SO_3H$; F: Cl: Br; 1; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^3$ = hydrogène; alkyle inférieur; aralkyle; aralkyle substitué; $-(CH_2)_mCOR^8$; $-(CH_2)_qR^8$; $-CO(CH_2)_pCOR^8$; $-(CH_2)_mSO_2R^8$; $-(CH_2)_mS(O)R^8$;

$R^4$ = alkyle inférieur; aralkyle; aralkyle substitué; $-(CH_2)_pCOR^8$; $-(CH_2)_pR^8$; F;

$R^5$ = alkyle inférieur; aralkyle; aralkyle substitué;

$R^6$ = alkyle inférieur; aralkyle; aralkyle substitué; $-(CH_2)_mCOR^8$; $-(CH_2)_qR^8$;

$R^7$ = alkyle inférieur; aralkyle; aralkyle substitué; $-(CH_2)_mCOR^8$;

$R^8$ = alkyle inférieur; aralkyle; aralkyle substitué; aryle; aryle substitué; hétéroaryle; hétéroaryle substitué; hydroxyle; alkoxyle inférieur; $R^9$;

$R^9$ =

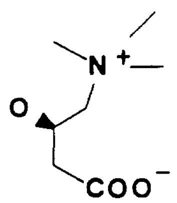

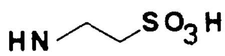

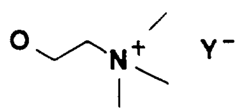

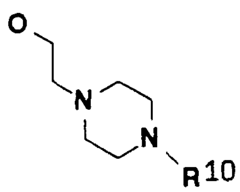

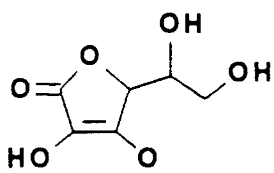

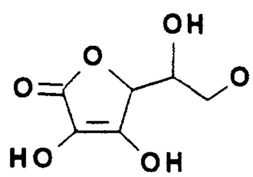

$R^{10}$ = hydrogène; alkyle inférieur; aralkyle ou aralkyle substitué; aryle ou aryle substitué;

$Y^-$ représente l'anion d'un acide pharmaceutiquement acceptable;
n = 0, 1;
m = 0, 1, 2;
p = 1, 2, 3;
q = 2, 3, 4;
r = 0,1.

et leurs sels d'acides ou de bases pharmaceutiquement acceptables;
sachant qu'il ne peut y avoir au plus qu'un seul substituant $R^9$ au sein de chaque molécule répondant à la formule générale I;
comme agents antioxydants.

L'invention couvre également les procédés de traitement thérapeutique correspondant à cette utilisation, comme cela est bien compréhensible pour un homme de l'art.

Selon un mode de réalisation avantageux, la présente invention concerne l'utilisation des composés précités de formule générale I pour la fabrication d'une composition pharmaceutique à activité antioxydante, en particulier pour:

* le traitement des pathologies dans lesquelles une surproduction d'hydroperoxydes cytotoxiques contribue aux altérations fonctionnelles des cellules ou des tissus;
* le traitement des pathologies à composante inflammatoire et/ou ischémique:

  - au niveau vasculaire: tel que par exemple le traitement de la prévention des re-sténoses artérielles consécutives à une intervention d'angioplastie, le traitement préventif des sténoses artérielles consécutives aux allogreffes d'artères, le traitement de la claudication intermittente chez les patients atteints d'ischémie obstructive des membres inférieurs; le traitement des accidents cérébro-vasculaires d'origine ischémiques; le traitement préventif ou curatif des syndrômes de détresse respiratoire de l'adulte (ARDS) ou de l'enfant (IRDS).
- au niveau articulaire: tel que par exemple le traitement de la polyarthrite rhumatoïde;

* le traitement des pathologies ophtalmiques: tel que par exemple le traitement des allergies ophtalmiques aigües; le traitement des altérations rétiniennes qui sont associées à une dégénérence maculaire; le traitement du glaucome;
* le traitement de disfonctionnements immunitaires tel que par exemple le traitement du syndrome d'immunodéficience acquise (SIDA);
* le traitement protecteur contre l'intoxication par des xenobiotiques générateurs de radicaux libres, en particulier lors de chimiothérapie anticancéreuse;
* la conservation de greffons en transplantation d'organes tels que le coeur, le foie, le rein et le poumon; par addition de l'un des composés de la présente invention aux milieux de conservation de ces organes.

Dans ces exemples, le principe actif peut être administré par voie orale, rectale ou topique (exemple: collyre pour applications ophtalmiques), ou bien en injection intra-musculaire ou intraveineuse.
Selon un autre mode de réalisation avantageux, le dérivé benzisoséléna-zoline ou -zine répondant à la formule générale (I) précitée est présent en une quantité comprise entre 0,1 et 5% en poids par rapport au poids total de la composition finale, de préférence entre 0,1 et 1% en poids.
Selon un autre mode de réalisation avantageux, la présente invention concerne l'utilisation de la composition sous forme de dose unitaire pouvant comprendre de 1 à 500 mg de dérivé benzisoséléna-zoline ou -zine répondant à la formule générale (I) précitée, éventuellement dans un excipient, véhicule ou support pharmaceutiquement acceptable.
Selon un troisième aspect, la présente invention fournit également une composition pharmaceutique, en particulier à activité antioxydante, caractérisée en ce qu'elle comprend, à titre d'ingrédient actif, au moins un composé benzisoséléna-zoline ou -zine répondant à la formule générale (I) suivante:

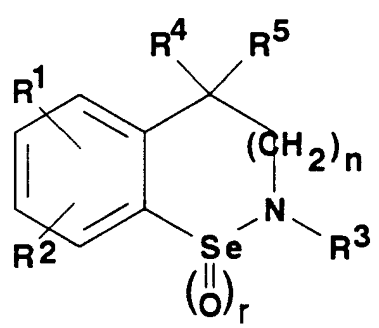

**Formule Générale I**

dans laquelle:

$R^1$ = hydrogène; alkyle inférieur; $-OR^6$: $-(CH_2)_mNR^6R^7$; $-(CH_2)_qNH_2$; $-(CH_2)_mNHSO_2(CH_2)_2NH_2$; -CN; $-SO_3H$; F; Cl; Br: I; $-(CH_2)_mR^8$; $-(CH_2)_mCOR^8$: $-S(O)NR^6R^7$; $-SO_2NR^6R^7$; $-CO(CH_2)_pCOR^8$; $R^9$;

$R^2$ = hydrogène; alkyle inférieur: $-OR^6$; $-(CH_2)_mNR^6R^7$; $-(CH_2)_qNH_2$; $-(CH_2)_mNHSO_2(CH_2)_2NH_2$; -CN; $-SO_3H$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^3$ = hydrogène; alkyle inférieur; aralkyle; aralkyle substitué; $-(CH_2)_mCOR^8$; $-(CH_2)_qR^8$; $-CO(CH_2)_pCOR^8$; $-(CH_2)_mSO_2R^8$; $-(CH_2)_mS(O)R^8$;

$R^4$ = alkyle inférieur; aralkyle; aralkyle substitué; $-(CH_2)_pCOR^8$; $-(CH_2)_pR^8$; F;

$R^5$ = alkyle inférieur; aralkyle; aralkyle substitué;

$R^6$ = alkyle inférieur; aralkyle; aralkyle substitué; $-(CH_2)_mCOR^8$; $-(CH_2)_qR^8$;

$R^7$ = alkyle inférieur; aralkyle; aralkyle substitué; $-(CH_2)_mCOR^8$;

$R^8$ = alkyle inférieur; aralkyle; aralkyle substitué; aryle; aryle substitué; hétéroaryle; hétéroaryle substitué; hydroxyle; alkoxyle inférieur; $R^9$;

$R^9$ =

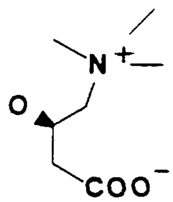 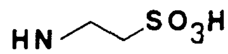 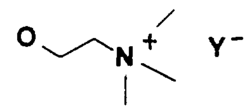

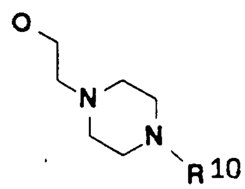 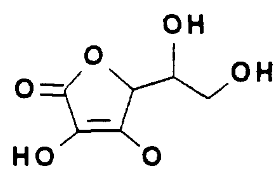 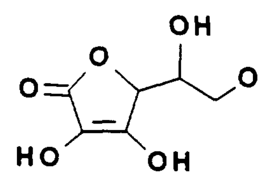

$R^{10}$ = hydrogène; alkyle inférieur; aralkyle ou aralkyle substitué; aryle ou aryle substitué;

$Y^-$ représente l'anion d'un acide pharmaceutiquement acceptable;
n = 0, 1;
m = 0, 1, 2;
p = 1, 2, 3;
q = 2, 3, 4;
r = 0, 1.

et leurs sels d'acides ou de bases pharmaceutiquement acceptables;
sachant qu'il ne peut y avoir au plus qu'un seul substituant $R^9$ au sein de chaque molécule répondant à la formule générale I;
éventuellement dans un excipient, support ou véhicule pharmaceutiquement acceptable.

D'autres modes de réalisation particuliers de cette composition apparaissent clairement à partir de la description précédente et apparaîtront aussi clairement à un homme de l'art à partir de la description suivante, incluant les exemples.

Comme il a été dit précédemment, les composés benzisosélén-azoline et -azine de la formule (I) précitée constituent de précieux agents antioxydants. Dans ce cadre, ils constituent de précieux agents actifs lors d'une application thérapeutique.

Les applications thérapeutiques potentielles des composés de structure générale (I) incluent d'une manière générale, le traitement de toute condition physiopathologique dans laquelle une surproduction d'hydroperoxydes cytotoxiques contribue aux altérations fonctionnelles des cellules ou des tissus. Une telle surproduction d'hydroperoxydes peut être la conséquence de l'activation de voies métaboliques telles que, par exemple, celles des oxygènases à flavine ou à cytochrome P-450, celles des monoamine oxydases. Elle peut être également due à l'activation de cellules endothéliales (xanthine oxydase, 15-lipoxygènase), de plaquettes sanguines (cyclooxygènase et 12-lipoxygènase) et de cellules inflammatoires (NADPH oxydase et 5-lipoxygènase) telles que par exemple des neutrophiles, des macrophages ou des lymphocytes. Elle peut être également due à une intoxication par un xénobiotique, tel que par exemple les anthracyclines, les nitro-imidazoles ou les dérivés de type dipyridinium, générateur de radicaux libres.

Elles incluent plus particulièrement:

* le traitement des pathologies à composante inflammatoire et/ou ischémique:

- au niveau vasculaire: tel que par exemple le traitement de la prévention des re-sténoses artérielles consécutives à une intervention d'angioplastie, le traitement préventif des sténoses artérielles consécutives aux allogreffes d'artères, le traitement de la claudication intermittente chez les patients atteints d'ischémie obstructive des membres inférieurs; le traitement des accidents cérébro-vasculaires d'origine ischémiques; le traitement préventif ou curatif des syndrômes de détresse respiratoire de l'adulte (ARDS) ou de l'enfant (IRDS).
- au niveau articulaire: tel que par exemple le traitement de la polyarthrite rhumatoïde;

* le traitement des pathologies ophtalmiques: tel que par exemple le traitement des allergies ophtalmiques aigües; le traitement des altérations rétiniennes qui sont associées à une dégénérence maculaire; le traitement du glaucome;
* le traitement de disfonctionnements immunitaires tel que par exemple le traitement du syndrome d'immunodéficience acquise (SIDA);
* le traitement protecteur contre l'intoxication par des xénobiotiques générateurs de radicaux libres, en particulier lors de chimiothérapie anticancéreuse;
* la conservation de greffons en transplantation d'organes tels que le coeur, le foie, le rein et le poumon; par addition de l'un des composés de la présente invention aux milieux de conservation de ces organes.

Selon un autre mode de réalisation avantageux, le dérivé benzisoséléna-zoline ou -zine répondant à la formule générale (I) précitée est présent en une quantité comprise entre 0,1 et 5% en poids par rapport au poids total de la composition finale, de préférence entre 0,1 et 1% en poids.

Dans ces applications thérapeutiques, les dérivés de formule générale (I) précitée de l'invention seront avantageusement présentés sous forme de dose unitaire pouvant comprendre de 1 à 500 mg, éventuellement dans un excipient, véhicule ou support pharmaceutiquement acceptable.

De tels excipients, véhicules ou supports pharmaceutiquement acceptables sont bien connus de l'homme de l'art et ne sont pas détaillés ici.

Dans ces exemples, le principe actif peut être administré par voie orale, rectale ou topique (exemple: collyre pour applications ophtalmiques), ou bien en injection intra-musculaire ou intraveineuse.

Les activités antioxydantes et thérapeutiques ou pharmacologiques des dérivés de la formule générale (I) précitée ont été mises en évidence selon des tests sûrs et fiables qui comprennent:

a/ la mesure de l'activité glutathion peroxydase;
b/ la mesure de l'activité réductrice par transfert monoélectronique;
c/ la mesure de l'effet cytoprotecteur sur des cellules endothéliales humaines telles que par exemple celles de veine de cordon ombilical (HUVEC) utilisées entre le ler et le 2ème passage, ou celles issues d'une lignée HUVEC immortalisées par transfection virale.

Compte tenu de ces activités antioxydantes et thérapeutiques ou pharmacologiques, les dérivés benzisosélén-azoline et -azine de la formule générale (I) précitée permettent de réaliser les applications thérapeutiques précédemment énoncées, et plus particulièrement:

* le traitement de pathologies dans lesquelles une surproduction d'hydroperoxydes cytotoxiques contribue aux altérations fonctionnelles des cellules ou des tissus;
* le traitement des pathologies à composante vasculaire inflammatoire et/ou ischémique tel que par exemple le traitement de la prévention des re-sténoses artérielles consécutives à une intervention d'angioplastie, le traitement préventif des sténoses artérielles consécutives aux allogreffes d'artères, le traitement de la claudication intermittente chez les patients atteints d'ischémie obstructive des membres inférieurs; le traitement des accidents cérébro-vasculaires d'origine ischémiques; du glaucome.

Selon un quatrième aspect, la présente invention fournit également un procédé de fabrication des composés répondant à la formule générale (II) suivante:

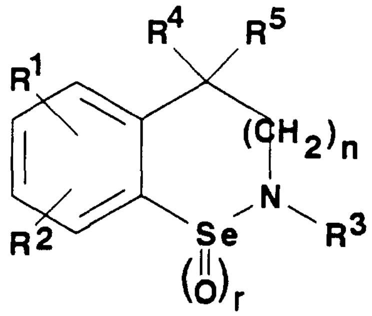

Formule Générale II

dans laquelle:

$R^1$ = hydrogène; alkyle inférieur; $-OR^6$; $-(CH_2)_mNR^6R^7$; $-(CH_2)_qNH_2$; $-(CH_2)_mNHSO_2(CH_2)_2NH_2$; $-NO_2$; -CN; $-SO_3H$; $-N^+(R^5)_2O^-$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^2$ = hydrogène; alkyle inférieur; $-OR^6$; $-(CH_2)_mNR^6R^7$; $-(CH_2)_qNH_2$; $-(CH_2)_mNHSO_2(CH_2)_2NH_2$; $-NO_2$; -CN; $-SO_3H$; $-N^+(R^5)_2O^-$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^3$ = hydrogène; alkyle inférieur; aralkyle; aralkyle substitué; $-(CH_2)_mCOR^8$; $-(CH_2)_qR^8$; $-CO(CH_2)_pCOR^8$; $-(CH_2)_mSO_2R^8$; $-(CH_2)_mS(O)R^8$;

$R^4$ = alkyle inférieur; aralkyle; aralkyle substitué; $-(CH_2)_pCOR^8$; $-(CH_2)_pR^8$; F;

$R^5$ = alkyle inférieur; aralkyle; aralkyle substitué;

$R^6$ = alkyle inférieur; aralkyle; aralkyle substitué; $-(CH_2)_mCOR^8$; $-(CH_2)_qR^8$;

$R^7$ = alkyle inférieur; aralkyle; aralkyle substitué; $-(CH_2)_mCOR^8$;

$R^8$ = alkyle inférieur; aralkyle; aralkyle substitué; aryle; aryle substitué; hétéroaryle; hétéroaryle substitué; hydroxyle; alkoxyle inférieur; $R^9$;

$R^9$ =

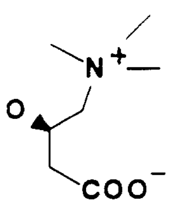

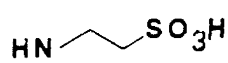

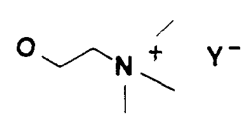

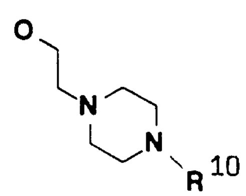

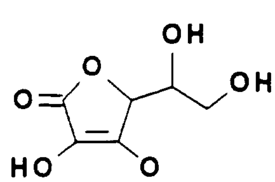

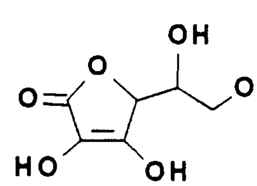

$R^{10}$ = hydrogène; alkyle inférieur; aralkyle ou aralkyle substitué; aryle ou aryle substitué;

$Y^-$ représente l'anion d'un acide pharmaceutiquement acceptable;
n = 0, 1;
m = 0, 1, 2;
p =1, 2, 3;
q = 2, 3, 4;
r = 0, 1.

et leurs sels d'acides ou de bases pharmaceutiquement acceptables;
sachant qu'il ne peut y avoir au plus qu'un seul substituant $R^9$ au sein de chaque molécule répondant à la formule générale II;
caractérisé en ce qu'il comprend les étapes essentielles suivantes (voir Schéma 1):

a/ Préparer ou utiliser un dérivé orthohalogènophényl-acétonitrile gem-disubstitué en position 2, puis selon la série considérée:

soit b1/ hydrolyser le dérivé nitrile en dérivé amide,

c1/ transformer celui-ci en dérivé aminé par une réaction de transposition selon les méthodes usuelles,
d1/ ce composé aminé est mis en réaction avec un dérivé sélénié de type nucléophile, éventuellement généré in situ, en présence d'un sel de cuivre, dans un solvant organique polaire, pour aboutir au dérivé benzisosélénazoline correspondant,
e1/ ce dernier est éventuellement N-alkylé ou N-acylé selon les procédures habituelles;
f1/ enfin, le dérivé ainsi obtenu est éventuellement oxydé au niveau du sélénium selon les procédures usuelles;

soit b2/ réduire le dérivé nitrile en dérivé aminé à l'aide par exemple de borane dans un solvant éthéré comme par exemple le tétrahydrofuranne,

c2/ ce composé aminé est mis en réaction avec un dérivé sélénié de type nucléophile, éventuellement généré in situ, en présence d'un sel de cuivre, dans un solvant organique polaire, pour aboutir au dérivé benzisosélénazine correspondant,
d2/ ce dernier est éventuellement N-alkylé ou N-acylé selon les procédures habituelles;
e2/ enfin, le dérivé ainsi obtenu est éventuellement oxydé au niveau du sélénium selon les procédures usuelles;

Selon un autre mode réalisation de ce procédé, celui-ci est caractérisé en ce que le composé sélénié de type nucléophile est de préférence un sel de sélénocyanate comme par exemple le sélénocyanate de potassium qui peut être:

* soit généré in situ à partir de sélénium métal (Se°) et d'un sel de cyanure comme par exemple le cyanure de potassium,
* soit additionné au milieu réactionnel en tant que tel.

Selon encore un autre mode réalisation particulier de ce procédé, celui-ci est caractérisé en ce que le sel de cuivre peut être un sel cuivreux ($Cu^I$), tel que par exemple l'iodure cuivreux.
Selon un autre mode réalisation de ce procédé, celui-ci est caractérisé en ce que le solvant organique polaire est de préférence le diméthylformamide.
Selon un autre mode réalisation de ce procédé, celui-ci est caractérisé en ce que l'oxydant éventuellement utilisé pour oxyder le sélénium peut être un peracide, tel que l'acide métachloroperbenzoïque, ou le peroxyde d'hydrogène.
D'autres buts, caractéristiques et avantages de l'invention apparaîtront clairement à la lumière de la description explicative qui va suivre faite en référence à divers exemples non limitatifs donnés simplement à titre d'illustration. Dans les exemples, tous les pourcentages sont donnés en poids sauf indication contraire.

**PRESENTATION DES DESSINS :**

- la figure 1 représente un histogramme des résultats obtenus lors d'un test de viabilité effectué sur des cellules endothéliales soumises à un stress oxydant induit par l'hydroperoxyde d'acide linoléïque par rapport au contrôle et où le témoin d'altération est illustré par le rectangle noir, à l'aide de divers composés de l'invention, à savoir BXT-51056, BXT-51072 et BXT-51077, selon le test objet de l'exemple 18.

- La figure 2 représente également un histogramme obtenu dans le cadre de l'étude de l'effet protecteur des composés BXT-51056, BXT-51072 et BXT-51077 de l'invention vis-à-vis de modifications endothéliales induites par des polymorphonucléaires neutrophiles en présence de TNF-$\alpha$, conformément à l'exemple 19 ;

- la figure 3 représente un histogramme similaire à celui de la figure 2 dans le cadre d'une étude de l'effet protecteur des composés BXT-51072 et BXT-51077 de l'invention sur la production endothéliale d'interleukine 8 induite par le TNF-$\alpha$, conformément à l'exemple 20 ;

- la figure 4 représente un histogramme similaire à ceux des figures 2 et 3 obtenu avec les composés BXT-51072 et BXT-51077 de l'invention dans le cadre de l'étude de la protection de cellules endothéliales contre la toxicité de l'interleukine 1, conformément à l'exemple 21;

- les figures 5 et 6 représentent les résultats de tolérance obtenus respectivement avec le contrôle (figure 5) et le composé de l'invention BXT-51072 (figure 6) après administration orale répétée chez le rat in vivo dans les conditions d'essai de l'exemple 24.

**PARTIE EXPERIMENTALE:**

Toutes les réactions ont été effectuées sous atmosphère inerte d'azote, sauf exception notifiée.
Les spectres de masse ont été enregistrés sur un appareil Nermag R10-10B. Le mode d'ionisation utilisé est soit l'impact électronique (EI) à 70 electrons-volts, soit l'ionisation chimique (IC) dans l'ammoniac, le butane ou l'isobutane, soit le bombardement par atonies rapides (FAB) sur une matrice glycérol.
Les spectres $^{1}H$ et $^{13}C$ RMN ont été enregistrés sur un appareil Varian Gemini-200. Les déplacements chimiques sont exprimés en ppm par rapport au tétraméthylsilane. Les multiplicités sont exprimées comme suit: "s" pour singulet, "sl" pour singulet large, "d" pour doublet, "t" pour triplet, "q" pour quadruplet et "m" pour multiplet.
Les points de fusion (pF°C) ont été enregistrés sur un appareil Gallenkamp et sont donnés non corrigés.
Les purifications par chromatographie liquide sur colonne ont été effectuées avec de la silice Merck$^{\mathbf{R}}$ Si60 $F_{254}$.
I/ Exemples de synthèse de composés répondant à la formule générale I:

Série où n = 0 et $R^1 = R^2$ = hydrogène:

Exemple 1: Préparation du 3.3-diméthyl-benzisosélénazoline: BXT 51056

A/ Préparation du 2-(2'-bromophényl)-2-méthyl-propionitrile:

A une suspension de NaH (4,0g; 100mmoles) dans du THF (100ml), portée au reflux et sous atmosphère inerte, est ajoutée lentement (60mn) la solution de 2-(2'-bromophényl)-acétonitrile (7,5g; 38mmoles) et d'iodométhane (16,2g; 114mmoles) dans du THF (30ml). La réaction est exothermique. Le chauffage au reflux est poursuivi pendant 2,5h. Puis le mélange réactionnel est agité à température ambiante pendant 15h. Le solvant est évaporé sous pression réduite; le résidu est repris par 100ml d'eau et extrait avec 2x100ml de terbutylméthyléther. Les phases organiques sont rassemblées, lavées avec 3x100ml d'une solution de NaCl saturée; séchées sur $MgSO_4$ et filtrées. Le solvant est évaporé sous pression réduite. Le produit désiré est obtenu sous forme d'une huile incolore après distillation sous pression réduite (T°= 125-140°C; p=0,lmbar).
Rendement: 79%
Ce produit a aussi été obtenu selon la procédure de W.E. Parham et L.D. Jones (voir J. Org. Chem.; (1976); 41; pages 1187-1191) avec un rendement de 90%.
Caractéristiques Physiques:

- * RMN $^1H$: ($CDCl_3$)
  1,90 ppm (s; 6H); 7,19 ppm (m; 1H; J= 7,5Hz); 7,35 ppm (t; 1H; J= 7,5Hz); 7,49 ppm (m; 1H; J= 7,5Hz); 7,67 ppm (d; 1H; J= 7,5Hz).
- * RMN $^{13}C$: ($CDCl_3$)
  27,13 ppm; 37,64 ppm; 123,08 ppm; 123,92 ppm; 127,76 ppm; 128,49 ppm; 130,15 ppm; 136,18 ppm; 138,77 ppm.
- * SM: (IE; 70eV)
  225/223 ($M^+$; 80%); 210/208 (100%); 183/181 (75%); 102 (20%).

B/ Préparation du 2-(2'-bromophényl)-2-méthyl-propionamide:

Le dérivé précédent (6,7g; 30mmoles) est dissout dans de l'éthanol (70ml); puis à cette solution est ajoutée une solution saturée de carbonate de potassium (70ml) et avec précaution le peroxyde d'hydrogène (solution à 50%; 2x70ml) à une température de 10-15°C. La réaction est exothermique. Le mélange réactionnel est agité à température ambiante pendant 14h. Du dichlorométhane (200ml) est additionné au milieu réactionnel, puis ce drenier est décanté. La phase aqueuse est extraite par 100ml de dichlorométhane. Les phases organiques sont rassemblées, lavées avec 3x200ml d'eau; séchées sur $MgSO_4$ et filtrées. Le produit désiré est obtenu sous forme d'une huile incolore très visqueuse, et est utilisé tel quel pour la prochaine étape.
Rendement: 90%
Caractéristiques Physiques:

- * RMN $^1H$: ($CDCl_3$)
  1,67 ppm (s; 6H); 5,1-5,5 ppm (sl; 2H); 7,17 ppm (td; 1H; $J_d$=2,0Hz $J_t$=7,5Hz); 7,36 ppm (td; 1H; $J_d$=2,0Hz $J_t$=7,5Hz); 7,52 ppm (dd; 1H; J= 2,0-7,5Hz); 7,63 ppm (dd; 1H; J= 2,0-7,5Hz).
- * RMN $^{13}C$: ($CDCl_3$)
  26,92 ppm; 48,78 ppm; 124,89 ppm; 128,34 ppm; 128,50 ppm; 129,42 ppm; 135,63 ppm; 143,84 ppm; 179,95 ppm.
- * SM: ( IE; 70eV)
  244/242 ($MH^+$; 3%); 225/223 (5%); 210/208 (10%); 171/169 (30%); 162 (100%); 145 (10%); 115 (30%); 91 (30%); 77 (25%).

C/ Préparation de la 1-(2'-bromophényl)-1-méthyl-éthylamine:

Le dérivé précédent, dissout dans un mélange acétonitrile/eau (50/50, 60ml), est additionné du bis(trifluoroacétoxy)iodosobenzène (10,88g; 25,3mmoles) en une portion. Le mélange réactionnel est agité à température ambiante pendant 24h. Après addition d'eau (450ml), l'agitation est maintenue pendant 30min. supplémentaires. Du tertiobutylméthyléther (150ml) est additionné au milieu réactionnel, puis ce dernier est décanté. La phase organique est extraite avec une solution d'acide chlorhydrique à 10%, puis la phase aqueuse est lavée par 3x150ml de tertiobutylméthyléther. La phase aqueuse est alcalinisée ($12<pH<14$) à 10°C, puis extraite par 3x150ml de dichlorométhane. Les phases organiques sont rassemblées, séchées sur $MgSO_4$ et filtrées. Le produit désiré est obtenu, sous forme d'une huile incolore très visqueuse, après distillation (T°= 60-70°C; p=0,1mbar).
Rendement: 83%
Caractéristiques Physiques:

- * RMN $^1H$: ($CDCl_3$)
  1,65 ppm (s; 6H); 2,15 ppm (sl; 2H); 7,05 ppm (td; 1H; $J_d$=2,0Hz $J_t$=7,5Hz); 7,25 ppm (td; 1H; $J_d$=2,0Hz $J_t$=7,5Hz); 7,57 ppm (m; 2H).
- * RMN $^{13}C$: ($CDCl_3$)
  30,71 ppm; 54,17 ppm; 122,22 ppm; 127,76 ppm; 127,97 ppm; 128,56 ppm; 136,12 ppm; 147,75 ppm.
- * SM: (IE; 70eV)
  216/214 ($MH^+$, 2%); 200/198 (100%); 58 (98%).
- * SM: (IC; butane)
  216/214 ($MH^+$; 100%); 199/197 (20%); 154 (25%); 93 (30%).

D/ Préparation du 3,3-diméthyl-benzisosélénazoline: BXT 51056

A une solution du dérivé précédent (1,605g; 7,5mmoles) dans du DMF (35ml) est ajoutée de la triéthylamine (3,13ml; 37,5mmoles), puis du sélénocyanate de potassium (1,19g; 8,25mmoles) et de l'iodure de cuivre CuI (1,49g; 7,5mmoles). Le mélange réactionnel est agité pendant 18h. à température ambiante; puis versé dans 150ml d'eau. La suspension obtenue est filtrée et lavée avec 150ml d'acétate d'éthyle. Après décantation, la phase aqueuse est

extraite par 2x150ml d'acétate d'éthyle. Les phases organiques sont rassemblées, lavées avec 2x100ml d'une solution de NaCl saturée; séchées sur $MgSO_4$ et filtrées. Le solvant est évaporé sous pression réduite. Le produit désiré est obtenu après purification par chromatographie liquide sur une colonne de silice (éluant: cyclohexane-acétate d'éthyle 5/1).
Rendement: 64%
Caractéristiques Physiques:

* pF°C: 38,5-39,5°C (rev.)
* RMN $^1H$: ($CDCl_3$)
  1,55 ppm (s; 6H); 4,10 ppm (sl; 1H); 7,05 ppm (m; 1H); 7,18 ppm (m; 2H); 7,30 ppm (m; 1H).
  (acétone d6)
  1,48 ppm (s, 6H); 4,70 ppm (sl; 1H); 7,05-7,25 ppm (m; 3H); 7,40 ppm (m; 1H). ( DMSO $d_6$)
  1,40 ppm (s; 6H); 5,25 ppm (sl; 1H); 7,00-7,20 ppm (m; 3H); 7,45 ppm (m; 1H).
* RMN $^{13}C$: ( acétone $d_6$)
  26,63 ppm; 71,29 ppm; 124,31 ppm; 125,08 ppm; 126,68 ppm; 128,61 ppm; 140,57 ppm; 150,85 ppm.
* SM: (IE; 70eV)
  213 ($M^+$; 20%); 198 (100%); 157 (25%); 80 (20%).

Exemple 2: Préparation du 2-acétyl-3,3-diméthyl-benzisosélénazoline: BXT 51057

Le dérivé 3,3-diméthyl-benzisosélénazoline (148mg; 0,7mmoles) est dissout à température ambiante dans de l'éther éthylique (4ml). Sont ajoutés de la triéthylamine (107µl; 0,77mmoles) et du chlorure d'acétyle (54µl; 0,77mmoles). Un précipité blanc apparait immédiatement. Après lh., le mélange réactionnel est additionné de 10ml d'eau. Le produit brut est extrait par 3x20ml de dichlorométhane. Les phases organiques sont rassemblées, lavées avec 2x20ml d'une solution de NaCl saturée; séchées sur $MgSO_4$ et filtrées. Le solvant est évaporé sous pression réduite. Le produit désiré est obtenu après purification par chromatographie liquide sur une colonne de silice (éluant: cyclohexane-acétate d'éthyle 1/1).
Rendement: 26%
Caractéristiques Physiques:

* pF°C: 91-92°C (rev.)
* RMN $^1H$: ($CDCl_3$)
  1,79 ppm (s; 6H); 2,32 ppm (sl; 3H); 7,00 ppm (m; 1H); 7,05-7,20 ppm (m; 3H). ( acétone $d_6$)
  1,80 ppm (s; 6H); 2,25 ppm (sl; 3H); 7,15-7,35 ppm (m; 3H); 7,43 ppm (m; 1H).
* RMN $^{13}C$: ( acétone $d_6$)
  22,0 ppm; 27,7 ppm; 67,0 ppm; 123,79 ppm; 125,75 ppm; 127,35 ppm; 129,63 ppm; 132,61 ppm; 149,13 ppm; 169,06 ppm.
* SM: (IE; 70eV)
  255 ($M^+$; 30%); 240 (30%); 198 (100%).

Exemple 3: Préparation du 3,3-diméthyl-2-éthyl-benzisosélénazoline: BXT 51058

A une solution du dérivé 3,3-diméthyl-benzisosélénazoline (107mg; 0,5mmoles), obtenu précédemment, dans du bromoéthane (1,5ml) est ajouté du diazabicycloundécéne (380mg; 2,5mmoles). Le mélange ainsi obtenu est agité pendant 10h. à température ambiante, puis la même quantité de diazabicycloundécéne est rajoutée. Après 14h. à température ambiante, le bromoéthane est évaporé sous pression réduite. Le produit désiré est obtenu, sous forme d'une huile jaune très visqueuse, après purification par chromatographie liquide sur une colonne de silice (éluant: cyclohexane-acétate d'éthyle 6/1).
Rendement: 53%
Caractéristiques Physiques:

* RMN $^1H$: ($CDCl_3$)
  1,16 ppm (t; 3H; J= 7,0Hz); 1,56 ppm (s; 6H); 2,84 ppm (q; 2H; J= 7,0Hz); 7,05 ppm (m; 1H); 7,18 ppm (m; 2H); 7,20 ppm (m; 1H).
* RMN $^{13}C$: ($CDCl_3$)
  16,78 ppm; 25,33 ppm; 47,82 ppm; 73,27 ppm; 124,01 ppm; 124,90 ppm; 126,29 ppm; 128,22 ppm; 136,29 ppm; 148,47 ppm.
* SM: (IE; 70eV)

241 ($M^+$; 30%); 226 (100%); 198 (30%); 157 (10%); 115 (10%).

Exemple 4 : Préparation du 3,3-diméthyl-benzisosélénazoline-1-oxide: BXT 51088

Le dérivé 3,3-diméthyl-benzisosélénazoline BXT 51056 (212mg; Immole) est dissous à température ambiante dans du méthanol (5ml) sous agitation. Après addition d'eau oxygenée à 5% (600 ·1; 1,05 mmole), l'agitation est maintenue pendant 15 min. Le solvant est evaporé sous pression réduite. Le résidu est repris par 25ml de dichlorométhane et lavé avec 3x3ml d'une solution de NaCl saturée; séché sur $Na_2SO_4$ et filtré. Après évaporation du solvant sous pression réduite, le produit désiré est obtenu sous forme de cristaux jaunâtres.
Rendement: 55%
Caractéristiques Physiques:

* RMN $^1H$: ($CDCl_3$)
  1,54 ppm (sl; 3H); 1,72 ppm (sl; 3H); 4,35 ppm (sl; 1H); 7,32 ppm (d; 1H; J = 8,0 Hz); 7,50 ppm (m; 2H); 7,76 ppm (d; 1H; J = 8,0 Hz).
* RMN $^{13}C$: ($CDCl_3$)
  30,98 ppm; 35,20 ppm; 71,40 ppm; 124,59 ppm; 126,62 ppm; 129,35 ppm; 132,29 ppm; 145,16 ppm; 151,73 ppm.
* SM: (FAB)
  230 ($MH^+$; 100%); 154 (90%); 136 (70%).

Série où n = 0 et R1 ≠ hydrogène:

Exemple 5: 3,3'-diméthyl-7-nitro-benzisosélénazoline: BXT 51062

A/ Préparation du (2'-bromo-3'-nitro)-phénylacétonitrile:

Ce composé est préparé selon les méthodes classiques à partir du 2-bromo-3-nitro-toluène par monohalogènation pour conduire au 2-bromo-3 nitro-bromobenzyle (voir A. Ricci et coll.; Ann. Chim. (Rome); (1963); 53; page 1860), puis par substitution à l'aide de cyanure de potassium pour obtenir le (2'-bromo-3'-nitro)-phénylacétonitrile (voir J. Weinstock et coll.; J. Med. Chem.; (1987); 30; page 1166).
Rendement global de ces deux étapes: 43%:
Caractéristiques Physiques:

* pF°C: 122-123°C (rev.)
* RMN $^1H$: ($CDCl_3$)
  3,95 ppm (s; 2H); 7,55 ppm (t; 1H; J= 8Hz); 7,76 ppm (m; 2H).
* RMN $^{13}C$: ($CDCl_3$)
  25,78 ppm; 115,84 ppm; 116,52 ppm; 125,44 ppm; 129,37 ppm; 139,10 ppm; 143,87 ppm; 152,01 ppm.
* SM: (IE, 70eV)
  242/240 ($M^+$; 70%); 212/210 (20%); 193/191 (30%); 115 (100%); 103 (50%); 88 (30%).

B/ Préparation du 2-(2'-bromo-3'-nitro)-phényl-2-méthyl-propionitrile:

A partir du dérivé précédent et selon un protocole similaire à celui décrit dans l'exemple 1/A, le dérivé désiré est obtenu.
Rendement: 70%
Caractéristiques Physiques:

* pF°C: 99-100°C (rev.)
* RMN $^1H$: ($CDCl_3$)
  1,95 ppm (s; 6H); 7,45-7,70 ppm (m; 3H).
* SM: (IE, 70eV)
  270/268 ($M^+$; 80%); 255/253 (100%); 228/226 (40%); 143 (20%); 127 (25%); 115 (30%); 61 (40%).

C/ Préparation du 2-(2'-bromo-3'-nitro)-phényl-2-méthyl-propionamide:

A partir du dérivé précédent et selon un protocole similaire à celui décrit dans l'exemple 1/B le dérivé désiré est obtenu.

Rendement: 77%
Caractéristiques Physiques:

* pF°C: 123°C (rev.)
* RMN $^1H$: ($CDCl_3$)
1,73 ppm (s; 6H); 5,40 ppm (sl; 2H); 7,45-7,57 ppm (m; 2H); 7,70 ppm (m; 1H).
* SM: (IC; butane)
289/287 ($M^+$; 100%); 207 (15%).

D/ Préparation de la 1-(2'-bromo-3'nitro)-phényl-lméthyl-éthylamine:

A partir du dérivé précédent et selon un protocole similaire à celui décrit dans l'exemple 1/C le dérivé désiré est obtenu.
Rendement: 57%
Caractéristiques Physiques:

* RMN $^1H$: ($CDCl_3$)
1,70 ppm (s; 6H); 1,90 ppm (sl; 2H); 7,40 ppm (m; 2H); 7,85 ppm (m; 1H).

E/ Préparation du 3,3'-diméthyl-7-nitro-benzisosélénazoline: BXT 51062

A partir du dérivé précédent et selon un protocole similaire à celui décrit dans l'exemple 1/D le dérivé désiré est obtenu.
Rendement: 45%
Caractéristiques Physiques:

* pF°C: 121°C (rev.)
* RMN $^1H$: ($CDCl_3$)
1,55 ppm (s; 6H); 3,75 ppm (sl; 1H); 7,25-7,43 ppm (m; 2H); 8,19 ppm (m; 1H).
* RMN $^1H$: (acetone $d_6$)
1,55 ppm (s; 6H); 4,65 ppm (sl; 1H); 7,46-7,60 ppm (m; 2H); 8,19 ppm (m; 1H).
* RMN $^{13}C$: (acetone $d_6$)
27,06 ppm; 71,43 ppm; 124,05 ppm; 128,84 ppm; 129,48 ppm; 137,18 ppm;
* SM: (IE; 70eV)
258 ($M^+$; 20%); 243 (100%); 197 (40%); 117 (10%).

Exemple 6: 3,3'-diméthyl-5-nitro-benzisosélénazoline: BXT 51075

A/ Préparation du 2-(2'-bromo-5'-nitro)-phényl-2-méthyl-propionitrile

Ce composé est préparé selon une procédure très similaire à celle du dérivé de l'exemple 1/A et est obtenu sous forme d'un solide jaune.
Rendement: 80%
Caractéristiques Physiques:

* pF°C: 96°C (rev.)
* RMN $^1H$: ($CDCl_3$)
1,95 ppm (s; 6H); 7,89 ppm (d; 1H; J=9,0Hz); 8,08 ppm (dd; 1H; J=2,5-9,0Hz); 8,32 ppm (d; 1H; J=2,5Hz).

B/ Préparation du 2-(2'-bromo-5'-nitro)-phényl-2-méthyl-propionamide

L'hydrolyse du composé précédent est réalisée selon une procédure très similaire à celle du dérivé de l'exemple 1/B, pour conduire au composé désiré, obtenu sous forme d'un poudre très légèrement jaune.
Rendement: 56%
Caractéristiques Physiques:

* pF°C: 206°C (rev.)
* RMN $^1H$: ($CDCl_3$)

1,74 ppm (s; 6H); 5,35 ppm (sl; 2H); 7,82 ppm (d; 1H; J=8,5Hz); 8,30 ppm (dd; 1H; J=2,5-8,5Hz); 8,37 ppm (d; 1H; J=2,5Hz).

* SM: (IC; isobutane)
  289/287 ($MH^+$; 100%); 207 (15%).

C/ Préparation du 1-(2'-bromo-5'-nitro)-phényl-1-méthyl-éthylamine

Ce composé est préparé selon une procédure très similaire à celle du dérivé de l'exemple 1/C et est obtenu sous forme d'un poudre légèrement jaune.
Rendement: 83%
Caractéristiques Physiques:

* pF°C: 105-106°C (rev.)
* RMN $^1$H: ($CDCl_3$)
  1,71 ppm (s; 6H); 1,90 ppm (sl; 2H); 7,77 ppm (d; 1H; J=9,0Hz); 7,93 ppm (dd; 1H; J=3,0-9,0Hz); 8,57 ppm (d; 1H; J=3,0Hz).
* RMN $^{13}$C: ($CDCl_3$)
  30,41 ppm; 54,49 ppm; 122,99 ppm; 123,12 ppm; 129,36 ppm; 137,07 ppm; 147,66 ppm (1); 150,14 pm.
* SM: (IC; isobutane)
  261/259 ($MH^+$; 100%).

D/ Préparation du 3,3-diméthyl-5-nitro-benzisosélénazoline:

Ce composé est préparé selon une procédure très similaire à celle du dérivé de l'exemple 1/D et obtenu sous forme des cristaux jaunes.
Rendement: 61%
Caractéristiques Physiques:

* pF°C: 107°C (rev.)
* RMN $^1$H: ($CDCl_3$)
  1,56 ppm (s; 6H); 4,20 ppm (sl; 1H); 7,44 ppm (d; 1H; J=8,5Hz); 7,83 ppm (d; 1H; J=2,0Hz); 8,08 ppm (dd; 1H; J=2,0-8,5Hz).
* RMN $^{13}$C: ($CDCl_3$)
  26,48 ppm; 70,86 ppm; 118,39 ppm; 123,72 ppm; 124,39 ppm; 147,48 ppm (1); 149,91 ppm; 151,65 ppm.
* SM: (IC; isobutane)
  259 ($MH^+$; 100%).

Exemple 7: Préparation du 3,3-diméthyl-7-fluoro-benzisosélénazoline: BXT 51076

A/ Préparation du 2-(3'-amino-2'-bromo)-phényl-2-méthyl-propionitrile:

Le composé obtenu dans l'exemple 4/B est réduit à l'aide du chlorure d'etain(II) pour conduire au dérivé désiré sous forme d'un solide marron et est utilisé tel quel pour l'étape suivante.
Rendement: 94%
Caractéristiques Physiques:

* RMN $^1$H: ($CDCl_3$)
  1,87 ppm (s; 6H); 4,30 ppm (sl; 2H); 6,79 ppm (m; 2H); 7,11 ppm (t; 1H; J=8,0Hz).
* SM: (IE, 70eV)
  240/238 ($M^+$; 100%); 225/223 (25%); 198/196 (30%); 144 (20%); 117 (20%).

B/ Préparation du 2-bromo-3-(2'-méthyl-2'-propionitrilo)-benzènediazonium hexafluorophosphate:

Le dérivé précédent (1,54 g; 6,44 mmoles) est dissous à température ambiante dans une solution constituée d'acide chlorhydrique concentré (2,7 ml) dilué par 20 ml d'eau. Du nitrite de sodium (533 mg; 7,73 mmoles) est ajouté sous agitation à une température de 0-5°C, ainsi que, aprés 5 min., de l'hexafluorophosphate de lithium (1,66 g; 10,95 mmoles). Un précipité blanc apparait immédiatement. Après 30 min., la suspension obtenue est filtrée et lavée avec 8 ml d'eau froide, puis avec 9 ml d'un mélange d'éther terbutylméthylique et de méthanol (2:1). Le composé desiré est

obtenu sous forme d'une poudre incolore et est utilisé tel quel pour la prochaine étape.
Rendement: 86%
Caractéristiques Physiques:

* pF°C: 127°C (dec.)
* RMN $^1$H: ( acétone $d_6$)
  2,05 ppm (s; 6H); 8,23 ppm (m; 1H); 8,60 ppm (m; 1H); 9,03 ppm (m; 1H).

C/ Préparation du 2-(2'-bromo-3'-fluoro)-phényl-2-méthyl-propionitrile

Ce composé est préparé selon la méthode de Schiemann par pyrolyse du composé précédent (5x300 mg; 3,78 mmoles), mélangé avec du fluorure de potassium (5x300 mg), sans solvant et sous vide (0,1 mbar). Le produit désiré est obtenu sous forme d'un poudre légèrement marron.
Rendement: 30%
Caractéristiques Physiques:

* pF°C: 39°C (rev.)
* RMN $^1$H: ($CDCl_3$)
  1,91 ppm (s; 6H); 7,15 ppm (td; 1H; $J_d$=2,0Hz, $J_t$=8,0Hz); 7,23-7,41 ppm (m; 2H).
* SM: (IE, 70eV)
  243/241 ($M^+$; 80%); 228/226 (100%); 201/199 (80%); 120 (30%).

D/ Préparation du 2-(2'-bromo-3'-fluoro)-phényl-2-méthyl-propionamide

L'hydrolyse du composé précédent est réalisée selon une procédure très similaire à celle du dérivé de l'exemple 1/B, pour conduire au composé désiré, obtenu sous forme de cristaux incolores.
Rendement: 76%
Caractéristiques Physiques:

* pF°C: 140°C (rev.)
* RMN $^1$H: ($CDCl_3$)
  1,69 ppm (s; 6H); 5,15-5,35 ppm (sl; 2H); 7,10 ppm (m; 1H); 7,23-7,41 ppm (m; 2H).
* SM: (IC; isobutane)
  262/260 ($MH^+$; 100%); 180 (20%).

E/ Préparation du 1-(2'-bromo-3'-fluoro)-phényl-1-méthyl-éthylamine

Ce composé est préparé selon une procédure très similaire à celle du dérivé de l'exemple 1/C et est obtenu sous forme d'un huile incolore.
Rendement: 72%
Caractéristiques Physiques:

* RMN $^1$H: ($CDCl_3$)
  1,67 ppm (s; 6H); 1,90 ppm (sl; 2H); 7,03 ppm (td; 1H; $J_d$=2,0Hz, $J_t$=8,0Hz); 7,24 ppm (td; 1H; $J_d$=5,5Hz, $J_t$=8,0Hz); 7,39 ppm (m; 1H; J=8,0Hz).
* SM: (IC; isobutane)
  234/232 ($MH^+$; 100%).

F/ Préparation du 3,3-diméthyl-7-fluoro-benzisosélénazoline:

Ce composé est préparé selon une procédure très similaire à celle du dérivé de l'exemple 1/D et est obtenu sous forme de cristaux jaunâtres.
Rendement: 52%
Caractéristiques Physiques:

* pF°C: 25°C (rev.)
* RMN $^1$H: ($CDCl_3$)
  1,50 ppm (s; 6H); 4,05 ppm (sl; 1H); 6,82 ppm (d; 1H; J=7,5Hz); 6,92 ppm (td; 1H; $J_d$=1,0Hz, $J_t$=7,5Hz); 7,16 ppm

(td; 1H; $J_d$=5,0Hz, $J_t$=7,5Hz).

* SM: (IE, 70eV)
  231 ($M^+$; 30%); 216 (100%); 175 (25%).

Série où n = 1 et $R^1$ = $R^2$ = hydrogène:

Exemple 8: Préparation du 4,4-diméthyl-benzisosélénazine: BXT 51072

A/ Préparation du 2-(2'-bromophényl)-2-méthyl-propylamine:

Le dérivé 2-(2'-bromophenyl)-2-méthyl-propionitrile de l'exemple 1/A (2,24 g; 10 mmoles) est dissous sous atmosphère inerte dans du THF (25 ml). La solution du borane $BH_3$ dans le THF (1 M; 25 ml; 25 mmoles) est ajoutée lentement au milieu réactionnel. La nouvelle solution, ainsi obtenue, est portée au reflux pendant 3 h. Le mélange réactionnel, ramené à température ambiante, est ajouté goutte à goutte à une solution aqueuse d'acide trifluoroacétique (50 ml; 1/1). Le mélange est porté au reflux pour 1 h., puis les solvants sont evaporés sous pression réduite. Le residu est repris par 60 ml d'acide chlorhydrique HCl (10 %) et lavé avec 3x100 ml d'acétate d'éthyle. La phase aqueuse est alcalinisée (12 < pH < 14), puis extraite par 3x50 ml de dichlorométhane. Les phases organiques sont rassemblées, lavées avec 2x100 ml d'une solution de chlorure de sodium saturée, séchées sur $MgSO_4$ et filtrées. Le solvant est évaporé sous pression réduite. Le composé désiré est obtenu sous forme d'une huile jaunâtre.
Rendement: 60%
Caractéristiques Physiques:

* RMN $^1H$: ($CDCl_3$)
  1,40 ppm (sl; 2H); 1,44 ppm (s; 6H); 3,19 ppm (s; 2H); 7,03 ppm (td; 1H; $J_d$=2,0Hz, $J_t$=8,0Hz); 7,24 ppm (td; 1H, $J_d$=1,5Hz, $J_t$=8,0Hz); 7,38 ppm (dd; 1H; J=2,0-8,0Hz); 7,57 ppm (dd; 1H; J=1,5-8,0Hz).
* RMN $^{13}C$: ($CDCl_3$)
  26,59 ppm; 42,43 ppm; 50,73 ppm; 122,76 ppm; 127,87 ppm; 128,46 ppm; 130,62 ppm; 136,42 ppm; 144,79 ppm.
* SM: (IC; isobutane)
  230/228 ($MH^+$; 100%); 148 (30%).

B/ Préparation de la 4,4-diméthyl-benzisosélénazine:

Ce composé est obtenu selon une procédure très similaire à celle du dérivé de l'exemple 1/D sous forme de cristaux incolores.
Rendement: 20%
Caractéristiques Physiques:

* pF°C: 81°C (rev.)
* RMN $^1H$: ($CDCl_3$)
  1,28 ppm (s; 6H); 3,25 ppm (s; 2H); 3,45 ppm (sl; 1H); 6,98-7,14 ppm (m; 3H); 7,43 ppm (m; 1H).
* RMN $^{13}C$: ($CDCl_3$)
  28,46 ppm; 33,02 ppm; 61,41 ppm; 125,21 ppm; 125,94 ppm; 126,83 ppm; 127,97 ppm; 129,87 ppm; 143,26 ppm.
* SM: (IE, 70eV)
  227 (M+; 85%); 198 (80%); 183 (100%); 132 (30%); 117 (50%); 102 (20%); 91 (25%).

Exemple 9: Préparation du 4,4-diméthyl-benzisosélénazine-1-oxide: BXT 51089

A partir du 4,4-diméthyl-benzisosélénazine BXT 51072 et selon un protocole similaire à celui décrit dans l'exemple 4, le dérivé désiré est obtenu.
Rendement: 84%
Caractéristiques Physiques:

* RMN $^1H$: ($CDCl_3$)
  1,34 ppm (s; 3H); 1,37 ppm (s; 3H); 2,79 ppm (d; 1H; J = 14 Hz); 3,3 ppm (sl; 1H); 3,98 ppm (d; 1H; J = 14 Hz); 7,28 ppm (m; 1H); 7,36 ppm (m; 3H).
  ($D_2O$)
  1,29 ppm (s; 3H); 1,37 ppm (s; 3H); 2,96 ppm (d; 1H; J = 14 Hz); 3,65 ppm (d; 1H; J = 14 Hz); 7,42 ppm (m; 1H); 7,60 ppm (m; 2H); 7,74 ppm (m; 1H).

* RMN $^{13}C$: ($D_2O$)
  28,43 ppm; 30,73 ppm; 36,79 ppm; 49,88 ppm; 130,53 ppm; 131,06 ppm; 132,52 ppm; 135,97 ppm; 137,04 ppm; 148,08 ppm.
* SM: (FAB):
  244 ($MH^+$; 100%); 154 (50%); 136 (38%).

Exemple 10: Préparation du 4,4-diméthyl-2-éthyl-benzisosélénazine: BXT 51078

A/ Préparation du N-[2-(2'-bromophényl)-2-méthyl-propyl]-N-éthyl-amine

De l'iodoéthane (146mg; 75·1; Immole) est ajouté au dérivé 2-(2'-bromophényl)-2-méthyl-propylamine de l'exemple 7/A (228mg; Immole). Le mélange réactionnel est agité à température ambiante. Après 30min., un précipité blanc apparait et après 60 min., on ajoute 500 · 1 de chloroforme. L'agitation est poursuivie pendant 1 h. Du dichlorométhane (40ml) et une solution de $NaHCO_3$ saturée (20ml) sont additionnés au milieu réactionnel, puis ce dernier est décanté. La phase organique est lavée avec 20ml d'une solution de NaCl saturée; séché sur $MgSO_4$ et filtré.Le solvant est evaporé sous pression réduite. Le produit désiré est obtenu sous forme d'une huile jaunâtre, en mélange avec12% du produit de départ (II) et 12% du N-[2-(2'-bromophényl)-2-méthylpropyl]-N,N-diéthyl-amine (III). Ce mélange est utilisé tel quel pour la prochaine étape.
Rendement brut: 76%
Caractéristiques Physiques:

* RMN $^{1}H$: ($CDCl_3$) (mélange du produit désiré (I), II et III; 76:12:12)

  I: 1,03 ppm (t; 3H; J = 7 Hz); 1,52 ppm (s; 6H); 2,62 ppm (q; 2H; J = 7 Hz); 3,13 ppm (s; 2H); 7,06 ppm (m; 1H); 7,28 ppm (m; 1H); 7,45 ppm (m; 1H); 7,60 ppm (m; 1H).
  II: 2-(2'-bromophényl)-2-méthyl-propylamine de l'exemple 7/A
  III: 0,85 ppm (t; 6H; J = 7 Hz); 2,39 ppm (q; 4H; J = 7 Hz); 3,22 ppm (s; 2H).

* SM: (IC; isobutane)
  258/256 ($M^+$; 100%).

B/ Préparation du 4,4-diméthyl-2-éthyl-benzisosélénazine:

Ce composé est obtenu selon une procédure trés similaire à celle du dérivé de l'exemple 1/D à partir du dérive précédent, sous forme d'une huile jaune.
Rendement: 20%
Caractéristiques Physiques:

* RMN $^{1}H$: ($CDCl_3$)
  1,19 ppm (t; 3H; J = 7 Hz); 1,35 ppm (s; 6H); 2,95 ppm (q; 2H; J = 7 Hz); 3,16 ppm (s; 2H); 7,07 ppm (m; 3H); 7,43 ppm (m; 1H).
* RMN $^{13}C$: ($CDCl_3$)
  14,63 ppm; 30,26 ppm; 36,39 ppm; 54,69 ppm; 69,93 ppm; 126,13 ppm; 126,67 ppm; 126,76 ppm; 127,93 ppm; 129,34 ppm; 143,64 ppm.
* SM: (IC; isobutane)
  256 ($MH^+$; 100%).

Série où n = 1 et $R^1 \neq$ hydrogène:

Exemple 11: Préparation du 4,4-diméthyl-6-méthoxy-benzisosélénazine: BXT 51077

A/ Préparation du 5-amino-2-bromo-phénylacétonitrile:

Le 2-(2'-bromo-5'-nitro)-phenylacétonitrile (1,65g; 6,85 mmoles) est réduit à l'aide du chlorure d'étain(II) selon la procédure décrite par F. BELLAMY et coll. (voir Tetrahedron Let.; (1984); 25; 8; pages 839-842) pour conduire au dérivé désiré, obtenu sous forme d'une poudre jaunâtre.
Rendement: 69%
Caractéristiques Physiques:

- * pF°C: 100°C (rev.)
- * RMN $^{1}H$: ($CDCl_3$)
  3,75 ppm (s; 2H); 3,80 ppm (sl; 2H); 6,53 ppm (dd; 1H; J=2,5-8,5Hz); 6,85 ppm (d; 1H;J=2,5Hz); 7,31 ppm (d; 1H; J=8,5Hz).
- * SM: (IE, 70eV)
  210/212 ($M^+$; 100); 131 (60); 104 (25); 77 (20).

B/ Préparation du 2-bromo-5-hydroxy-phénylacétonitrile:

Le dérivé précédent (1 g; 4,74 mmoles) est dissous dans l'acide sulfurique (35%; 20 ml); puis à cette solution est ajoutée une solution du nitrite de sodium (409 mg; 6 mmoles) dans l'eau (5 ml) à une température de 0°C. Après 5 min., une solution de nitrate de cuivre(II) (17,2 g; 71 mmoles) dans l'eau (100 ml) est ajoutée, puis de l'oxide de cuivre(I) solide (678 mg; 4,74 mmoles). Le mélange réactionnel est agité à température ambiante pendant 2 h., puis extrait avec 3x100 ml d'éther terbutylméthylique. Les phases organiques sont rassemblées, puis extraites avec 2x100 ml de soude NaOH (1N). Les phases aqueuses sont rassemblées, acidifiées (pH=2), puis extraites avec 3x100 ml de dichlorométhane. Les phases organiques sont rassemblées, séchées sur $MgSO_4$ et filtrées. Le produit désiré est obtenu sous forme d'une poudre marron, et est utilisé tel quel pour la prochaine étape.
Rendement: 69%
Caractéristiques Physiques:

- * RMN $^{1}H$: ($CDCl_3$)
  3,81 ppm (s; 2H); 5,70 ppm (sl; 1H); 6,74 ppm (dd; 1H; J=2,5-8,5Hz); 7,08 ppm (d; 1H; J=2,5Hz); 7,44 ppm (d; 1H; J=8,5Hz).
- * SM: (IE, 70eV)
  213/211 ($M^+$; 100); 132 (98); 104 (20); 77 (30).

C/ Préparation du 2-(2'-bromo-5'-methoxy)-phényl-2-méthyl-propionitrile:

A une suspension de l'hydrure de sodium NaH (60%; 506 mg; 12,64 mmoles) dans du DMF (15 ml) est ajoutée lentement (6 min.) une solution composée du dérivé précédent (670 mg; 3,16 mmoles) et d'iodométhane (2,7 g; 19 mmoles) dans du DMF (15 ml), à une température de 0°C. L'agitation est poursuivie pendant 10 min. à cette température, puis pendant 2 h. à température ambiante. Le mélange réactionnel est versé avec précaution dans 50 ml d'eau et extrait avec 2x50 ml d'acétate d'éthyle. Les phases organiques sont rassemblées, lavées avec 50 ml d'une solution de chlorure de sodium NaCl saturée, séchées sur $MgSO_4$ et filtrées. Le solvant est évaporé sous pression réduite. Le produit désiré est obtenu sous forme de cristaux jaunes après purification par chromatographie liquide sur une colonne de silice (éluant: cyclohexane-acétate d'éthyle 4/1).
Rendement: 83%
Caractéristiques Physiques:

- * pF°C: 43 °C (rev.)
- * RMN $^{1}H$: ($CDCl_3$)
  1,87 ppm (s; 6H); 3,81 ppm (s; 3H); 6,75 ppm (dd; 1H; J=3,0-8,5Hz); 7,04 ppm (d; 1H; J=3,0Hz); 7,55 ppm (d; 1H; J=8,5Hz).
- * SM: (IE, 70eV)
  255/253 ($M^+$; 100); 240/238 (40); 213/211 (50); 132 (20).

D/ Préparation du 2-(2'-bromo-5'-methoxy)-phényl-2-méthyl-propylamine:

Le dérivé précédent (386 mg; 1,5 mmoles) est dissous sous atmosphère inerte dans du THF (15 ml). Est ajoutée lentement la solution du borane dans le THF (1 M; 3,75 ml; 3,75 mmoles). Le mélange réactionnel est porté au reflux pendant 3 h. Après refroidissement à température ambiante et avec précaution, est ajouté l'acide acétique (solution à 90%; 2 ml). Le mélange est, de nouveau, porté au reflux pour 30 min., puis versé dans 50 ml d'une solution d'acide chlorhydrique HCl (1N) et lavé avec 3x50 ml d'éther terbutylméthylique. La phase aqueuse est alcalinisée (12 < pH < 14), puis extraite par 2x50 ml de dichlorométhane. Les phases organiques sont rassemblées, lavées avec 50 ml d'une solution de chlorure de sodium NaCl saturée, séchées sur $MgSO_4$ et filtrées. Le solvant est évaporé sous pression réduite. Le composé désiré est obtenu sous forme d'une huile jaune.
Rendement: 78%
Caractéristiques Physiques:

* RMN $^1H$: ($CDCl_3$)
  1,35 ppm (sl; 2H); 1,45 ppm (s; 6H); 3,20 ppm (s; 2H); 3,79 ppm (s; 3H); 6,62 ppm (dd; 1H; J=3,0-8,5Hz); 6,97 ppm (d; 1H; J=3,0Hz); 7,49 ppm (d; 1H; J=8,5Hz).
* SM: (IC, isobutane)
  260/258 ($MH^+$; 100); 178 (80).

E/ Préparation du 4,4-diméthyl-6-méthoxy-benzisosélénazine:

Ce composé est obtenu selon une procédure très similaire à celle du dérivé de l'exemple 1/D sous forme des aiguilles légèrement jaunes.
Rendement: 42 %
Caractéristiques Physiques:

* pF°C: 86°C (rev.)
* RMN $^1H$: ($CDCl_3$) 1,27 ppm (s; 6H); 3,23 ppm (s; 2H); 3,40 ppm (sl; 1H); 3,78 ppm (s; 3H); 6,69 ppm (dd; 1H; J=2,5-8,0Hz); 6,93 ppm (d; 1H; J=8,0Hz); 7,03 ppm (d; 1H; J=2,5Hz).
* RMN $^{13}C$: ($CDCl_3$)
  28,60 ppm; 33,40 ppm; 55,64 ppm; 61,50 ppm; 112,54 ppm; 114,59 ppm; 119,90 ppm; 126,03 ppm; 144,49 ppm; 158,31 ppm.
* SM: (IE, 70eV)
  257 ($M^+$; 100); 228 (80); 213 (75); 197 (20);148 (20).

Exemple 12: Préparation du 4,4-diméthyl-6-(2'-(4"-méthyl-piperazine-1"-yl)éthoxy-benzisosélénazine: BXT 51080

A/ Préparation du 2-(2'-bromo-5'-hydroxy)-phényl-2-méthyl-propylamine

Le dérivé 2-(2'-bromo-5'-méthoxy)-phényl-2-méthyl-propylamine de l'exemple 11/D (3,0g; 11,6 mmoles) est dissous sous atmosphère inerte dans du dichlorométhane (11,6ml). La solution de tribromure de bore dans le dichlorométhane (1M; 23,2 mmoles) est ajoutée lentement au milieu réactionnel, à une température de 0°C. L'agitation est poursuivie pendant 15 min. à cette température, puis pendant 45 min. à température ambiante. Après refroidissement à 0°C et avec précaution, est ajouté de l'eau (20ml). Le mélange réactionnel est neutralisé par l'addition de bicarbonate de potassium. Le précipité ainsi obtenu est filtré et lavé avec de l'eau (10ml), puis avec d'éther terbutylméthylique (10ml). Le produit désiré est obtenu après séchage sous forme d'un poudre légèrement marron-gris et est utilisé tel quel pour la prochaine étape.
Rendement: 80%
Caractéristiques Physiques:

* RMN $^1H$: ($CD_3OD$)
  1,56 ppm (s; 6H); 3,56 ppm (si; 2H); 6,65 ppm (dd; 1H; J = 9 - 3 Hz); 6,85 ppm (d; 1H; J = 3Hz); 7,43 ppm (d; 1H; J = 9Hz).
  (DMSO- $d_6$)
  1,48 ppm (s; 6H); 3,34 ppm (s; 2H); 3,4 ppm (si; 2H); 6,67 ppm (dd; 1H; J = 9 - 3 Hz); 6,93 ppm (d; 1H; J = 3 Hz); 7,41 ppm (d; 1H; J = 9 Hz); 7,80 ppm (sl; 1H).
* RMN $^{13}C$: (DMSO- $d_6$)
  25,69 ppm; 38,85 ppm; 46,29 ppm; 109,85 ppm; 116,18 ppm; 117,69 ppm; 136,58 ppm; 142,86 ppm; 157,44 ppm.
* SM: (IC; isobutane)
  246/244 ($MH^+$; 100%); 166 (70%).

B/ Préparation du N-2-(2'-bromo-5'-hydroxy-phényl)-2-méthyl-propyl-N-(*tert*-butyloxycarbonyl)-amine

Le dérivé précédent (340mg; 1,4 mmoles) est dissous à température ambiante dans une solution de soude (1N; 7ml). Le dicarbonate de diterbutyl (670mg; 3 mmoles) est ajouté; puis le mélange réactionnel est agité vigoureusement pendant 1,5 h à une température de 35°C. Le méthanol (8ml) et une solution de soude concentrée (1,12ml) sont ajoutés sequentiellement, et la solution homogène chauffée pendant 1 h à 85°C. Le méthanol est évaporé sous pression réduite, puis le résidu aqueux est neutralisé. Le précipité est filtré, lavé à l'eau (20ml) et séché. Le produit désiré est ainsi obtenu sous forme d'une poudre grise et est utilisé tel quel pour la prochaine étape.
Rendement: 56%
Caractéristiques Physiques:

* RMN $^{1}H$: ($CDCl_3$)
1,39 ppm (s; 6H); 1,41 ppm (s; 9H); 3,69 ppm (d; 2H; J = 7 Hz); 4,30 ppm (t; 1H; J = 7 Hz); 6,59 ppm (dd; 1H; J = 9 - 2,5 Hz); 6,90 ppm (d; 1H; J = 2,5 Hz); 7,38 ppm (d; 1H; J = 9 Hz).

C/ Préparation du 2-(2'-bromo-5'-(2"-chloroéthoxy)-phényl)-2-méthylpropylamine

Le dérivé précédent (565mg; 1,64 mmoles) est dissous à température ambiante dans du THF (10ml). La triphénylphosphine (645mg; 2,46 mmoles), le chloroéthanol (196mg; 160µl; 2 mmoles) et finalement le DEAD (428mg; 390µl; 2,46 mmoles) sont ajoutés. Le mélange réactionnel est porté à reflux pendant 2h. Le solvant est évaporé sous pression réduite. Le résidu est purifié par chromatographie sur colonne de silice (éluant: cyclohexane-acétate d'éthyle 5/1). Le dérivé N-2-(2'-bromo-5'-(2"-chloroéthoxy)-phényl)-2-méthyl-propyl-N-(*tert*-butyloxycarbonyl)-amine, ainsi obtenu sous forme d'une huile incolore (428mg), est dissous dans du dichlorométhane (30ml). Sous agitation, de l'acide trifluoroacétique (3ml) est ajouté, puis l'agitation est maintenue à température ambiante pendant 1h. Le mélange réactionnel est lavé avec une solution saturée de bicarbonate de porassium (2x30ml), séché et filtré. Après évaporation du solvant sous pression réduite, le produit désiré est obtenu sous forme d'une huile jaunâtre.
Rendement: 64%
Caractéristiques Physiques:

* RMN $^{1}H$: ($CDCl_3$)
1,44 ppm (s; 6H); 1,73 ppm (sl; 2H); 3,22 ppm (sl; 2H); 3,79 ppm (t; 2H; J = 6 Hz); 4,18 ppm (t; 2H; J = 6 Hz); 6,60 ppm (dd; 1H; J = 9 - 3 Hz); 6,89 ppm (d; 1H; J = 3 Hz); 7,44 ppm (d; 1H; J = 9 Hz).
* RMN 13C: ($CDCl_3$)
26,49 ppm; 42,09 ppm; 50,34 ppm; 53,73 ppm; 68,45 ppm; 112,26 ppm; 113,77 ppm; 118,58 ppm; 136,99 ppm; 145,15 ppm: 157,93 ppm.

D/ Préparation du 6-(2'-chloroéthoxy)-4,4'-diméthyl-benzisosélénazine

Ce composé est obtenu, selon une procédure très similaire à celle du dérivé de l'exemple 1/D à partir du dérivé précédent, sous forme de cristaux incolores.
Rendement: 48%
Caractéristiques Physiques:

* RMN $^{1}H$: ($CDCl_3$)
1,24 ppm (s; 6H); 3,22 ppm (sl; 3H); 3,77 ppm (t; 2H; J = 6 Hz); 4,18 ppm (t; 2H; J = 6 Hz); 6,66 ppm (dd; 1H; J = 8 - 2,5 Hz); 6,91 ppm (d; 1H; J = 8 Hz); 7,04 ppm (d; 1H; J = 2,5 Hz).
* SM: (IE; 70eV)
305 ($M^+$; 90%); 276 (100%); 213 (60%).

E/ Préparation du 4,4-diméthyl-6-(2'-(4"-méthyl-piperazine-1"-yl)éthoxy)benzisosélénazine: BXT 51080

Le dérivé précédent (86mg; 0,28 mmoles) est dissous dans de la N-méthyl-piperazine (2,83g; 3,15ml; 28 mmoles). Cette solution est chauffée à la température de 60-70°C pendant 22h. Après l'addition de dichlorométhane (50 ml), la solution est lavée avec 25 ml d'une solution saturée de bicarbonate de sodium, puis avec 25 ml d'une solution saturée de chlorure de sodium; séchée sur sulfate de sodium et filtrée. Le solvant est évaporé sous pression réduite. Le produit désiré est obtenu sous forme d'une huile jaune après purification par chromatographie liquide sur une colonne d'oxyde d'alumine (éluant: cyclohexane-acétate d'éthyle 1/1).
Rendement: 71%
Caractéristiques Physiques:

* RMN $^{1}H$: ($CDCl_3$)
1,23 ppm (s; 6H); 2,27 ppm (s; 3H); 2,47 ppm (m; 4H); 2,60 ppm (m; 4H); 2,78 ppm (t; 2H; J = 6 Hz); 3,20 ppm (sl; 2H); 3,34 ppm (sl; 1H); 4,04 ppm (t; 2H; J = 6 Hz); 6,66 ppm (dd; 1H; J = 9 - 2,5 Hz); 6,68 ppm (d; 1H; J = 9 Hz); 7,05 ppm (d; 1H; J = 2,5 Hz).
* RMN $^{13}C$: ($CDCl_3$)
28,54 ppm; 33,36 ppm; 46,30 ppm; 53,85 ppm; 55,30 ppm; 57,51 ppm; 61,46 ppm; 66,25 ppm; 113,27 ppm; 115,27 ppm; 120,09 ppm; 125,96 ppm; 144,45 ppm; 157,46 ppm.
* SM: (IE. 70eV)
369 ($M^+$; 30%); 127 (70%); 113 (100%); 70 (50%).

II/ Exemples d'applications:

Les protocoles opératoires décrits ci-après sont des exemples non limitatifs d'applications du procédé selon l'invention.

EXEMPLE 13: **MESURE DE L'ACTIVITE GLUTATHION PEROXYDASE DE COMPOSES DE STRUCTURE GENERALE I:**

Dans 1,5 ml de tampon HEPES 50 mM, pH=7,3, contenant 0,2 mM de DTPA, 0,144 mM de NADPH, 2,2 mM de glutathion réduit et 1,1 U/ml de glutathion disulfure réductase, et pré-équilibré 2 minutes à 37°C, on ajoute 100 µl d'une solution-mère éthanolique du composé testé ou 100 µl d'éthanol absolu (blanc). Chaque composé est testé à une concentration finale de 20 µM.

Puis après ajout de 50 µl:

- d'hydroperoxyde de tertiobutyle (t-BuOOH) à 6,6 mM dans de l'eau de qualité ultrapure,
- ou de peroxyde d'hydrogène ($H_2O_2$) à 1,6 mM dans de l'eau de qualité ultrapure,
- ou d'hydroperoxyde d'acide linoléique (18:2-OOH), préparé à l'aide de lipoxygénase de soja (voir M.O. Funk et coll.; Lipids; (1976); 11; pages 113-117), à 3,3 mM dans un mélange eau/méthanol, 85/15 (V/V), l'activité glutathion peroxydase est dosée à 37°C en mesurant pendant 5 minutes la diminution d'absorbance à 340 nm. Ladite activité ou vitesse enzymatique initiale est proportionnelle à la pente du décours temporel d'absorbance.

L'activité catalytique de réduction de l'oxygène des composés testés correspond à la vitesse de consommation de NADPH en l'absence d'hydroperoxyde. Lorsque cette vitesse est significativement supérieure à celle du contrôle, l'activité glutathion oxydase correspondante peut être vérifiée par mesure directe de la cinétique de consommation de l'oxygène dissout à l'aide d'une électrode de Clark.

Les résultats d'activité glutathion peroxydase obtenus sont présentés dans le tableau 1. Ils sont exprimés en nmoles d'hydroperoxyde réduit par minute.

A l'examen de ces résultats, on constate que les composés de structure générale I décrits dans la présente invention catalysent, en présence de glutathion GSH, la réduction du peroxyde d'hydrogène ou celle d'un hydroperoxyde organique.

EXEMPLE 14: **MESURE DE L'ACTIVITE REDUCTRICE PAR TRANSFERT MONO-ELECTRONIQUE:**

La capacité des molécules de structure générale I décrites dans la présente invention à catalyser, en présence de glutathion GSH, la réduction par transfert monoélectronique d'entité oxydante est mise en évidence par une mesure spectrophotomètrique de la réduction du cytochrome c ferrique en cytochrome c ferreux, à pH=7,3 et à 37°C, en fonction du temps.

Le milieu réactionnel est constitué par un tampon phosphate de potassium 100 mM (pH=7,3), contenant 75 µM de cytochrome c ferrique, 250 µM de glutathion GSH, 0,1 mM de DTPA, 10 µg/ml de SOD et 110 U/ml de catalase. Après ajout du composé à tester (à 10 µM dans le milieu réactionnel), on mesure pendant 15 minutes l'augmentation d'absorbance à 550 nm.

La vitesse de formation du cytochrome c ferreux est proportionnelle à la pente de la courbe d'absorbance en fonction du temps (V, exprimée en unité d'absorbance par minute), et elle est rapportée à celle observée en présence de glutathion GSH 250 µM et en l'absence du composé testé ($V_{GSH}$), toutes choses étant égales par ailleurs. Les résultats correspondants sont présentés dans le tableau 2.

Ces résultats démontrent que les composés de structure générale I décrits dans la présente invention catalysent en présence de glutathion GSH, en excès, la réduction mono-électronique de molécules dont le pouvoir oxydant est thermodynamiquement supérieur ou égal à celui du cytochrome c ferrique. Etant donné que ces composés ne réduisent pas l'oxygène en présence de glutathion GSH, ils se comportent comme des piégeurs de radicaux libres oxydants et d'initiateurs de réactions en chaine sans production concomittente de formes activées de l'oxygène.

Ceci constitue l'un des avantages majeurs des molécules organoséléniées de structure générale I par opposition à de nombreuses autres molécules organoséléniées qui réduisent l'oxygène en superoxyde cytotoxique.

Aucun effet toxique des composés antioxydants de structure générale I n'est observé sur cellules endothéliales à une concentration inférieure ou égale à 15 µM.

EXEMPLE 15: **PROTECTION DE CELLULES ENDOTHELIALES SOUMISES A UN STRESS OXYDANT INDUIT PAR L'HYDROPEROXYDE D'ACIDE LINOLEIQUE:**

Des cellules endothéliales humaines sont cultivées à 37°C dans des tubes de Leighton et sous une atmosphère saturée d'humidité constituée d'un mélange gazeux à 95% d'air et 5% de $CO_2$. Leur milieu de culture est constitué par un milieu EGM (Clonetics) pH=7,4 contenant notamment 2% de sérum de veau foetal, 10 ng/ml du facteur de croissance EGF humain recombinant, 10 µg/ml d'héparine, 50 µg/ml de Gentamicine et 50 µg/ml d'Amphotéricine B.

Lorsque les cellules sont proches de la confluence, elles sont incubées pendant une heure en présence ou non (contrôle) du composé de structure générale I à tester, incorporé dans le milieu de culture à une concentration comprise entre 1 et 10 µM, toutes choses étant égales par ailleurs. Puis, après trois lavages successifs des cellules avec un tampon PBS, celles-ci sont ensuite incubées en présence ou non (contrôle) d'hydroperoxyde d'acide linoléique à 55 µM dans le milieu de culture. Au bout d'une heure d'incubation, les cellules sont lavées trois fois avec du tampon PBS et elles sont colorées selon la procédure de May-Grunwald/Giemsa qui fait apparaitre le noyau en mauve et le cytoplasme en violet clair.

A l'examen sous microscope des lames obtenues, on observe des cellules témoins adhérentes dont la morphologie est caractéristique de cellules endothéliales. L'effet de l'hydroperoxyde d'acide linoléique sur ces cellules consiste, d'une part, en une diminution de 25 à 35% de la densité cellulaire et, d'autre part, en une modification morphologique drastique d'environ 70% des cellules restantes, dont les noyaux ne sont plus distinguables du reste du corps cellulaire qui s'est condensé et présente une coloration violet-noire intense. Des expériences d'exclusion du bleu trypan (colorant vital), faites par ailleurs, indiquent que plus de 30% de ces cellules sont mortes. En revanche, si préalablement au traitement à l'hydroperoxyde d'acide linoléique on incube les cellules avec un composé de formule générale I, on retrouve au moins 80% de cellules endothéliales morphologiquement normales.

Ces résultats mettent donc en évidence que les composés de formule générale I décrits dans la présente invention sont captés par les cellules endothéliales qu'ils protègent contre les effets délétères d'un hydroperoxyde d'acide gras.

EXEMPLE 16: **PROTECTION DE CELLULES ENDOTHELIALES SOUMISES A UN STRESS OXYDANT INDUIT PAR LE PEROXYDE D'HYDROGENE:**

Des cellules endothéliales humaines sont cultivées à 37°C dans des plaques 6 puits ou des boites de Petri (diamètre 35 mm) sous une atmosphère saturée d'humidité constituée d'un mélange gazeux à 95% d'air et 5% de $CO_2$. Leur milieu de culture est constitué par un milieu M 199 pH=7,4 contenant 20% de sérum de veau foetal, 2 mM de L-glutamine, 100 U/ml de pénicilline et 100 µg/ml de Streptomycine.

Lorsque les cellules sont confluentes, elles sont incubées en présence ou non (contrôle) d'un composé de structure générale I dont la concentration dans le milieu de culture est comprise entre 1 et 10 µM, toutes choses étant égales par ailleurs. Puis, après trois lavages successifs des cellules avec un tampon PBS,

celles-ci sont incubées en présence ou non (contrôle) de peroxyde d'hydrogène à 500 ou 100 µM dans le milieu réactionnel. Les cellules sont ensuite lavées trois fois avec du tampon PBS. Elles sont alors soit colorées selon la procédure de May-Grunwald/Giemsa, qui fait apparaitre le noyau en mauve et le cytoplasme en violet clair, soit utilisées pour mesurer leur capacité à produire l'oxyde nitrique NO-(EDRF). La mesure de la production de NO· par les cellules endothéliales est effectuée à l'aide d'une électrode à NO·, après stimulation par la bradykinine 100 nM, selon la procédure décrite par H. Tsukahara et coll. (Biochem. and Biophys. Res. Comm.; (1993); 193; pages 722-729).

A l'examen sous microscope des plaques obtenues après coloration, on observe des cellules témoins adhérentes dont la morphologie est caractéristique de cellules endothéliales. L'effet du peroxyde d'hydrogène à 500 µM consiste essentiellement en une diminution de la densité cellulaire de 70 à 80%. En revanche, lorsque les cellules sont préalablement traitées avec un composé de formule générale I comme décrit précédemment dans l'exemple 7, plus de 50% de la densité cellulaire est conservée. A titre d'exemple, environ 70% de la densité cellulaire est conservée lorsque les cellules sont préalablement incubées avec le composé BXT-51056.

Le traitement par le peroxyde d'hydrogène 100 µ M n'a pas d'effet significatif sur la densité cellulaire. Les cellules apparaissent morphologiquement normales à l'examen sous microscope. Ce traitement conduit par contre à une altération de la production de NO· de plus de 90%. En revanche, le pré-traitement des cellules endothéliales par un composé de formule générale I comme décrit précédemment, à une concentration comprise entre 0,2 et 1 µM, conduit à une récupération significative de la réponse normale à la bradykinine. A titre d'exemple, le pré-traitement des cellules par le composé BXT-51056 à une concentration de 1 µM conduit à une récupération de plus de 80% de la réponse normale à la bradykinine.

Ces résultats mettent donc en évidence que les composés de formule générale I décrits dans la présente invention sont captés par les cellules endothéliales qu'ils protègent contre les effets délétères du peroxyde d'hydrogène.

EXEMPLE 17: **PROTECTION DE CELLULES ENDOTHELIALES SOUMISES A UN STRESS OXYDANT INDUIT PAR DES POLYMORPHONUCLEAIRES NEUTROPHILES EN PRESENCE DE TNF-α:**

Des cellules endothéliales humaines sont cultivées à 37°C dans des flacons de 25 $cm^2$ sous une atmosphère saturée d'humidité constituée d'un mélange gazeux à 95% d'air et 5% de $CO_2$. Leur milieu de culture est constitué par un milieu EGM (Clonetics) pH=7,4 contenant notamment 2% de sérum de veau foetal, 10 ng/ml du facteur de croissance EGF humain recombinant, 10 μg/ml d'héparine, 50 μg/ml de Gentamicine et 50 μg/ml d'Amphotéricine B.

Lorsque les cellules sont proches de la confluence, elles sont incubées pendant une heure en présence ou non (contrôle) du composé de structure générale I à tester, incorporé dans le milieu de culture à une concentration comprise entre 1 et 10 μM, toutes choses étant égales par ailleurs. Après trois lavages successifs des cellules avec un tampon PBS, celles-ci sont incubées en présence ou non (contrôle) de TNF-α (à 100 U/ml dans le milieu de culture) et de polymorphonucléaires neutrophiles (PMN), dans un rapport d'environ 10 PMN pour 1 cellule endothéliale, préparés à partir de sang humain hépariné ( voir A. Boyum; Scand. J. Clin. Lab. Invest.; (1968); 21; pages 77-89). Au bout de 4 heures d'incubation, les cellules sont lavées trois fois avec du tampon PBS et elles sont colorées selon la procédure de May-Grunwald/Giemsa qui fait apparaitre le noyau en mauve et le cytoplasme en violet clair.

A l'examen sous microscope des flacons colorés obtenus, on observe des cellules témoins adhérentes dont la morphologie est caractéristique de cellules endothéliales. L'effet des PMN sur ces cellules,en présence de TNF-α, consiste en une diminution de 30 à 40% de la densité des cellules endothéliales et une modification drastique de plus de 70% de ces cellules restant adhérées, dont les noyaux ne sont plus distinguables du reste du corps cellulaire qui s'est condensé et présente une coloration violet-noire intense. Dans ce cas, on observe également la présence de très nombreux PMN adhérés sur le support et en particulier contre la face externe de la membrane plasmique de ces cellules endothéliales. En revanche, si préalablement à l'incubation des cellules endothéliales avec les PMN et le TNF-α on incube ces cellules en présence d'un composé de formule générale I, on retrouve majoritairement des cellules endothéliales morphologiquement normales et dépourvues de PMN adhérents.

Ces résultats mettent donc en évidence que les composés de formule générale I décrits dans la présente invention sont captés par les cellules endothéliales qu'ils protègent contre les effets délétères des polymorphonucléaires neutrophiles activés.

EXEMPLE 18: **TEST DE VIABILITE EFFECTUE SUR DES CELLULES ENDOTHELIALES SOUMISES A UN STRESS OXYDANT INDUIT PAR L'HYDROPEROXYDE D'ACIDE LINOLEIOUE**

Des cellules endothéliales humaines sont cultivées à 37°C dans des boites de Pétri ou des plaques multi-puits sous une atmosphère saturée d'humidité constituée d'un mélange gazeux à 95% d'air et 5% de $CO_2$. Leur milieu de culture est constitué par un milieu M 199 pH=7,4 contenant 20% de sérum de veau foetal, 2 mM de L-glutamine, 100 U/ml de pénicilline, 100 μg/ml de Streptomycine et 1% en volume d'un supplément de milieu contenant de l'héparine et un facteur de croissance pour ces cellules.

Lorsque les cellules sont proches de la confluence, elles sont incubées pendant une heure en présence ou non d'un des composés suivants: BXT-51056, BXT-51072,

BXT-51077 ou ebselen. Chacun de ces composés est incorporé à 4 μM dans le milieu de culture contenant 2% de sérum de veau foetal, toutes choses étant égales par ailleurs.

Après trois lavages successifs des cellules avec un tampon PBS, celles-ci sont incubées en présence ou non (contrôle) d'hydroperoxyde d'acide linoléique (18:2-OOH) à 50 μM dans le milieu de culture défini précédemment. Au bout de deux heures d'incubation, les cellules sont lavées trois fois avec un tampon PBS et elles sont mises à nouveau à incuber dans le milieu de culture à 20% de sérum de veau foetal défini initialement. Au bout d'environ vingt heures, la viabilité des cellules est détermminée par une mesure au bromure de 3-(4,5-dimethylthiazol-2-yl)-2,5-diphenyl-2H-tctrazolium (MTT).

Les résultats obtenus sont présentés sur la figure 1, où ils sont exprimés en pourcentage de survie par rapport au contrôle et où le témoin d'altération est illustré par le rectangle noir. On constate que parmi les composés testés, seuls les BXT-51056, BXT-51072 et BXT-51077 protègent significativement les cellules de la mort induite par le 18:2-OOH.

Comme dans l'exemple 15, ces résultats démontrent que les composés de structure générale I décrits dans la présente invention sont captés par les cellules endothéliales qu'ils protègent contre les effets délétères d'un hydroperoxyde d'acide gras.

EXEMPLE 19: **EFFET PROTECTEUR DES COMPOSES BXT-51056, BXT-51072 ET BXT-51077: QUANTIFICATION BIOCHIMIQUE DES MODIFICATIONS ENDOTHELIALES INDUITES PAR DES POLYMORPHONUCLEAIRES NEUTROPHILES EN PRESENCE DE TNF-α**

Des cellules endothéliales humaines sont cultivées dans les mêmes conditions que celles décrites dans

l'exemple 18.
Lorsque les cellules sont proches de la confluence, elles sont incubées pendant une heure en présence ou non d'un des composés suivants: BXT-51072, BXT-51077 ou pentoxifylline. Les composés BXT-51072 et BXT-51077, chacun à 10 µM, et la pentoxifylline, à 50 µM, sont incorporés dans le milieu de culture contenant 2% de sérum de veau foetal, toutes chose étant égales par ailleurs. Puis les cellules sont lavées avec un tampon PBS. Les cellules non pré-traitées avec les composés testés sont alors incubées ou non (contrôle) en présence de TNF-α, à 1 ng/ml, et/ou de polymorphonucléaires neutrophiles (PMN), comme indiqué dans l'exemple 15, dans le milieu de culture à 2% de sérum de veau foetal. Les cellules qui ont été pré-traitées sont incubées dans les mêmes conditions, mais le milieu de culture contient en outre le composé testé à la concentration définie précédemment. Au bout d'une heure, d'une part, et de trois heures et demi d'incubation, d'autre part, le milieu de culture est collecté, le tapis cellulaire est lavé trois fois avec un tampon PBS et les PMN adhérés sont lysés. La myeloperoxydase (MPO), enzyme contenue spécifiquement dans les PMN est alors dosée dans les lysats cellulaires obtenus afin d'évaluer l'adhésion des PMN sur les cellules endothéliales. Le facteur von Willebrandt (vWf) libéré dans le milieu de culture par les cellules endothéliales est dosé au bout des trois heures et demi d'incubation, comme marqueur d'altération de ces cellules.
L'incubation des cellules endothéliales en présence de PMN et de TNF-a conduit à une augmentation très significative de la quantité de MPO et de vWf par rapport aux contrôles respectifs (PMN seuls, cellules endothéliales seules respectivement), traduisant donc l'adhésion des PMN activés sur les cellules endothéliales et l'altération de celles-ci.
Les résultats obtenus pour les trois composés testés sont présentés sur la figure 2. Ils sont exprimés en pourcentage d'inhibition en rapportant les mesures de MPO et de vWf aux concentrations respectives de MPO et de vWf déterminées lorsque les cellules endothéliales sont incubées avec le TNF-a et les PMN.
Ces résultats montrent que les composés BXT-51072 et BXT-51077 inhibent l'adhésion des PMN induite par le TNF-α, et ceci beaucoup plus efficacement que la pentoxifylline. A l'inverse de la pentoxifylline, ils protègent également les cellules endothéliales des effets délétères des PMN activés.

EXEMPLE 20: **EFFET PROTECTEUR DES COMPOSES BXT-51072 ET BXT-51077: QUANTIFICATION BIOCHIMIQUE DE LA PRODUCTION ENDOTHELIALE D'INTERLEUKINE 8 INDUITE PAR LE TNF-α**

Des cellules endothéliales humaines sont cultivées dans les mêmes conditions que celles décrites dans l'exemple 18.
Lorsque les cellules sont proches de la confluence, elles sont incubées pendant une heure en présence ou non d'un des composés suivants: BXT-51072, BXT-51077 ou ebselen. Chacun de ces composés est incorporé à 10 µM dans le milieu de culture contenant 2% de sérum de veau foetal, toutes chose étant égales par ailleurs. Après élimination du milieu de culture, les cellules sont mises à incuber en présence ou non (contrôle) de TNF-α, à 0,1, 1 ou 10 ng/ml dans le même milieu de culture que précédemment, contenant ou non le composé testé à 10 µM. Au bout de quatre heures d'incubation, l'interleukine 8 (IL-8) est dosée dans le milieu de culture.
Les résultats obtenus sont présentés sur la figure 3. Ces résultats montrent que l'incubation des cellules en présence de TNF-α conduit à une augmentation de la production d'IL-8 dans le milieu de culture, et que le traitement des cellules par les composés BXT-51072 et BXT-51077 inhibe cet effet d'au moins 70%, alors que l'ebselen n'a pas d'effet inhibiteur.
Ces résultats démontrent que de tels composés peuvent agir comme des antagonistes du TNF-α en terme de libération d'interleukine 8 par les cellules endothéliales.

EXEMPLE 21: **PROTECTION DE CELLULES ENDOTHELIALES CONTRE LA TOXICITE DE L'INTERLEUKINE 1**

Des cellules endothéliales humaines sont cultivées dans les mêmes conditions que celles décrites dans l'exemple 18.
Lorsque les cellules sont proches de la confluence, elles sont incubées pendant une heure en présence ou non du composé BXT-51072 ou BXT-51077, chacun à 10 µM dans le milieu de culture contenant 2% de sérum de veau foetal, toutes chose étant égale par ailleurs. Les cellules sont ensuite lavées trois fois avec un tampon PBS. Celles qui n'ont pas été pré-traitées par un composé sont mises à incuber en présence ou non (contrôle) d'interleukine 1α (IL-1α), à 50 U/ml, dans le même milieu de culture. Les cellules qui ont été pré-traitées sont incubées dans les mêmes conditions, mais le milieu contient ou non en outre le composé testé à la concentration indiquée précédemment. Au bout de quatre heures d'incubation, le facteur von Willebrandt (vWf) est dosé dans le milieu de culture.
Les résultats obtenus sont présentés sur la figure 4. Ces résultats montrent que l'augmentation de la libération de vWf induite par l'IL-1 est inhibée de plus de 70% en traitant les cellules par le composé BXT-51072 ou BXT-51077, que chacun d'eux soit présent ou non lors de l'incubation des cellules avec l'interleukine 1.
Ces résultats démontrent que de tels composés sont captés par les cellules endothéliales et qu'ils peuvent agir comme des antagonistes de l'interleukine 1 en terme de libération de facteur von Willebrandt par les cellules endothéliales.

EXEMPLE 22: **INHIBITION DE L'EXPRESSION ENDOTHELIALE DE LA P-SELECTINE INDUITE PAR LE TNF-α**

Des cellules endothéliales humaines sont cultivées dans les mêmes conditions que celles décrites dans l'exemple 18.
Lorsque les cellules sont proches de la confluence, elles sont incubées pendant une heure en présence ou non du composé BXT-51072 à 10 µM dans le milieu de culture contenant 2% de sérum de veau foetal, toutes chose étant égale par ailleurs.
Les cellules sont ensuite lavées trois fois avec un tampon PBS. Celles qui n'ont pas été pré-traitées par un composé sont mises à incuber en présence ou non (contrôle) de TNF-α, à 1 ng/ml, dans le même milieu de culture. Les cellules pré-traitées sont incubées dans les mêmes conditions, mais le milieu contient en outre le composé BXT-51072 à 10 µM. Au bout de 3 à 4 heures d'incubation, les cellules sont lavées avec du tampon PBS et elles sont fixées avec du formaldéhyde à 2% dans un tampon PBS. L'expression de P-sélectine sur les cellules est alors mesurée par un dosage ELISA en incubant successivement les cellules en présence d'un anticorps monoclonal de souris anti-P-sélectine et d'un anti-anticorps de souris marqué à la phosphatase alcaline, et en révélant par l'ajout de paranitrophényl phosphape dont l'hydrolyse est suivie à 405 nm.Les résultats obtenus montrent que l'incubation des cellules en présence de TNF-α induit une augmentation d'un facteur 4 de l'expression de P-sélectine, qui est inhibée de plus de 90% quand les cellules sont traitées avec le composé BXT-51072.
Ces résultats démontrent que de tels composés sont capables d'inhiber l'expression d'une molécule d'adhésion cellulaire telle que la P-sélectine, induite par le TNF-α.

EXEMPLE 23: **ABSENCE DE TOXICITE DES COMPOSES BXT-51056 ET BXT-51072 SUR CULTURE DE CELLULES ENDOTHELIALES IN VITRO**

Des cellules endothéliales humaines sont cultivées dans les mêmes conditions que celles décrites dans l'exemple 18, sur des plaques 96 puits.
Lorsque les cellules sont proches de la confluence, elles sont incubées ou non (contrôle) pendant 24 ou 48 heures en présence du composé BXT-51056 ou BXT-51072 incorporé dans le milieu de culture à une concentration inférieure ou égale à 10 µM. Au bout de 24 ou 48 heures, les cellules sont rincées trois fois avec un tampon PBS et la viabilité des cellules est mesurée au bromure de 3-(4,5-dimethylthiazol-2-yl)-2,5-diphenyl-2H-tetrazolium (MTT).
Aucune différence significative n'est mesurée par rapport, démontrant que les composés BXT-51056 et BXT-51072 ne sont pas toxiques pour les cellules endothéliales, à une concentration inférieure ou égale à 10 µM.

EXEMPLE 24: **TOLERANCE DU COMPOSE BXT-51072 APRES ADMINISTRATION ORALE REPETEE CHEZ LE RAT IN VIVO**

Des rats mâles adultes Sprague-Dawley sont répartis de façon aléatoire en 4 groupes. A un groupe de 10 rats et à un autre groupe de 15 rats est administrée par voie orale (gavage) chaque jour, pendant 14 jours, respectivement une dose de 10 et une dose de 100 µmoles du composé BXT-51072 par kilogramme de poids corporel. Ces doses sont administrées à l'aide d'une solution aqueuse contenant 1% de carboxyméthylcellulose de sodium (CMC).
Selon le même protocole, un troisième groupe composé de 12 rats reçoit uniquement le vecteur (CMC à 1%) et un quatrième groupe de 3 rats ne subit aucun gavage.
Afin d'évaluer l'éventuelle toxicité du composé testé, tous les animaux sont pesés régulièrement pendant les 14 jours. Leur apparence et leur comportement sont observés quotidiennement.
A l'issue des 14 jours de traitement, aucun mort n'est compté parmi les animaux. Environ 16 heures après la dernière administration, les rats sont anesthésiés à la kétamine et un prélèvement sanguin est effectué par ponction intracardiaque afin de mesurer différents paramètres hématologiques et biochimiques. Les courbes de poids des animaux sont présentées sur les figures 5 et 6 et le tableau 3 récapitule les résultats des mesures effectuées dans le sang des animaux et les écarts-types correspondant.
Malgré un léger ralentissement de la croissance pondérale à la dose la plus élevée du composé BXT-51072 de l'invention (100 µmoles/kg, figure 6), aucun signe de toxicité n'est par ailleurs mis en évidence (tableau 3). Ces résultats démontrent que le composé BXT-51072 de l'invention est bien toléré chez le rat in vivo après administration orale répétée.

TABLEAU 1

| **Activité glutathion peroxydase** (en nmoles d'hydroperoxyde réduit/min) pH = 7,3; 37°C; [GSH] = 2 mM | | | |
|---|---|---|---|
| | t-BuOOH | $H_2O_2$ | 18:2-OOH |
| BXT-51056 (Ex. 1) | 8,8 | 12 | 29,9 |
| BXT-51058 (Ex. 3) | 3,1 | 2,1 | 22,9 |
| BXT-51072 (Ex. 8) | 27,4 | 42,0 | - |
| BXT-51075 (Ex. 6) | 3,2 | 5,9 | - |
| BXT-51076 (Ex. 7) | 4,5 | 6,6 | - |
| BXT-51077 (Ex. 11) | 35,4 | - | - |
| BXT-51078 (Ex. 10) | 16,5 | - | - |

TABLEAU 2

| | $V/V_{GSH}$ |
|---|---|
| BXT-51056 (Ex. 1) | 4,8 |
| BXT-51057 (Ex. 2) | 1,5 |

TABLEAU 3

| | | HEMATOLOGIE | | | | | | | | | | | BIOCHIMIE | | | | | | | | |
|---|---|---|---|---|---|---|---|---|---|---|---|---|---|---|---|---|---|---|---|---|---|
| | n | *GR* [T/l] | *HB* [mM] | *HCT* | *VGM* [fl] | *GB* [G/l] | *NEU* [%] | *EOS* [%] | *BAS* [%] | *LYM* [%] | *MON* [%] | *PLT* [G/l] | *Na⁺* [mM] | *K⁺* [mM] | *Cl⁻* [mM] | *Urée* [mM] | *Prot* [g/l] | *PAL* [UI/l] | *ALAT* [UI/l] | *ASAT* [UI/l] |
| CONTROLE (- CMC) | 3 | 6,79<br>(0,16) | 9,11<br>(0,25) | 0,41<br>(0,01) | 60,5<br>(1,39) | 8,83<br>(2,05) | 12<br>(2) | 0 | 0 | 88<br>(2) | 0 | 654<br>(146) | 143<br>(0)<br>*n=2* | 4,7<br>(0,2)<br>*n=2* | 94<br>(0,6) | 3,65<br>(0,29) | 55,1<br>(2,5) | 314<br>(91) | 63<br>(12) | 145<br>(15) |
| CONTROLE (+ CMC) | 6 | 6,76<br>(0,29) | 8,90<br>(0,20) | 0,40<br>(0,01) | 60,0<br>(2,36) | 8,83<br>(2,16) | 12<br>(3) | 0 | 0 | 87<br>(3) | 1<br>(0,4) | 682<br>(197) | 144<br>(0,6)<br>*n=3* | 4,7<br>(0,5)<br>*n=3* | 95<br>(0,8) | 4,73<br>(0,83) | 54,8<br>(2,1) | 334<br>(70) | 58<br>(6) | 124<br>(29) |
| BXT-51072 10 (µmoles/kg) | 4 | 6,88<br>(0,36) | 9,08<br>(0,31) | 0,41<br>(0,01) | 59,8<br>(2,36) | 8,45<br>(1,99) | 18<br>(5) | 0 | 0 | 82<br>(5) | 0 | 541<br>(280) | 143<br>*n=1* | 5,0<br>*n=1* | 93<br>(1,4)<br>*n=2* | 4,03<br>(0,68) | 53,9<br>(0,4)<br>*n=2* | 320<br>(84) | 50<br>(9)<br>*n=3* | 136<br>(60)<br>*n=3* |
| 100 (µmoles/kg) | 7 | 7,44<br>(0,40) | 9,46<br>(0,46) | 0,43<br>(0,02) | 57,6<br>(1,99) | 7,57<br>(1,83) | 18<br>(7) | 1<br>(0,8) | 0 | 81<br>(8) | 0 | 517<br>(200) | 143<br>(0)<br>*n=2* | 5,0<br>(0,1)<br>*n=2* | 93<br>(1,3)<br>*n=4* | 4,05<br>(0,43)<br>*n=5* | 55,0<br>(2,6)<br>*n=4* | 310<br>(66)<br>*n=5* | 58<br>(12)<br>*n=4* | 133<br>(18)<br>*n=4* |

Abréviations: *GR*, Globules Rouges; *HB*, Hémoglobine; *HCT*, Hématocrite; *VGM*, Volume Globulaire Moyen des GR; *GB*, Globules Blancs; *NEU*, Neutrophiles; *EOS*, Eosinophiles; *BAS*, Basophiles; *LYM*, Lymphocytes; *MON*, Monocytes; *PLT*, Plaquettes; *Prot*, Protéines; *PAL*, Phosphatase Alcaline; ALAT, Alanine Aminotransférase; *ASAT*, Aspartate Aminotransférase.

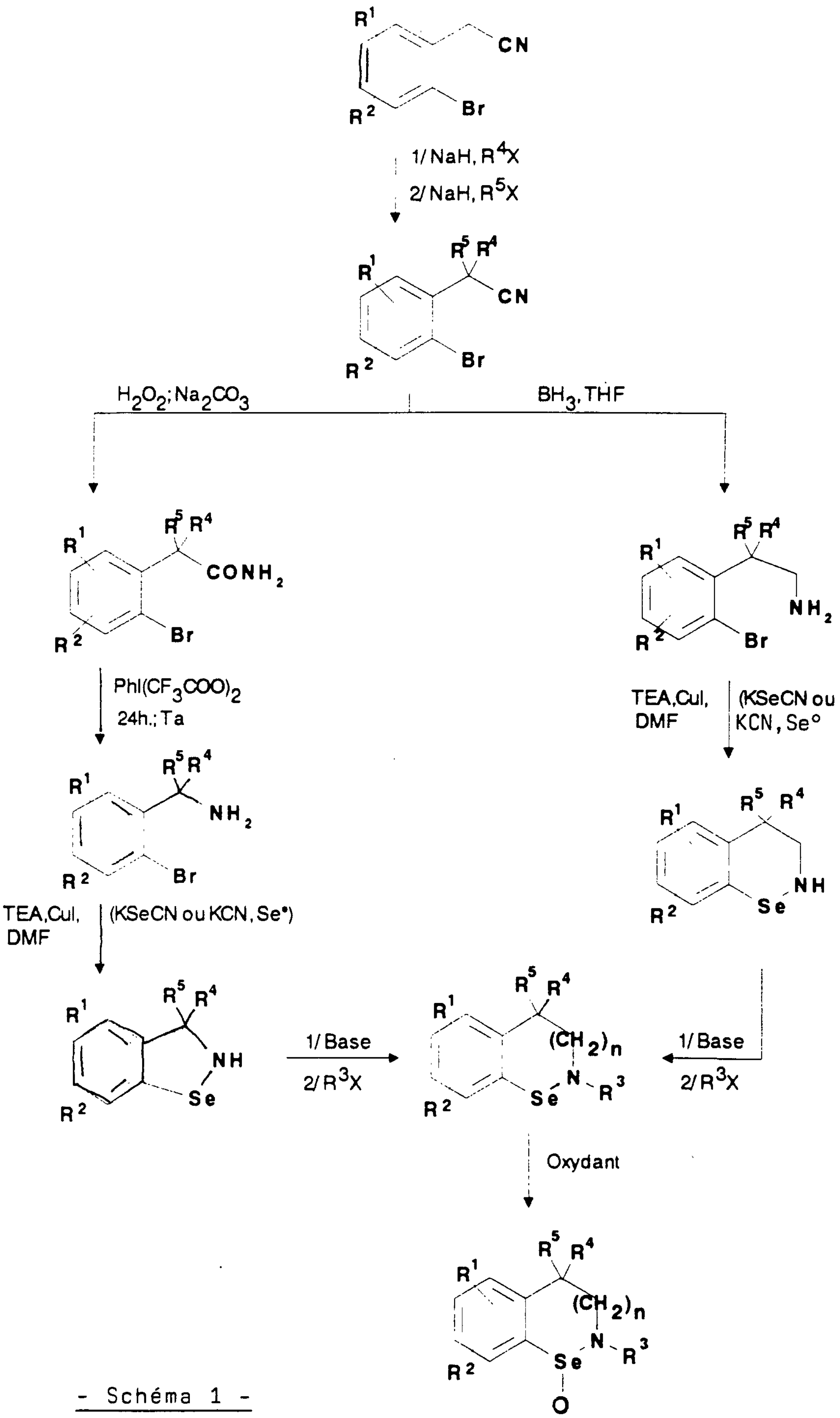
1/ NaH, R4X
2/ NaH, R5X
H2O2; Na2CO3
BH3, THF
PhI(CF3COO)2
24h.; Ta
TEA,CuI, DMF
(KSeCN ou KCN, Se°)
TEA,CuI, DMF
(KSeCN ou KCN, Se°)
1/ Base
2/ R3X
1/ Base
2/ R3X
Oxydant

- Schéma 1 -

## Revendications

**1.** Composés benzisosélén-azoline et -azine répondant à la formule générale (II) suivante:

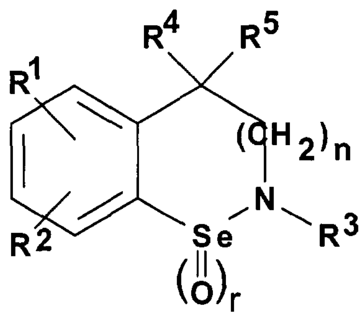

**Formule Générale II**

dans laquelle:

$R^1$ = hydrogène; alkyle inférieur, linéaire ou ramifié, comprenant de 1 à 6 atomes de carbone; $-OR^6$; $-(CH_2)_mNR^6R^7$; $-(CH_2)_qNH_2$; $-(CH_2)_mNHSO_2(CH_2)_2NH_2$; $-NO_2$; -CN; $-SO_3H$; $-N^+(R^5)_2O^-$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^2$ = hydrogène; alkyle inférieur, linéaire ou ramifié, comprenant de 1 à 6 atomes de carbone; $-OR^6$; $-(CH_2)_mNR^6R^7$; $-(CH_2)_qNH_2$; $-(CH_2)_mNHSO_2(CH_2)_2NH_2$; $-NO_2$; -CN; $-SO_3H$; $-N^+(R^5)_2O^-$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^3$ = hydrogène; alkyle inférieur, linéaire ou ramifié, comprenant de 1 à 6 atomes de carbone; aralkyle, dans lequel alkyle est linéaire ou ramifié comprenant de 1 a 6 atomes de carbone; aralkyle substitué, le terme substitué affectant le groupement aralkyle, signifie que celui-ci est substitué sur la partie aromatique par un ou plusieurs groupements identiques ou différents choisis parmi alkyle inférieur linéaire ou ramifié comprenant de 1 à 6 atomes de carbone, trifluorométhyl, alkoxyl inférieur, linéaire ou ramifié comprenant de 1 à 6 atomes de carbone, hydroxyl, nitro, amino, (alkyl, linéaire ou ramifié en $C_{1-6}$) amino; di-(alkyl, linéaire ou ramifié en $C_{1-6}$) amino; sulfonyl; sulfonamide; sulfo(alkyl, linéaire ou ramifié en $C_{1-6}$); carboxyl; carb(alkoxyl, linéaire ou ramifié en $C_{1-6}$); ou par 1 ou plusieurs atomes d'hydrogène; $-(CH_2)_mCOR^8$; $-(CH_2)_qR^8$; $-CO(CH_2)_pCOR^8$; $-(CH_2)_mSO_2R^8$; $-(CH_2)_mS(O)R^8$;

$R^4$ = alkyle inférieur, linéaire ou ramifié, comprenant de 1 à 6 atomes de carbone; aralkyle, dans lequel alkyle est linéaire ou ramifié comprenant de 1 a 6 atomes de carbone; aralkyle substitué, le terme substitué affectant le groupement aralkyle, signifie que celui-ci est substitué sur la partie aromatique par un ou plusieurs groupements identiques ou différents choisis parmi alkyle inférieur linéaire ou ramifié comprenant de 1 à 6 atomes de carbone, trifluorométhyl, alkoxyl inférieur, linéaire ou ramifié comprenant de 1 à 6 atomes de carbone, hydroxyl, nitro, amino, (alkyl, linéaire ou ramifié en $C_{1-6}$) amino; di-(alkyl, linéaire ou ramifié en $C_{1-6}$) amino; sulfonyl; sulfonamide; sulfo(alkyl, linéaire ou ramifié en $C_{1-6}$); carboxyl; carb(alkoxyl, linéaire ou ramifié en $C_{1-6}$); ou par 1 ou plusieurs atomes d'hydrogène; $-(CH_2)_pCOR^8$; $-(CH_2)_pR^8$; F;

$R^5$ = alkyle inférieur, linéaire ou ramifié, comprenant de 1 à 6 atomes de carbone; aralkyle, dans lequel alkyle est linéaire ou ramifié comprenant de 1 a 6 atomes de carbone; aralkyle substitué, le terme substitué affectant le groupement aralkyle, signifie que celui-ci est substitué sur la partie aromatique par un ou plusieurs groupements identiques ou différents choisis parmi alkyle inférieur linéaire ou ramifié comprenant de 1 à 6 atomes de carbone, trifluorométhyl, alkoxyl inférieur, linéaire ou ramifié comprenant de 1 à 6 atomes de carbone, hydroxyl, nitro, amino, (alkyl, linéaire ou ramifié en $C_{1-6}$) amino; di-(alkyl, linéaire ou ramifié en $C_{1-6}$) amino; sulfonyl; sulfonamide; sulfo(alkyl, linéaire ou ramifié en $C_{1-6}$); carboxyl; carb(alkoxyl, linéaire ou ramifié en $C_{1-6}$); ou par 1 ou plusieurs atomes d'hydrogène;

$R^6$ = alkyle inférieur, linéaire ou ramifié, comprenant de 1 à 6 atomes de carbone; aralkyle, dans lequel alkyle est linéaire ou ramifié comprenant de 1 a 6 atomes de carbone; aralkyle substitué, le terme substitué affectant le groupement aralkyle, signifie que celui-ci est substitué sur la partie aromatique par un ou plusieurs groupements identiques ou différents choisis parmi alkyle inférieur linéaire ou ramifié comprenant de 1 à 6 atomes de carbone, trifluorométhyl, alkoxyl inférieur, linéaire ou ramifié comprenant de 1 à 6 atomes de carbone, hydroxyl, nitro, amino, (alkyl, linéaire ou ramifié en $C_{1-6}$) amino; di-(alkyl, linéaire ou ramifié en $C_{1-6}$) amino; sulfonyl; sulfonamide; sulfo(alkyl, linéaire ou ramifié en $C_{1-6}$); carboxyl; carb(alkoxyl, linéaire ou ramifié en $C_{1-6}$); ou par 1 ou plusieurs atomes d'hydrogène; $-(CH_2)_mCOR^8$; $-(CH_2)_qR^8$;

$R^7$ = alkyle inférieur, linéaire ou ramifié, comprenant de 1 à 6 atomes de carbone; aralkyle, dans lequel alkyle est linéaire ou ramifié comprenant de 1 a 6 atomes de carbone; aralkyle substitué, le terme substitué affectant le groupement aralkyle, signifie que celui-ci est substitué sur la partie aromatique par un ou plusieurs groupements identiques ou différents choisis parmi alkyle inférieur linéaire ou ramifié comprenant de 1 à 6 atomes de carbone, trifluorométhyl, alkoxyl inférieur, linéaire ou ramifié comprenant de 1 à 6 atomes de carbone, hydroxyl, nitro, amino, (alkyl, linéaire ou ramifié en $C_{1-6}$) amino; di-(alkyl, linéaire ou ramifié en $C_{1-6}$) amino; sulfonyl; sulfonamide; sulfo(alkyl, linéaire ou ramifié en $C_{1-6}$); carboxyl; carb(alkoxyl, linéaire ou ramifié en $C_{1-6}$); ou par 1 ou plusieurs atomes d'hydrogène; $-(CH_2)_mCOR^8$;

$R^8$ = alkyle inférieur, linéaire ou ramifié comprenant de 1 à 6 atomes de carbone; aryle; aralkyle dans lequel alkyle est linéaire ou ramifié comprenant de 1 a 6 atomes de carbone; hétéroaryle, signifiant tout noyau aromatique mono ou bicyclique, chaque cycle comprenant de 5 à 6 sommets et incluant dans son squelette carboné 1 ou éventuellement plusieurs hétéroatomes identiques ou différents choisis parmi azote, oxygène ou soufre; aralkyle substitué; aryle substitué, hétéroaryle substitué; le terme substitué affectant un groupement aralkyle, aryle ou hétéroaryle signifiant que celui-ci est substitué sur la partie aromatique par un ou plusieurs groupements identiques ou différents choisis parmi alkyle inférieur, linéaire ou ramifié comprenant de 1 à 6 atomes de carbone, trifluorométhyl, alkoxyl inférieur, linéaire ou ramifié comprenant de 1 à 6 atomes de carbone, hydroxyle, nitro, amino, (alkyle, linéaire ou ramifié comprenant de 1 à 6 atomes de carbone) amino; di (alkyl, linéaire ou ramifié comprenant de 1 à 6 atomes de carbone) amino; sulfonyl; sulfonamide; sulfo(alkyle, linéaire ou ramifié comprenant de 1 à 6 atomes de carbone); carboxyle; carb(alkoxyle, linéaire ou ramifié, comprenant de 1 à 6 atomes de carbone); ou par 1 ou plusieurs atomes d'hydrogène; hydroxyle; alkoxyle inférieur, linéaire ou ramifié ayant de 1 à 6 atomes de carbone; $R^9$;

$R^9$ =

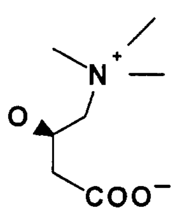
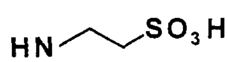
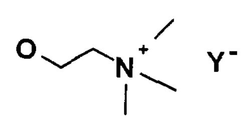

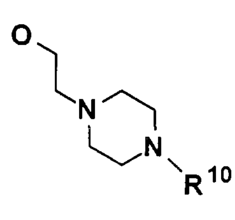
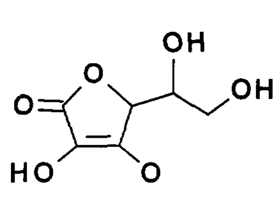
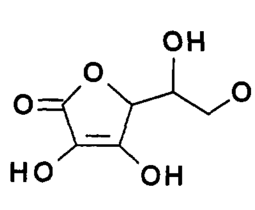

$R^{10}$ = hydrogène; alkyle inférieur, linéaire ou ramifié, comprenant de 1 à 6 atomes de carbone; aryle; aralkyle dans lequel alkyle est linéaire ou ramifié comprenant de 1 a 6 atomes de carbone; aryle substitué ou aralkyle substitué, le terme substitué affectant les groupements aryle, aralkyle, signifie que ceux-ci sont substitués sur

la partie aromatique par un ou plusieurs groupements identiques ou différents choisis parmi alkyle inférieur linéaire ou ramifié comprenant de 1 à 6 atomes de carbone, trifluorométhyl, alkoxyl inférieur, linéaire ou ramifié comprenant de 1 à 6 atomes de carbone, hydroxyl, nitro, amino, (alkyl, linéaire ou ramifié en $C_{1-6}$), amino; di-(alkyl, linéaire ou ramifié en $C_{1-6}$), amino; sulfonyl; sulfonamide; sulfo(alkyl, linéaire ou ramifié en $C_{1-6}$); carboxyl; carb(alkoxyl, linéaire ou ramifié en $C_{1-6}$); ou par 1 ou plusieurs atomes d'hydrogène;

Y- représente l'anion d'un acide pharmaceutiquement acceptable;

n = 0, 1;

m=0, 1, 2;

p =1,2,3;

q =2,3,4;

r = 0, 1.

et leurs sels d'acides ou de bases pharmaceutiquement acceptables;
sachant qu'il ne peut y avoir au plus qu'un seul substituant $R^9$ au sein de chaque molécule répondant à la formule générale II.

**2.** Utilisation de composés de type benzisosélén-azoline et -azine selon la revendication 1 répondant à la formule générale (I) suivante:

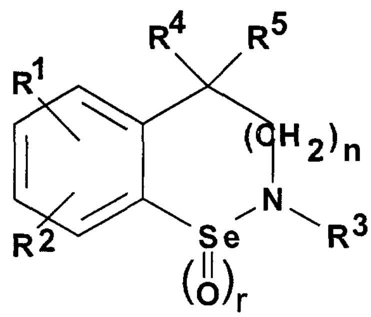

**Formule Générale I**

dans laquelle:

$R^1$ = hydrogène; alkyle inférieur; $-OR^6$; $-(CH_2)_mNR^6R^7$; $-(CH_2)_qNH_2$; $-(CH_2)_mNHSO_2(CH_2)_2NH_2$; -CN; $-SO_3H$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^2$ = hydrogène; alkyle inférieur; $-OR^6$; $-(CH_2)_mNR^6R^7$; $-(CH_2)_qNH_2$; $-(CH_2)_mNHSO_2(CH_2)_2NH_2$; -CN; $-SO_3H$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^3$ = hydrogène; alkyle inférieur; aralkyle; aralkyle substitué;$-(CH_2)_mCOR^8$; $-(CH_2)_qR^8$; $-CO(CH_2)_pCOR^8$; $-(CH_2)_mSO_2R^8$; $-(CH_2)_mS(O)R^8$;

$R^4$ = alkyle inférieur; aralkyle; aralkyle substitué; $-(CH_2)_pCOR^8$;$-(CH_2)_pR^8$; F;

$R^5$ = alkyle inférieur; aralkyle; aralkyle substitué;

$R^6$ = alkyle inférieur; aralkyle; aralkyle substitué; $-(CH_2)_mCOR^8$;$-(CH_2)_qR^8$;

$R^7$ = alkyle inférieur; aralkyle; aralkyle substitué; $-(CH_2)_mCOR^8$;

$R^8$ = alkyle inférieur; aralkyle; aralkyle substitué; aryle; aryle substitué; hétéroaryle; hétéroaryle substitué; hydroxyle; alkoxyle inférieur; $R^9$;

$R^9$ =

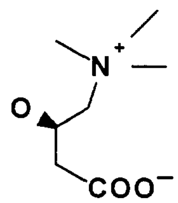
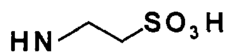
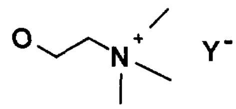

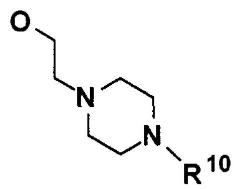
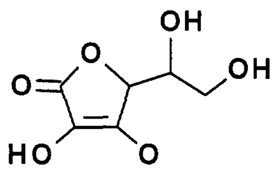
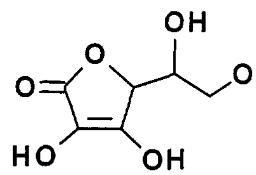

$R^{10}$ = hydrogène; alkyle inférieur; aralkyle ou aralkyle substitué; aryle ou aryle substitué;

dans laquelle alkyle inférieur, aralkyle, aralkyle substitué, aryle substitué, hétéroaryle et hétéroaryle substituté sont tels que définis à la revendication 1

$Y^-$ représente l'anion d'un acide pharmaceutiquement acceptable;
n=0, 1;
m=0, 1,2;
p = 1, 2, 3;
q =2,3,4;
r = 0, 1.

et leurs sels d'acides ou de bases pharmaceutiquement acceptables;
sachant qu'il ne peut y avoir au plus qu'un seul substituant $R^9$ au sein de chaque molécule répondant à la formule générale I;
comme agents antioxydants, en particulier pour la fabrication d'une composition pharmaceutique à activité antioxydante ou gluthatione peroxydase, en particulier pour:

- le traitement des pathologies dans lesquelles une surproduction d'hydroperoxydes cytotoxiques contribue aux altérations fonctionnelles des cellules ou des tissus;
- le traitement des pathologies à composante inflammatoire et/ou ischémique:
  - au niveau vasculaire: tel que par exemple le traitement de la prévention des re-sténoses artérielles consécutives à une intervention d'angioplastie, le traitement préventif des sténoses artérielles consécutives aux allogreffes d'artères, le traitement de la claudication intermittente chez les patients atteints d'ischémie obstructive des membres inférieurs; le traitement des accidents cérébro-vasculaires d'origine ischémiques; le traitement préventif ou curatif des syndrômes de détresse respiratoire de l'adulte (ARDS) ou de l'enfant (IRDS).
  - au niveau articulaire: tel que par exemple le traitement de la polyarthrite rhumatoïde;

- le traitement des pathologies ophtalmiques: tel que par exemple le traitement des allergies ophtalmiques aigües; le traitement des altérations rétiniennes qui sont associées à une dégénérence maculaire; le traitement du glaucome;
- le traitement de disfonctionnements immunitaires tel que par exemple le traitement du syndrome d'immunodéficience acquise (SIDA);
- le traitement protecteur contre l'intoxication par des xénobiotiques générateurs de radicaux libres, en particulier lors de chimiothérapie anticancéreuse;
- la conservation de greffons en transplantation d'organes tels que le coeur, le foie, le rein et le poumon; par addition de l'un des composés de la présente invention aux milieux de conservation de ces organes.

**3.** Utilisation selon la revendication 2, caractérisée en ce que le dérivé benzisoséléna-zoline ou -zine de formule générale (I) précitée est présent en une quantité comprise entre 0,1 et 5 % en poids par rapport au poids total de la composition finale, de préférence entre 0,1 et 1 % en poids ; ou sous forme de dose unitaire comprenant de 1 à 500 mg de dérivé, éventuellement dans un excipient, véhicule ou support pharmaceutiquement acceptable.

**4.** Composition pharmaceutique, en particulier à activité antioxydante ou gluthatione peroxydase, caractérisée en ce qu'elle comprend, à titre d'ingrédient actif, au moins un composé benzisoséléna-zoline ou -zine répondant à la formule générale (I) définie à la revendication 2, éventuellement dans un excipient, support ou véhicule pharmaceutiquement acceptable.

**5.** Composition pharmaceutique selon la revendication 4, caractérisée en ce que le dérivé benzisoséléna-zoline ou -zine répondant à la formule générale
(I) précitée est présent en une quantité comprise entre 0,1 et 5 % en poids par rapport au poids total de la composition finale, de préférence entre 0,1 et 1 % en poids ; ou sous forme de dose unitaire comprenant de 1 à 500 mg de ce dérivé, éventuellement dans un excipient, véhicule ou support pharmaceutiquement acceptable.

**6.** Composition pharmaceutique selon la revendication 4 ou 5, caractérisée en ce qu'il s'agit d'une composition pharmaceutique pour le traitement de toute condition physiopathologique dans laquelle une surproduction d'hydroperoxydes cytotoxiques contribue aux altérations fonctionnelles des cellules ou des tissus, par exemple conséquence de l'activation de voies métaboliques telles que, par exemple, celles des oxygènases à flavine ou à cytochrome P-450, celles des monoamine oxydases, celles de la xanthine oxydase ou de la 15-lipoxygènase des cellules endothéliales, celles de la cyclooxygènase ou de la 12-lipoxygènase des plaquettes sanguines, celles de la NADPH oxydase ou de la 5-lipoxygènase des neutrophiles, des macrophages ou des lymphocytes, ou encore une intoxication par un xénobiotique, tel que par exemple les anthracyclines, les nitro-imidazoles ou les dérivés de type dipyridinium, générateur de radicaux libres.

**7.** Composition pharmaceutique selon l'une des revendications 4 à 6, caractérisée en ce qu'il s'agit d'une composition pharmaceutique pour le traitement des pathologies à composante inflammatoire et/ou ischémique au niveau vasculaire ou articulaire ; pour le traitement des pathologies ophtalmiques, tel que par exemple le traitement des allergies ophtalmiques aigües, le traitement des altérations rétiniennes qui sont associées à une dégénérescence maculaire, le traitement du glaucome ; le traitement de disfonctionnements immunitaires tel que par exemple le traitement du syndrome d'immunodéficience acquise (SIDA) ; le traitement protecteur contre l'intoxication par des xénobiotiques générateurs de radicaux libres, en particulier lors de chimiothérapie anticancéreuse ; pour la conservation de greffons en transplantation d'organes tels que le coeur, le foie, le rein et le poumon, par addition de l'un des composés de la formule (I) précitée au milieu de concentration de ces organes.

**8.** Procédé de fabrication des composés répondant à la formule générale (II) définie à la revendication 1 caractérisé en ce qu'il comprend les étapes essentielles suivantes :

a/ Préparer ou utiliser un dérivé orthohalogènophényl-acétonitrile gem-disubstitué en position 2,
puis selon la série considérée:

soit b1/ hydrolyser le dérivé nitrile en dérivé amide,

c1/ transformer celui-ci en dérivé aminé par une réaction de transposition selon les méthodes usuelles,
d1/ ce composé aminé est mis en réaction avec un dérivé sélénié de type nucléophile, éventuellement généré in situ, en présence d'un sel de cuivre,
dans un solvant organique polaire, pour aboutir au dérivé benzisosélénazoline correspondant,

e1/ ce dernier est éventuellement N-alkylé ou N-acylé selon les procédures habituelles;
f1/ enfin, le dérivé ainsi obtenu est éventuellement oxydé au niveau du sélénium selon les procédures usuelles;

soit b2/ réduire le dérivé nitrile en dérivé aminé à l'aide par exemple de borane dans un solvant éthéré comme par exemple le tétrahydrofuranne,

c2/ ce composé aminé est mis en réaction avec un dérivé sélénié
de type nucléophile, éventuellement généré in situ, en présence d'un sel de cuivre, dans un solvant organique polaire, pour aboutir au dérivé benzisosélénazine correspondant,
d2/ ce dernier est éventuellement N-alkylé ou N-acylé selon les procédures habituelles;
e2/ enfin, le dérivé ainsi obtenu est éventuellement oxydé au niveau du sélénium selon les procédures usuelles;

**9.** Procédé selon la revendication 8, caractérisé en ce que le composé sélénié de type nucléophile est de préférence un sel de sélénocyanate comme par exemple le sélénocyanate de potassium qui peut être:

* soit généré in situ à partir de sélénium métal (Se°) et d'un sel de cyanure comme par exemple le cyanure de potassium,
* soit additionné au milieu réactionnel en tant que tel.

**10.** Procédé selon la revendication 8 ou 9, caractérisé en ce que le sel de cuivre peut être un sel cuivreux ($Cu^{I}$), tel que par exemple l'iodure cuivreux.

**11.** Procédé selon l'une des revendications 8 à 10, caractérisé en ce que le solvant organique polaire est de préférence le diméthylformamide.

**12.** Procédé selon l'une des revendications 8 à 11, caractérisé en ce que l'oxydant, éventuellement utilisé pour réaliser l'oxydation au niveau du sélénium, peut être un peracide, tel que l'acide métachloroperbenzoïque, ou le peroxyde d'hydrogène.

**13.** 3,3-diméthyl-benzisosélénazoline selon la revendication 1 et ses sels d'acides ou de bases pharmaceutiquement acceptables.

**14.** 4,4-diméthyl-benzisosélénazine selon la revendication 1 et ses sels d'acides ou de bases pharmaceutiquement acceptables.

**15.** 4,4-diméthyl-6-methoxy- benzisosélénazine selon la revendication 1 et ses sels d'addition d'acides ou de bases pharmaceutiquement acceptables.

**16.** Composé benzisosélén-azoline ou -azine selon la revendication 1 choisi parmi le groupe consistant de 2-acétyl-3,3-diméthyl-benzisosélénazoline, 3,3-diméthyl-2-éthylbenzisosélénazoline, 3,3-diméthyl-benzisosélénazoline-1-oxyde, 3,3'-diméthyl-7-nitro-benzisosélénazoline, 3,3'-diméthyl-5-nitro-benzisosélénazoline, 3,3'-diméthyl-7-fluoro-benzisosélénazoline, 4,4-diméthyl-benzisosélénazine-1-oxyde, 4,4-diméthyl-2-éthyl benzisosélénazine, et 4,4- diméthyl-6-(2'-(4"-méthylpiperazine-1"-yl)ethoxy-benzisosélénazine.

**17.** Composition pharmaceutique, caractérisée en ce qu'elle comprend comme ingrédient actif la 4,4-diméthyl-benzisosélénazine selon la revendication 1 ou un sel de base ou d'acide pharmaceutiquement acceptable de celle-ci, dans un excipient pharmaceutiquement acceptable.

**18.** Composition pharmaceutique, caractérisée en ce qu'elle comprend comme ingrédient actif la 3,3-diméthyl-benzisosélénazoline selon la revendication 1 ou un sel d'acide ou de base pharmaceutiquement acceptable de celle-ci, dans un excipient pharmaceutiquement acceptable.

**19.** Composition pharmaceutique, caractérisée en ce qu'elle comprend comme ingrédient actif la 4,4- diméthyl-6-méthoxybenzisosélénazine selon la revendication 1 ou un sel d'acide ou de base pharmaceutiquement acceptable de celle-ci, dans un excipient pharmaceutiquement acceptable.

**20.** Composition pharmaceutique, caractérisée en ce qu'elle comprend comme ingrédient actif au moins une benzisosélén-azoline ou-azine selon la revendication 1 choisie parmi le groupe consistant de 2-acétyl-3,3-diméthyl-benzisosélénazoline, 3,3-diméthyl-2-éthyl- benzisosélénazoline, 3,3-diméthyl-benzisosélénazoline-1-oxyde, 3,3'-diméthyl-7-fluoro-benzisosélénazoline, 4,4-diméthyl-benzisosélénazine-1-oxyde, 4,4-diméthyl-2-éthyl benzisosélénazine, et 4,4- diméthyl-6-(2'-(4"-méthyl-piperazine-1"-yl)ethoxy-benzisosélénazine, ou un sel d'acide ou de base pharmaceutiquement acceptable de celle-ci, dans un excipient pharmaceutiquement acceptable.

**21.** Composition pharmaceutique selon l'une des revendications 17 à 20 caractérisée en ce que l'ingrédient actif est présent en une quantité comprise entre 0,1% 5% en poids de la composition pharmaceutique.

**22.** Composition pharmaceutique selon l'une des revendications 17 à 21 caractérisée en ce qu'elle se présente sous forme d'une dose unitaire comprenant de là 500 mg dudit ingrédient actif.

**23.** Utilisation d'un composé tel que défini à l'une quelconque des revendications 14 à 16 comme antioxydant en particulier pour la fabrication d'une composition pharmaceutique à activité antioxydante gluthatione peroxydase en particulier pour réaliser un traitement tel que défini à la revendication 2,6 ou 7, et de préférence pour traiter la polyarthrite rhumatoïde.

**Patentansprüche**

**1.** Benzisoselenazolin- und -azinverbindungen der folgenden allgemeinen Formel (II):

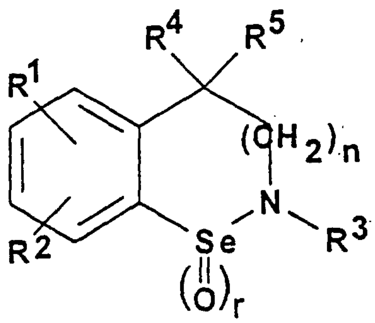

Allgemeine Formel II

worin:

$R^1$ = Wasserstoff, lineares oder verzweigtes nied.Alkyl mit 1 bis 6 Kohlenstoffatomen; $-OR^6$; $-(CH_2)_mNR^6R^7$; $-(CH_2)_qNH_2$; $-(CH_2)_mNHSO_2(CH_2)_2NH_2$; $-NO_2$; -CN; $-SO_3H$; $-N^+(R^5)_2O^-$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^2$ = Wasserstoff, lineares oder verzweigtes nied.Alkyl mit 1 bis 6 Kohlenstoffatomen; $-OR^6$; $-(CH_2)_mNR^6R^7$; $-(CH_2)_qNH_2$; $-(CH_2)_mNHSO_2(CH_2)_2NH_2$; $-NO_2$; -CN; $-SO_3H$; $-N^+(R^5)_2O^-$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^3$ = Wasserstoff, lineares oder verzweigtes nied.Alkyl mit 1 bis 6 Kohlenstoffatomen; Aralkyl, worin Alkyl linear oder verzweigt ist mit 1 bis 6 Kohlenstoffatomen; substituiertes Aralkyl, wobei der Ausdruck substituiert, der die Aralkylgruppe betrifft, bedeutet, dass diese am aromatischen Teil durch eine oder mehrere gleiche oder verschiedene Gruppen substituiert ist, ausgewählt aus linearem oder verzweigtem nied.Alkyl mit 1 bis 6 Kohlenstoffatomen, Trifluormethyl, linearem oder verzweigtem nied.Alkoxy mit 1 bis 6 Kohlenstoffatomen, Hydroxyl, Nitro, Amino, (Alkyl, linear oder verzweigt mit $C_{1-6}$)-Amino; di-(Alkyl, linear oder verzweigt mit $C_{1-6}$)-Amino; Sulfonyl; Sulfonamid, Sulfo(alkyl, linear oder verzweigt mit $C_{1-6}$); Carboxyl; Carb(alkoxy, linear oder verzweigt mit $C_{1-6}$); oder durch 1 oder mehrere Wasserstoffatome; $-(CH_2)_mCOR^8$; $-(CH_2)_qR^8$; -CO

$(CH_2)_pCOR^8$; $-(CH_2)_mSO_2R^8$; $-(CH_2)_mS(O)R^8$;

R4 = lineares oder verzweigtes nied.Alkyl mit 1 bis 6 Kohlenstoffatomen; Aralkyl, worin Alkyl linear oder verzweigt ist mit 1 bis 6 Kohlenstoffatomen; substituiertes Aralkyl, wobei der Ausdruck substituiert, der die Aralkylgruppe betrifft, bedeutet, dass diese am aromatischen Teil durch eine oder mehrere gleiche oder verschiedene Gruppen substituiert ist, ausgewählt aus linearem oder verzweigtem nied.Alkyl mit 1 bis 6 Kohlenstoffatomen, Trifluormethyl, linearem oder verzweigtem nied.Alkoxy mit 1 bis 6 Kohlenstoffatomen, Hydroxyl, Nitro, Amino, (Alkyl, linear oder verzweigt mit $C_{1-6}$)-Amino; di-(Alkyl, linear oder verzweigt mit $C_{1-6}$)-Amino; Sulfonyl; Sulfonamid, Sulfo(alkyl, linear oder verzweigt mit $C_{1-6}$); Carboxyl; Carb(alkoxy, linear oder verzweigt mit $C_{1-6}$); oder durch 1 oder mehrere Wasserstoffatome; $-(CH_2)_pCOR^8$; $-(CH_2)_pR^8$; F;

R5 = lineares oder verzweigtes nied.Alkyl mit 1 bis 6 Kohlenstoffatomen; Aralkyl, worin Alkyl linear oder verzweigt ist mit 1 bis 6 Kohlenstoffatomen; substituiertes Aralkyl, wobei der Ausdruck substituiert, der die Aralkylgruppe betrifft, bedeutet, dass diese am aromatischen Teil durch eine oder mehrere gleiche oder verschiedene Gruppen substituiert ist, ausgewählt aus linearem oder verzweigtem nied.Alkyl mit 1 bis 6 Kohlenstoffatomen, Trifluormethyl, linearem oder verzweigtem nied.Alkoxy mit 1 bis 6 Kohlenstoffatomen, Hydroxyl, Nitro, Amino, (Alkyl, linear oder verzweigt mit $C_{1-6}$)-Amino; di-(Alkyl, linear oder verzweigt mit $C_{1-6}$)-Amino; Sulfonyl; Sulfonamid, Sulfo(alkyl, linear oder verzweigt mit $C_{1-6}$); Carboxyl; Carb(alkoxy, linear oder verzweigt mit $C_{1-6}$); oder durch 1 oder mehrere Wasserstoffatome;

R6 = lineares oder verzweigtes nied.Alkyl mit 1 bis 6 Kohlenstoffatomen; Aralkyl, worin Alkyl linear oder verzweigt ist mit 1 bis 6 Kohlenstoffatomen; substituiertes Aralkyl, wobei der Ausdruck substituiert, der die Aralkylgruppe betrifft, bedeutet, dass diese am aromatischen Teil durch eine oder mehrere gleiche oder verschiedene Gruppen substituiert ist, ausgewählt aus linearem oder verzweigtem nied.Alkyl mit 1 bis 6 Kohlenstoffatomen, Trifluormethyl, linearem oder verzweigtem nied.Alkoxy mit 1 bis 6 Kohlenstoffatomen, Hydroxyl, Nitro, Amino, (Alkyl, linear oder verzweigt mit $C_{1-6}$)-Amino; di-(Alkyl, linear oder verzweigt mit $C_{1-6}$)-Amino; Sulfonyl; Sulfonamid, Sulfo(alkyl, linear oder verzweigt mit $C_{1-6}$); Carboxyl; Carb(alkoxy, linear oder verzweigt mit $C_{1-6}$); oder durch 1 oder mehrere Wasserstoffatome; $-(CH_2)_mCOR^8$; $-(CH_2)_qR^8$;

R7= lineares oder verzweigtes nied.Alkyl mit 1 bis 6 Kohlenstoffatomen; Aralkyl, worin Alkyl linear oder verzweigt ist mit 1 bis 6 Kohlenstoffatomen; substituiertes Aralkyl, wobei der Ausdruck substituiert, der die Aralkylgruppe betrifft, bedeutet, dass diese am aromatischen Teil durch eine oder mehrere gleiche oder verschiedene Gruppen substituiert ist, ausgewählt aus linearem oder verzweigtem nied.Alkyl mit 1 bis 6 Kohlenstoffatomen, Trifluormethyl, linearem oder verzweigtem nied.Alkoxy mit 1 bis 6 Kohlenstoffatomen, Hydroxyl, Nitro, Amino, (Alkyl, linear oder verzweigt mit $C_{1-6}$)-Amino; di-(Alkyl, linear oder verzweigt mit $C_{1-6}$)-Amino; Sulfonyl; Sulfonamid, Sulfo(alkyl, linear oder verzweigt mit $C_{1-6}$); Carboxyl; Carb(alkoxy, linear oder verzweigt mit $C_{1-6}$); oder durch 1 oder mehrere Wasserstoffatome; $-(CH_2)_mCOR^8$;

R8 = lineares oder verzweigtes nied.Alkyl mit 1 bis 6 Kohlenstoffatomen; Aryl; Aralkyl, worin Alkyl linear oder verzweigt ist mit 1 bis 6 Kohlenstoffatomen; Heteroaryl, was jeden aromatischen mono- oder bicyclischen Kern bedeutet, wobei jeder Ring 5 bis 6 Ecken aufweist und in seinem Kohlenstoffskelett ein oder gegebenenfalls mehrere gleiche oder verschiedene Heteroatome enthält, ausgewählt aus Stickstoff, Sauerstoff oder Schwefel; substituiertem Aralkyl; substituiertem Aryl, substituiertem Heteroaryl; wobei der Ausdruck substituiert, der eine Aralkyl- Aryl- oder Heteroarylgruppe betrifft, bedeutet, dass diese am aromatischen Teil durch eine oder mehrere gleiche oder verschiedene Gruppen substituiert ist, ausgewählt aus linearem oder verzweigtem nied.Alkyl mit 1 bis 6 Kohlenstoffatomen, Trifluormethyl, linearem oder verzweigtem nied.Alkoxy mit 1 bis 6 Kohlenstoffatomen, Hydroxyl, Nitro, Amino, (Alkyl, linear oder verzweigt mit $C_{1-6}$)-Amino; di-(Alkyl, linear oder verzweigt mit $C_{1-6}$)-Amino; Sulfonyl; Sulfonamid, Sulfo(alkyl, linear oder verzweigt mit $C_{1-6}$); Carboxyl; Carb(alkoxy, linear oder verzweigt mit $C_{1-6}$); oder durch 1 oder mehrere Wasserstoffatome; Hydroxyl, lineares oder verzweigtes nied.Alkoxy mit 1 bis 6 Kohlenstoffatomen; $R^9$;

$R^9$ =

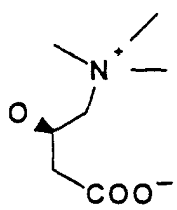

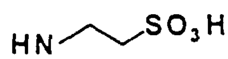

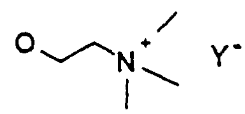

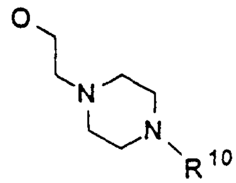

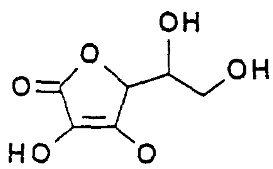

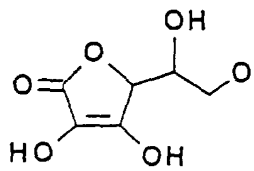

R10 = Wasserstoff; lineares oder verzweigtes nied.Alkyl mit 1 bis 6 Kohlenstoffatomen; Aryl; Aralkyl, worin Alkyl linear oder verzweigt ist mit 1 bis 6 Kohlenstoffatomen; substituiertes Aryl oder substituiertes Aralkyl, wobei der Ausdruck substituiert, der die Gruppen Aryl, Aralkyl betrifft, bedeutet, dass diese am aromatischen Teil durch eine oder mehrere gleiche oder verschiedene Gruppen substituiert sind, ausgewählt aus linearem oder verzweigtem nied.Alkyl mit 1 bis 6 Kohlenstoffatomen, Trifluormethyl, linearem oder verzweigtem nied. Alkoxy mit 1 bis 6 Kohlenstoffatomen, Hydroxyl, Nitro, Amino, (Alkyl, linear oder verzweigt mit $C_{1-6}$)-Amino; di-(Alkyl, linear oder verzweigt mit $C_{1-6}$)-Amino; Sulfonyl; Sulfonamid, Sulfo(alkyl, linear oder verzweigt mit $C_{1-6}$); Carboxyl; Carb(alkoxy, linear oder verzweigt mit $C_{1-6}$); oder durch 1 oder mehrere Wasserstoffatome;

$Y^-$ das Anion einer pharmazeutisch annehmbaren Säure darstellt;
n=0, 1;
m=0, 1, 2;
p = 1, 2, 3;
q = 2, 3, 4;
r=0, 1

und die pharmazeutisch annehmbaren Salze derselben mit Säuren oder Basen;
mit der Maßgabe, dass es nur höchstens einen Substituent $R^9$ in jedem Molekül der allgemeinen Formel II geben kann.

2. Verwendung von Verbindungen vom Benzisoselenazolin- und -azin-Typ nach Anspruch 1 der folgenden allgemeinen Form

nel (I):

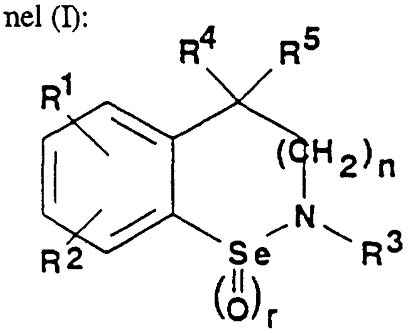

Allgemeine Formel II

worin:

$R^1$ = Wasserstoff; nied.Alkyl; $-OR^6$; $-(CH_2)_mNR^6R^7$; $-(CH_2)_qNH_2$; $-(CH_2)_mNHSO_2(CH_2)_2NH_2$; -CN; $-SO_3H$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^2$ = Wasserstoff; nied.Alkyl; $-OR^6$; $-(CH_2)_mNR^6R^7$; $-(CH_2)_qNH_2$; $-(CH_2)_mNHSO_2(CH_2)_2NH_2$; -CN; $-SO_3H$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^3$ = Wasserstoff: nied.Alkyl; Aralkyl; substituiertes Aralkyl; $(CH_2)_mCOR^8$; $-(CH_2)_qR^8$; $-CO(CH_2)_pCOR^8$; $-(CH_2)_mSO_2R^8$; $-(CH_2)_mS(O)R^8$;

$R^4$ = nied.Alkyl; Aralkyl; substituiertes Aralkyl; $-(CH_2)_pCOR^8$; $-(CH_2)_pR^8$; F;

$R^5$ = nied.Alkyl; Aralkyl; substituiertes Aralkyl;

$R^6$ = nied.Alkyl; Aralkyl; substituiertes Aralkyl; $-(CH_2)_mCOR^8$; $-(CH_2)_qR^8$;

$R^7$ = nied.Alkyl; Aralkyl; substituiertes Aralkyl; $-(CH_2)_mCOR^8$;

$R^8$ = nied.Alkyl; Aralkyl; substituiertes Aralkyl; Aryl; substituiertes Aryl; Heteroaryl, substituiertes Heteroaryl; Hydroxyl, nied. Alkoxy; $R^9$;

$R^9$ =

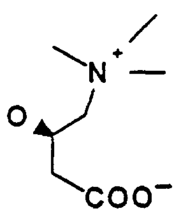

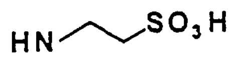

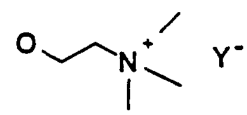

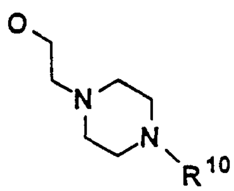

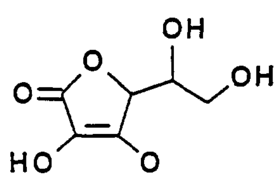

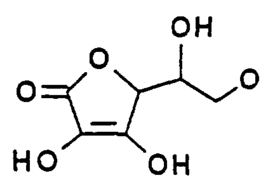

$R^{10}$ = Wasserstoff, nied.Alkyl, Aralkyl oder substituiertes Aralkyl; Aryl oder substituiertes Aryl; worin nied.Alkyl, Aralkyl, substituiertes Aralkyl; substituiertes Aryl, Heteroaryl und substituiertes Heteroaryl wie in Anspruch 1 definiert sind;

Y das Anion einer pharmazeutisch annehmbaren Säure darstellt;
n=0, 1; 1;
m = 0, 1, 2;
p = 1, 2, 3;
q = 2, 3, 4;
T = 0, 1

und der pharmazeutisch annehmbaren Salze derselben mit Säuren oder Basen;
mit der Maßgabe, dass es nur höchstens einen Substituent $R^9$ in jedem Molekül der allgemeinen Formel I geben kann;
als Antioxidationsmittel, insbesondere zur Herstellung einer pharmazeutischen Zusammensetzung mit Antioxidations- oder Glutathionperoxidase-Aktivität, insbesondere

- zur Behandlung von Pathologien, bei denen Überproduktion von zytotoxischen Wasserstoffperoxiden zu funktionellen Veränderungen von Zellen oder Geweben führt;
- zur Behandlung von Pathologien mit einer Entzündungs- und/oder Ischämie-Komponente;
  - im vaskulären Bereich: wie beispielsweise zur präventiven Behandlung von Arterien-Restenosen infolge eines angioplastischen Eingriffs, zur präventiven Behandlung von Arterien-Stenosen in Folge von Arterien-Alloplastik, zur Behandlung von Claudicatio intermittens bei Patienten mit Verschlusskrankheit der unteren Gliedmaßen; zur Behandlung von zerebrovaskulären Verletzungen ischämischen Ursprungs; zur präventiven oder kurativen Behandlung von akutem Atemnot-Syndrom des Erwachsenen (ARDS) und des Kindes (IRDS);
  - im artikulären Bereich: wie beispielsweise zur Behandlung von rheumatischer Polyarthritis;
- zur Behandlung von Augenerkrankungen: wie beispielsweise zur Behandlung von akuten Augenallergien; zur Behandlung von Netzhautveränderungen in Verbindung mit einer Macula-Degeneration; zur Behandlung eines Glaukoms;
- zur Behandlung von Immunstörungen wie beispielsweise zur Behandlung von erworbener Immuninsuffizienz (AIDS);
- zur Behandlung als Schutz gegen Intoxikation durch freie Radikale erzeugende Xenobiotika, insbesondere bei Chemotherapien gegen Krebs;
- zur Konservierung von Transplantaten bei Transplantationen von Organen wie dem Herzen, der Leber, der Niere und der Lunge; durch Zugabe einer der erfindungsgemäßen Verbindungen zu den Konservierungsmitteln für diese Organe.

**3.** Verwendung nach Anspruch 2, dadurch gekennzeichnet, dass das Benzisoselenazolin- oder -azin-Derivat der allgemeinen Formel (I) in einer Menge zwischen 0,1 und 5 Gew.-%, bezogen auf das Gesamtgewicht der Endzusammensetzung, vorzugsweise zwischen 0,1 und 1 Gew.-% oder in Dosiseinheitsform mit 1 bis 500 mg Derivat, gegebenenfalls in einem pharmazeutisch annehmbaren Exzipienten, Vehikel oder Träger, vorliegt.

**4.** Pharmazeutische Zusammensetzung, insbesondere mit Antioxidations- oder Glutathionperoxidase-Aktivität, dadurch gekennzeichnet, dass sie als Wirkstoff mindestens eine Benzisoselenazolin oder -azin-Verbindung der in Anspruch 2 definierten allgemeinen Formel (I), gegebenenfalls in einem pharmazeutisch annehmbaren Exzipienten, Vehikel oder Träger, enthält.

**5.** Pharmazeutische Zusammensetzung nach Anspruch 4, dadurch gekennzeichnet, dass das Benzisoselenazolin- oder -azin-Derivat der allgemeinen Formel (1) in einer Menge zwischen 0,1 und 5 Gew.-%, bezogen auf das Gesamtgewicht der Endzusammensetzung, vorzugsweise zwischen 0,1 und 1 Gew.-% oder in Dosiseinheitsform mit 1 bis 500 mg dieses Derivats, gegebenenfalls in einem pharmazeutisch annehmbaren Exzipienten, Vehikel oder Träger, vorliegt.

**6.** Pharmazeutische Zusammensetzung nach Anspruch 4 oder 5, dadurch gekennzeichnet, dass es sich um eine pharmazeutische Zusammensetzung zur Behandlung von jedwedem physiopathologischem Zustand handelt, bei dem eine Überproduktion von zytotoxischen Wasserstoffperoxiden zu funktionellen Veränderungen der Zellen oder Gewebe führt, beispielsweise in Folge der Aktivierung von Metabolismen wie beispielsweise jenen von Flavin- oder Cytochrom-P-450-Oxygenasen, von Monoamin-Oxydasen, der Xanthin-Oxidase oder der 15-Lipoxygenase von Endothelzellen, der Cyclooxygenase oder der 12-Lipoxygenase von Blutplättchen, der NADPH-Oxydase oder der 5-Lipoxygenase von Neutrophilen, Makrophagen oder Lymphozyten, oder aber einer Intoxikation durch Xenobiotika wie beispielsweise Anthracycline, Nitroimidazole oder Derivate vom Dipyridinium-Typ, die freie Radikale erzeugen.

**7.** Pharmazeutische Zusammensetzung nach einem der Ansprüche 4 bis 6, dadurch gekennzeichnet, dass es sich um eine pharmazeutische Zusammensetzung zur Behandlung von Pathologien mit einer Entzündungs- und/oder Ischämie-Komponente im vaskulären oder artikulären Bereich, zur Behandlung von Augenerkrankungen wie beispielsweise zur Behandlung von akuten Augenallergien, zur Behandlung von Netzhautveränderungen in Verbindung mit einer Macula-Degeneration, zur Behandlung eines Glaukoms; zur Behandlung von Immunstörungen wie beispielsweise zur Behandlung von erworbener Immuninsuffizienz (AIDS); zur Behandlung als Schutz gegen Intoxikation durch freie Radikale erzeugende Xenobiotika, insbesondere bei Chemotherapien gegen Krebs; zur Konservierung von Transplantaten bei Transplantationen von Organen wie dem Herzen, der Leber, der Niere und der Lunge durch Zugabe einer der Verbindungen der Formel (I) zum Konzentrationsmilieu dieser Organe handelt.

**8.** Verfahren zur Herstellung von Verbindungen der in Anspruch 1 definierten allgemeinen Formel (II), dadurch gekennzeichnet, dass es die folgenden wesentlichen Schritte umfasst:
a) Herstellen oder Verwenden eines in Position 2 gem-disubstituierten Orthohalogenphenylacetonitrils,
dann je nach in Betracht gezogener Serie:
entweder

b1) Hydrolysieren des Nitril-Derivats zu einem Amid-Derivat,
c1) Überführen desselben in ein Amino-Derivat durch eine Umlagerungsreaktion nach üblichen Methoden,
d1) diese Amino-Verbindung wird mit einem Selen-Derivat vom nucleophilen Typ, das gegebenenfalls in situ erzeugt wird, in Anwesenheit eines Kupfersalzes in einem polaren organischen Lösungsmittel zur Umsetzung gebracht, um zum entsprechenden Benzisoselenazolin-Derivat zu gelangen,
e1) letzteres wird gegebenenfalls mittels üblicher Verfahren N-alkyliert oder N-acyliert;
f1) das so erhaltene Derivat wird schließlich gegebenenfalls mittels üblicher Verfahren am Selen oxidiert;
oder
b2) Reduzieren des Nitril-Derivats in ein Amino-Derivat mit Hilfe von beispielsweise Bor in einem etherischen Lösungsmittel wie z.B. Tetrahydrofuran,
c2) diese Amino-Verbindung wird mit einem Selen-Derivat vom nucleophilen Typ, das gegebenenfalls in situ erzeugt wird, in Anwesenheit eines Kupfersalzes in einem polaren organischen Lösungsmittel zur Umsetzung gebracht, um zum entsprechenden Benzisoselenazin-Derivat zu gelangen,
d2) letzteres wird gegebenenfalls mittels üblicher Verfahren N-alkyliert oder N-acyliert;
e2) das so erhaltene Derivat wird schließlich gegebenenfalls mittels üblicher Verfahren am Selen oxidiert.

**9.** Verfahren nach Anspruch 8, dadurch gekennzeichnet, dass die Selen-Verbindung vom nucleophilen Typ vorzugsweise ein Selenocyanat-Salz wie z.B. Kaliumselenocyanat ist, das

* entweder in situ aus metallischem Selen ($Se^{\circ}$) und einem Cyanidsalz wie z.B. Kaliumcyanid hergestellt
* oder als solches dem Reaktionsmedium zugesetzt

werden kann.

**10.** Verfahren nach Anspruch 8 oder 9, dadurch gekennzeichnet, dass das Kupfersalz ein Kupfer(I)-Salz ($Cu^{I}$) wie z. B. Kupfer(I)-iodid sein kann.

**11.** Verfahren nach einem der Ansprüche 8 bis 10, dadurch gekennzeichnet, dass das polare organische Lösungsmittel vorzugsweise Dimethylformamid ist.

**12.** Verfahren nach einem der Ansprüche 8 bis 11, dadurch gekennzeichnet, dass das Oxidationsmittel, das gegebenenfalls zur Durchführung der Oxidation am Selen verwendet wird, eine Persäure wie Metachlorperbenzoesäure oder Wasserstoffperoxid sein kann.

**13.** 3,3-Dimethylbenzisoselenazolin nach Anspruch 1 und seine pharmazeutisch annehmbaren Salze mit Säuren oder Basen.

**14.** 4,4-Dimethylbenzisoselenazin nach Anspruch 1 und seine pharmazeutisch annehmbaren Salze mit Säuren oder Basen.

**15.** 4,4-Dimethyl-6-methoxybenzisoselenazin nach Anspruch 1 und seine pharmazeutisch annehmbaren Salze mit Säuren oder Basen.

**16.** Benzisoselenazolin- oder -azin-Verbindung nach Anspruch 1, ausgewählt aus der Gruppe bestehend aus 2-Acetyl-3,3-dimethylbenzisoselenazolin, 3,3-Dimethyl-2-ethylbenzisoselenazolin, 3,3-Dimethylbenzisoselenazolin-1-oxid, 3,3'-Dimethyl-7-nitrobenzisoselenazolin, 3,3-Dimethyl-5-nitrobenzisoselenazolin, 3,3'-Dimethyl-7-fluorbenzisoselenazolin, 4,4-Dimethylbenzisoselenazin-1-oxid, 4,4-Dimethyl-2-ethylbenzisoselenazin und 4,4-Dimethyl-6-(2'-(4"-methylpiperazin-1"-yl)-ethoxybenzisoselenazin.

**17.** Pharmazeutische Zusammensetzung, dadurch gekennzeichnet, dass sie als Wirkstoff 4,4-Dimethylbenzisoselenazin nach Anspruch 1 oder ein pharmazeutisch annehmbares Salz desselben mit einer Säure oder Base in einem pharmazeutisch annehmbaren Exzipienten enthält.

**18.** Pharmazeutische Zusammensetzung, dadurch gekennzeichnet, dass sie als Wirkstoff 3,3-Dimethylbenzisoselenazolin nach Anspruch 1 oder ein pharmazeutisch annehmbares Salz desselben mit einer Säure oder Base in einem pharmazeutisch annehmbaren Exzipienten enthält.

**19.** Pharmazeutische Zusammensetzung, dadurch gekennzeichnet, dass sie als Wirkstoff 4,4-Dimethyl-6-methoxybenzisoselenazin nach Anspruch 1 oder ein pharmazeutisch annehmbares Salz desselben mit einer Säure oder Base in einem pharmazeutisch annehmbaren Exzipienten enthält.

**20.** Pharmazeutische Zusammensetzung, dadurch gekennzeichnet, dass sie als Wirkstoff mindestens ein Benzisoselenazolin- oder -azin nach Anspruch 1, ausgewählt aus der Gruppe bestehend aus 2-Acetyl-3,3-dimethylbenzisoselenazolin, 3,3-Dimethyl-2-ethylbenzisoselenazolin, 3,3-Dimethylbenzisoselenazolin-1-oxid, 3,3'-Dimethyl-7-fluorbenzisoselenazolin, 4,4-Dimethylbenzisoselenazin-1-oxid, 4,4-Dimethyl-2-ethylbenzisoselenazin und 4,4-Dimethyl-6-(2'-(4"-methylpiperazin-1"-yl)ethoxybenzisoselenazin, oder ein pharmazeutisch annehmbares Salz desselben mit einer Säure oder Base in einem pharmazeutisch annehmbaren Exzipienten enthält.

**21.** Pharmazeutische Zusammensetzung nach einem der Ansprüche 17 bis 20, dadurch gekennzeichnet, dass der Wirkstoff in einer Menge zwischen 0,1 Gew.-% und 5 Gew.-% der pharmazeutischen Zusammensetzung vorhanden ist.

**22.** Pharmazeutische Zusammensetzung nach einem der Ansprüche 17 bis 20, dadurch gekennzeichnet, dass der Wirkstoff in Dosiseinheitsform mit 1 bis 500 mg Wirkstoff vorliegt.

**23.** Verwendung einer Verbindung nach einem der Ansprüche 14 bis 16 als Antioxidationsmittel, insbesondere zur Herstellung einer pharmazeutischen Zusammensetzung mit Antioxidations- oder Glutathionperoxidase-Aktivität, insbesondere zur Durchführung einer Behandlung nach einem der Ansprüche 2, 6 oder 7 und vorzugsweise zur Behandlung von rheumatischer Polyarthritis.

**Claims**

**1.** Benzisoselen-azoline and -azine compounds with the following general formula (II):

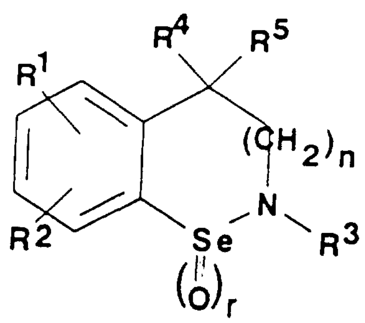

General formula II

where:

$R^1$ = hydrogen; linear or branched lower alkyl comprising 1 to 6 carbon atoms; $-OR^6$; $-(CH_2)_mNR^6R^7$; $-(CH_2)_qNH_2$;
$-(CH_2)_mNHSO_2(CH_2)_2NH_2$; $-NO_2$; -CN; $-SO_3H$; $-N^+(R^5)_2O^-$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^2$ = hydrogen; linear or branched lower alkyl comprising 1 to 6 carbon atoms; $-OR^6$; - $(CH_2)_mNR^6R^7$; $-(CH_2)_qNH_2$;

$-(CH_2)_mNHSO_2(CH_2)_2NH_2$; $-NO_2$; -CN; $-SO_3H$; $-N^+(R^5)_2O^-$; F; Cl; Br; I; $-(CH_2)_mR^8$; $-(CH_2)_mCOR^8$; -S(O) $NR^6R^7$; $-SO_2NR^6R^7$; $-CO(CH_2)_pCOR^8$; $R^9$;

$R^3$ = hydrogen; linear or branched lower alkyl comprising 1 to 6 carbon atoms; aralkyl, in which the alkyl is a linear or branched alkyl comprising 1 to 6 carbon atoms; substituted aralkyl, the term substituted affecting the aralkyl group, means that it is substituted on the aromatic part by one or more identical or different groups selected from the linear or branched lower alkyl comprising 1 to 6 carbon atoms, trifluoromethyl, linear or branched lower alkoxy comprising 1 to 6 carbon atoms, hydroxyl, nitro, amino, (alkyl, linear or branched, in $C_{1-6}$)amino; di-(alkyl, linear or branched, in $C_{1-6}$)amino; sulfonyl; sulfonamide; sulfo(alkyl, linear or branched, in $C_{1-6}$); carboxyl; carb(alkoxyl, linear or branched, in $C_{1-6}$); or by 1 or more hydrogen atoms; $-(CH_2)_mCOR^8$; $-(CH_2)_qR^8$; $-CO(CH_2)_pCOR^8$; $-(CH_2)_mSO_2R^8$; $-(CH_2)_mS(O)R^8$;

$R^4$ = linear or branched lower alkyl comprising 1 to 6 carbon atoms; aralkyl, in which the alkyl is a linear or branched alkyl comprising 1 to 6 carbon atoms; substituted aralkyl, the term substituted affecting the aralkyl group, means that it is substituted on the aromatic part by one or more identical or different groups selected from the linear or branched lower alkyl comprising 1 to 6 carbon atoms, trifluoromethyl, linear or branched lower alkoxy comprising 1 to 6 carbon atoms, hydroxyl, nitro, amino, (alkyl, linear or branched, in $C_{1-6}$)amino; di-(alkyl, linear or branched, in $C_{1-6}$)amino; sulfonyl; sulfonamide; sulfo(alkyl, linear or branched, in $C_{1-6}$); carboxyl; carb(alkoxyl, linear or branched, in $C_{1-6}$); or by 1 or more hydrogen atoms;$-(CH_2)pCOR^8$; $-(CH_2)_pR^8$; F;

$R^5$ = linear or branched lower alkyl comprising 1 to 6 carbon atoms; aralkyl, in which the alkyl is a linear or branched alkyl comprising 1 to 6 carbon atoms; substituted aralkyl, the term substituted affecting the aralkyl group, means that it is substituted on the aromatic part by one or more identical or different groups selected from the linear or branched lower alkyl comprising 1 to 6 carbon atoms, trifluoromethyl, linear or branched lower alkoxy comprising 1 to 6 carbon atoms, hydroxyl, nitro, amino, (alkyl, linear or branched, in $C_{1-6}$)amino; di-(alkyl, linear or branched, in $C_{1-6}$)amino; sulfonyl; sulfonamide; sulfo(alkyl, linear or branched, in $C_{1-6}$); carboxyl; carb(alkoxyl, linear or branched, in $C_{1-6}$); or by 1 or more hydrogen atoms;

$R^6$ = linear or branched lower alkyl comprising 1 to 6 carbon atoms; aralkyl, in which the alkyl is a linear or branched alkyl comprising 1 to 6 carbon atoms; substituted aralkyl, the term substituted affecting the aralkyl group, means that it is substituted on the aromatic part by one or more identical or different groups selected from the linear or branched lower alkyl comprising 1 to 6 carbon atoms, trifluoromethyl, linear or branched lower alkoxy comprising 1 to 6 carbon atoms, hydroxyl, nitro, amino, (alkyl, linear or branched, in $C_{1-6}$)amino; di-(alkyl, linear or branched, in $C_{1-6}$)amino; sulfonyl; sulfonamide; sulfo(alkyl, linear or branched, in $C_{1-6}$); carboxyl; carb(alkoxyl, linear or branched, in $C_{1-6}$); or by 1 or more hydrogen atoms; $-(CH_2)_mCOR^8$; $-(CH_2)_qR^8$;

$R^7$ = linear or branched lower alkyl comprising 1 to 6 carbon atoms; aralkyl, in which the alkyl is a linear or branched alkyl comprising 1 to 6 carbon atoms; substituted aralkyl, the term substituted affecting the aralkyl group, means that it is substituted on the aromatic part by one or more identical or different groups selected from the linear or branched lower alkyl comprising 1 to 6 carbon atoms, trifluoromethyl, linear or branched lower alkoxy comprising 1 to 6 carbon atoms, hydroxyl, nitro, amino, (alkyl, linear or branched, in $C_{1-6}$)amino; di-(alkyl, linear or branched, in $C_{1-6}$)amino; sulfonyl; sulfonamide; sulfo(alkyl, linear or branched, in $C_{1-6}$); carboxyl; carb(alkoxyl, linear or branched, in $C_{1-6}$); or by 1 or more hydrogen atoms; $-(CH_2)_mCOR^8$;

$R^8$ = linear or branched lower alkyl comprising 1 to 6 carbon atoms; aryl; aralkyl, in which the alkyl is a linear or branched alkyl comprising 1 to 6 carbon atoms; heteroaryl, meaning any mono or bicyclic aromatic nucleus, with each cycle comprising 5 to 6 peaks and including in its carbonated skeleton 1 or optionally more identical or different heteroatoms selected from nitrogen, oxygen or sulfur; substituted aralkyl; substituted aryl, substituted heteroaryl; the term substituted affecting an aralkyl, aryl, or heteroaryl group meaning that it is substituted on the aromatic part by one or more identical or different groups selected from the linear or branched lower alkyl comprising 1 to 6 carbon atoms, trifluoromethyl, linear or branched lower alkoxy comprising 1 to 6 carbon atoms, hydroxyl, nitro, amino, (alkyl, linear or branched, comprising 1 to 6 carbon atoms)amino; di(alkyl, linear or branched, comprising 1 to 6 carbon atoms)amino; sulfonyl; sulfonamide; sulfo(alkyl, linear or branched, comprising 1 to 6 carbon atoms); carboxyl; carb(alkoxyl, linear or branched, comprising 1 to 6 carbon atoms); or by 1 or more hydrogen atoms; hydroxyl; linear or branched lower alkoxy having 1 to 6 carbon atoms; $R^9$;

$R^9$ =

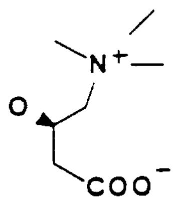

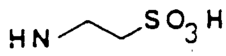

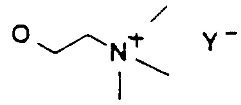

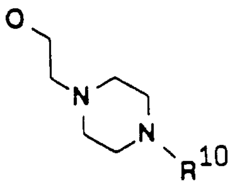

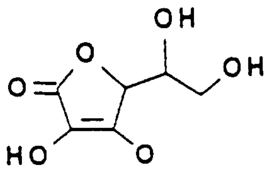

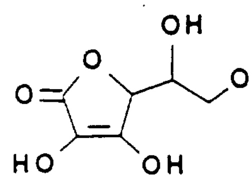

$R^{10}$ = hydrogen; linear or branched lower alkyl comprising 1 to 6 carbon atoms; aryl; aralkyl, in which the alkyl is a linear or branched alkyl comprising 1 to 6 carbon atoms; substituted aryl or substituted aralkyl, the term substituted affecting the aryl, aralkyl groups, means that they are substituted on the aromatic part by one or more identical or different groups selected from the linear or branched lower alkyl comprising 1 to 6 carbon atoms, trifluoromethyl, linear or branched lower alkoxy comprising 1 to 6 carbon atoms, hydroxyl, nitro, amino, (alkyl, linear or branched, in $C_{1-6}$) amino; di-(alkyl, linear or branched, in $C_{1-6}$)amino; sulfonyl; sulfonamide; sulfo(alkyl, linear or branched, in $C_{1-6}$); carboxyl; carb(alkoxyl, linear or branched, in $C_{1-6}$); or by 1 or more hydrogen atoms;

$Y^-$ represents the anion of a pharmaceutically acceptable acid;
n = 0, 1;
m = 0, 1, 2;
p = 1, 2, 3;
q = 2, 3, 4;
r = 0, 1;

and their pharmaceutically acceptable salts of acids or bases;
there being no more than one substituent $R^9$ in each molecule with general formula II.

**2.** The use of benzisoselen-azoline and -azine compounds according to claim 1 with the following general formula (I):

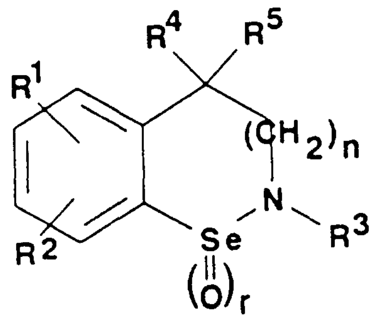

General formula I

where:

$R^1$ = hydrogen; lower alkyl; $-OR^6$; $-(CH_2)_mNR^6R^7$; $-(CH_2)_qNH_2$; $-(CH_2)_mNHSO_2(CH_2)_2NH_2$; -CN; $-SO_3H$; F; Cl; Br; I; $-(CH_2)_mR^8$; $-(CH_2)_mCOR^8$; $-S(O)NR^6R^7$; $-SO_2NR^6R^7$; $-CO(CH_2)_pCOR^8$; $R^9$;

$R^2$ = hydrogen; lower alkyl; $-OR^6$; $-(CH_2)_mNR^6R^7$; $-(CH_2)_qNH_2$; $-(CH_2)_mNHSO_2(CH_2)_2NH_2$; -CN ; $-SO_3H$; F; Cl; Br; I; $-(CH_2)_mR^8$; - $(CH_2)_mCOR^8$; $-S(O)NR^6R^7$; $-SO_2NR^6R^7$; $-CO(CH_2)_pCOR^8$; $R^9$;

$R^3$ = hydrogen; lower alkyl; aralkyl; substituted aralkyl; $-(CH_2)_mCOR^8$; $-(CH_2)_qR^8$; $-CO(CH_2)_pCOR^8$; $-(CH_2)_mSO_2R^8$; $-(CH_2)_mS(O)R^8$;

$R^4$ = lower alkyl; aralkyl; substituted aralkyl; $-(CH_2)_pCOR^8$; $-(CH_2)_pR^8$; F;

$R^5$ = lower alkyl; aralkyl; substituted aralkyl;

$R^6$ = lower alkyl; aralkyl; substituted aralkyl; $-(CH_2)_mCOR^8$; $-(CH_2)_qR^8$;

$R^7$ = lower alkyl; aralkyl; substituted aralkyl; $-(CH_2)_mCOR^8$;

$R^8$ = lower alkyl; aralkyl; substituted aralkyl; aryl; substituted aryl; heteroaryl; substituted heteroaryl; hydroxy; lower alkoxy; $R^9$;

$R^9$ =

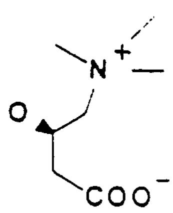

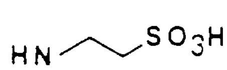

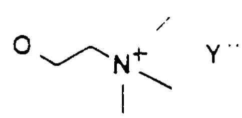

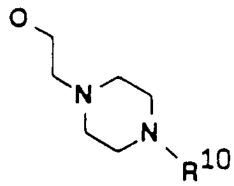 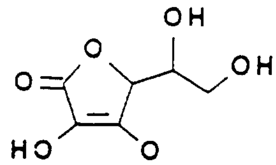 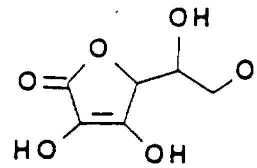

$R^{10}$ = hydrogen; lower alkyl; aralkyl or substituted aralkyl; aryl or substituted aryl;

in which lower alkyl, aralkyl, substituted aralkyl, substituted aryl, heteroaryl and substituted heteroaryl are as defined in claim 1;

$Y^-$ represents the anion of a pharmaceutically acceptable acid;
n = 0, 1;
m = 0, 1, 2;
p = 1, 2, 3;
q = 2, 3, 4;
r = 0, 1;

and their pharmaceutically acceptable salts of acids or bases;
there being no more than one substituent $R^9$ in each molecule with general formula I,
as antioxidizing agents, in particular for the manufacture of a pharmaceutical composition with an anti-oxidizing or glutathione peroxidase activity, in particular for:

- treating pathologies in which an overproduction of cytotoxic hydroperoxides contributes to functional alterations in cells or tissues;
- treating pathologies with an inflammatory and/or ischemic component;
- vascular: such as treatment to prevent arterial re-stenosis following angioplastic intervention, treatment to prevent arterial stenosis following arterial allografts, treatment of intermittent claudication in patients with obstructive ischemia of the lower limbs; treatment of cerebro-vascular injuries of ischemic origin; preventative or curative treatment of adult (ARDS) or infant (IRDS) respiratory distress syndrome;
- articulatory: such as the treatment of rheumatoid polyarthritis;
- treating ophthalmic pathologies: such as treating acute ophthalmic allergies; treating retinal changes associated with macular degeneration; treating glaucoma;
- treating immune system dysfunctions such as treating acquired immunodeficiency syndrome (AIDS);
- protective treatment against intoxication by xenobiotic substances generating free radicals, in particular during cancer chemotherapy;
- storing organs for transplanting such as the heart, liver, kidney and lung; by adding one of the compounds of the present invention to storage media for these organs.

**3.** Use according to claim 2, characterised in that the benzisoselen-azoline or -azine derivative of aforesaid general formula (I) is present in a quantity in the range 0.1% to 5% by weight with respect to the total final composition weight, preferably in the range 0.1% to 1% by weight; or in the form of a unit dose comprising 1 milligram (mg) to 500 mg of the derivative, optionally in a pharmaceutically acceptable excipient, vehicle or support.

**4.** A pharmaceutical composition, in particular with antioxidizing or glutathione peroxidase activity, characterised in that it comprises, as an active ingredient, at least one benzisoselen-azoline or-azine compound having the general formula (I) defined in claim 2, optionally in a pharmaceutically acceptable excipient, support or vehicle.

**5.** A pharmaceutical composition according to claim 4, characterised in that the benzisoselen-azoline or -azine derivative with aforesaid general formula (I) is present in a quantity in the range 0.1% to 5% by weight with respect to the total final composition weight, preferably in the range 0.1% to 1% by weight; or in the form of a unit dose comprising 1 milligram (mg) to 500 mg of the derivative, optionally in a pharmaceutically acceptable excipient, vehicle or support.

**6.** A pharmaceutical composition according to claim 4 or 5, characterised in that it is a pharmaceutical composition for the treatment of any physiopathological condition in which an overproduction of cytotoxic hydroperoxides contributes to functional changes in cells or tissues, for example as the consequence of the activation of metabolic routes such as flavine or cytochrome P-450 oxygenases, monoamine oxidases, xanthine oxidase or 15-lipoxygenase of endothelial cells, cyclooxygenase or 12-lipoxygenase in blood platelets, NADPH oxidase or 5-lipoxygenase in neutrophiles, macrophages or lymphocytes, or intoxication by a xenobiotic substance such as anthracyclines, nitro-imidazoles or dipyridinium type derivatives which generate free radicals.

**7.** A pharmaceutical composition according to one of claims 4 to 6, characterised in that it is a pharmaceutical composition for the treatment of pathologies with an inflammatory and/or ischemic component on the vascular or articular level; for the treatment of ophthalmic pathologies, such as the treatment of acute ophthalmic allergies, retinal changes associated with macular degeneration, glaucoma; the treatment of immune system dysfunctions such as acquired immunodeficiency syndrome (AIDS); protective treatment against intoxication by xenobiotic free radical generators, in particular during cancer chemotherapy; preservation of organ transplant grafts such as the heart, liver, kidney and lung, by the addition of one of the compounds of aforesaid formula (I) to the preservation medium for these organs.

**8.** A method for the production of compounds having the general formula (II) defined in claim 1 characterised in that it comprises the following main steps:
a) preparing or using an orthohalogenophenyl-acetonitrile derivative gem-disubstituted in the 2 position,
then depending on the series considered:
either

b1) hydrolysing the nitrile derivative to an amide derivative,
c1) transforming the amide derivative to an amine derivative by a transposition reaction using normal methods,
d1) reacting the amine compound with a nucleophilic selenium compound which may be generated in situ, in the presence of a copper salt, in a polar organic solvent, to produce the corresponding benzisoselenazoline derivative,
e1) N-alkylating or N-acylating the latter using normal procedures,
f1) finally, oxidizing the derivative obtained at the selenium atom if necessary, using normal procedures; or
b2) reducing the nitrile derivative to an amine derivative using, for example, borane in an ethereal solvent such as tetrahydrofuran,
c2) reacting the amine compound with a nucleophilic selenium derivative, which may be generated in situ, in the presence of a copper salt, in a polar organic solvent, to produce the corresponding benzisoselenazine derivative,
d2) N-alkylating or N-acylating the latter using normal procedures,
e2) finally, oxidizing the derivative obtained at the selenium atom if necessary, using normal procedures.

**9.** A method according to claim 8, characterised in that the nucleophilic selenium compound is preferably a selenocyanate salt such as potassium selenocyanate which can be:

- either generated in situ from selenium metal (Se°) and a cyanide salt such as potassium cyanide,
- or added to the reaction mixture as it is.

**10.** A method according to claim 8 or claim 9, characterised in that the copper salt may be a cuprous ($Cu^I$) salt, such as cuprous iodide.

**11.** A method according to one of claims 8 to 10, characterised in that the polar organic solvent is preferably dimethylformamide.

**12.** A method according to one of claims 8 to 11, characterised in that the oxidizing agent, which may be used to oxidize the selenium, may be a peracid such as metachloroperbenzoic acid, or hydrogen peroxide.

**13.** 3,3-dimethyl-benzisoselenazoline according to claim 1 and its pharmaceutically acceptable salts of acids or bases.

**14.** 4,4-dimethyl-benzisoselenazine according to claim 1 and its pharmaceutically acceptable salts of acids or bases.

**15.** 4,4-dimethyl-6-methoxy-benzisoselenazine according to claim 1 and its pharmaceutically acceptable addition salts

of acids or bases.

**16.** Benzisoselen-azoline or -azine compound according to claim 1 selected from the group consisting of 2-acetyl-3,3-dimethyl-benzisoselenazoline, 3,3-dimethyl-2-ethyl-benzisoselenazoline, 3,3-dimethylbenziso-selenazoline-1-oxide, 3,3'-dimethyl-7-nitrobenziso-selenazoline, 3,3'-dimethyl-5-nitrobenzisoselenazo-line, 3,3'-dimethyl-7-fluorobenzisoselenazoline, 4,4-dimethyl-benzisoselenazine-1-oxide, 4,4-dimethyl-2-ethyl-benzisoselenazine, and 4,4-dimethyl-6-(2'-(4"-methylpiperazine-1"-yl)ethoxybenzisoselenazine.

**17.** Pharmaceutical composition, characterised in that it comprises as active ingredient 4,4-dimethylbenzisoselenazine according to claim 1 or a pharmaceutically acceptable salt of acid or base thereof, in a pharmaceutically acceptable excipient.

**18.** Pharmaceutical composition, characterised in that it comprises as active ingredient 3,3-dimethylbenzisoselenazoline according to claim 1 or a pharmaceutically acceptable salt of acid or base thereof, in a pharmaceutically acceptable excipient.

**19.** Pharmaceutical composition, characterised in that it comprises as active ingredient 4,4-dimethyl-6-methoxy-benzisoselenazine according to claim 1 or a pharmaceutically acceptable salt of acid or base thereof, in a pharmaceutically acceptable excipient.

**20.** Pharmaceutical composition, characterised in that it comprises as active ingredient at least one benzisoselenazoline or -azine according to claim 1 selected from the group consisting of 2-acetyl-3,3-dimethyl-benzisoselenazoline, 3,3-dimethyl-2-ethyl-benzisoselenazoline, 3,3-dimethyl-benzisoselenazoline-1-oxide, 3,3'-dimethyl-7-fluorobenzisoselenazoline, 4,4-dimethyl-benzisoselenazine-1-oxide, 4,4-dimethyl-2-ethyl-benzisoselenazine, and 4,4-dimethyl-6-(2'-(4"-methylpiperazine-1"-yl)ethoxybenzisoselenazine or a pharmaceutically acceptable salt of acid or base thereof, in a pharmaceutically acceptable excipient.

**21.** Pharmaceutical composition according to one of claims 17 to 20, characterised in that the active ingredient is present in a quantity in the range 0.1% to 5% by weight of the pharmaceutical composition.

**22.** Pharmaceutical composition according to one of claims 17 to 21, characterised in that it is in the form of a unit dose comprising 1 mg to 500 mg of said active ingredient.

**23.** Use of a compound as defined in any one of claims 14 to 16 as antioxidant in particular for the production of a pharmaceutical composition having a glutathione peroxidase antioxidizing activity in particular to carry out a treatment as defined in claim 2, 6 or 7, and preferably to treat rheumatoid polyarthritis.

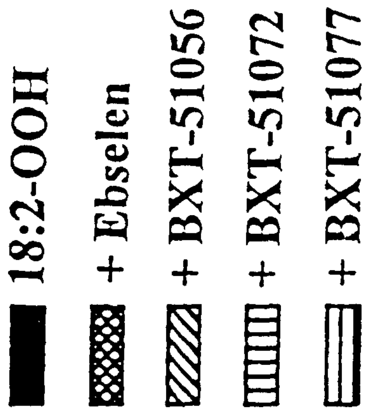
18:2-OOH
+ Ebselen
+ BXT-51056
+ BXT-51072
+ BXT-51077

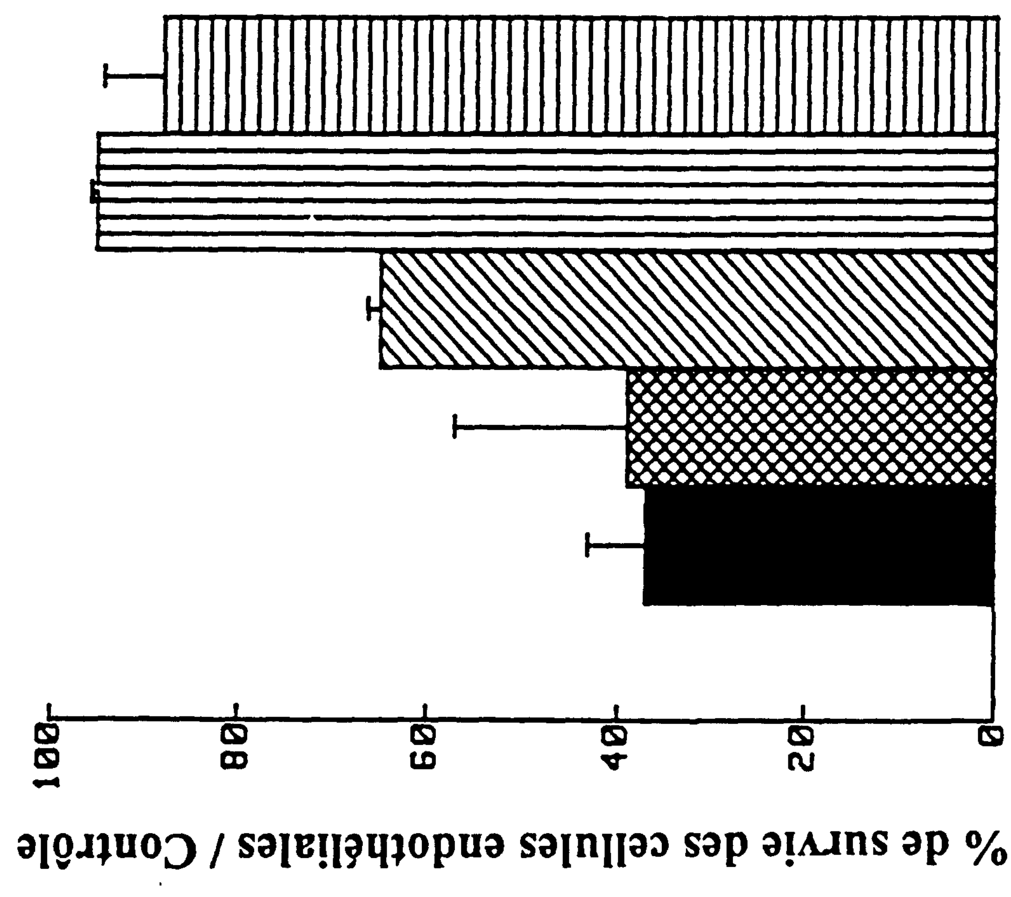
% de survie des cellules endothéliales / Contrôle
0
20
40
60
80
100

FIG.1

FIG.2

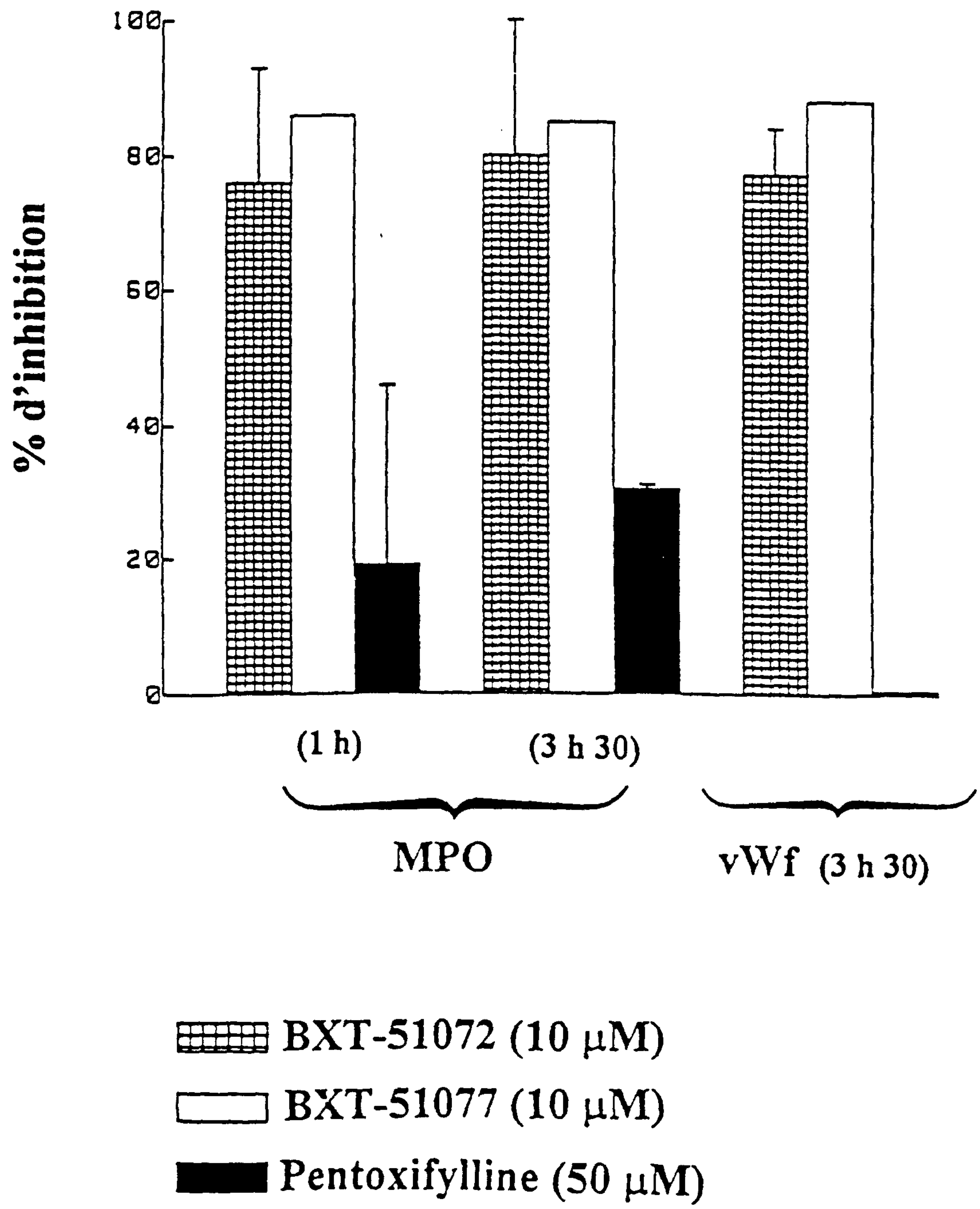
100
80
60
40
20
0
% d'inhibition
(1 h)
(3 h 30)
MPO
vWf (3 h 30)
BXT-51072 (10 µM)
BXT-51077 (10 µM)
Pentoxifylline (50 µM)

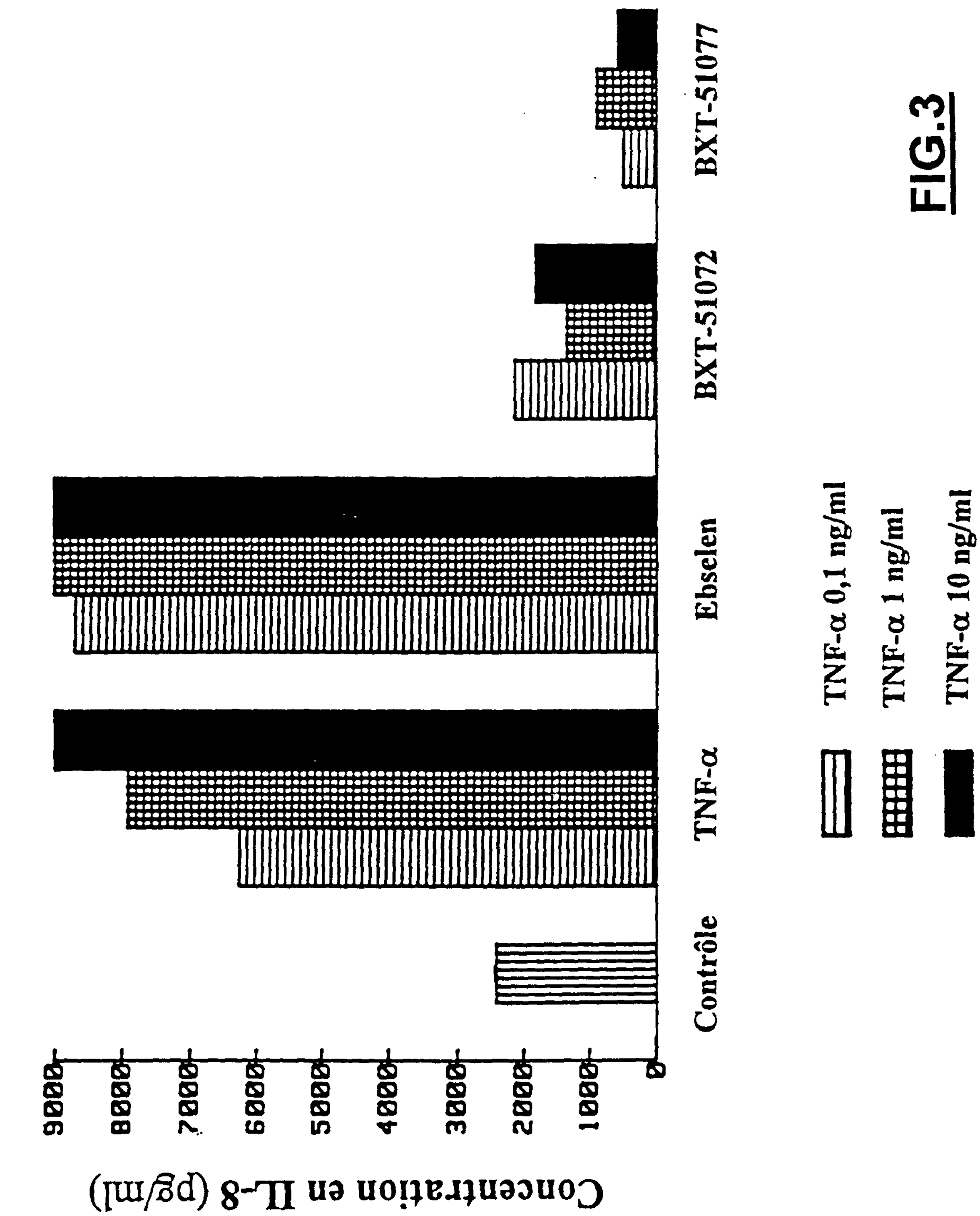
Concentration en IL-8 (pg/ml)
9000
8000
7000
6000
5000
4000
3000
2000
1000
0
Contrôle
TNF-α
Ebselen
BXT-51072
BXT-51077
TNF-α 0,1 ng/ml
TNF-α 1 ng/ml
TNF-α 10 ng/ml

FIG.3

# FIG.4

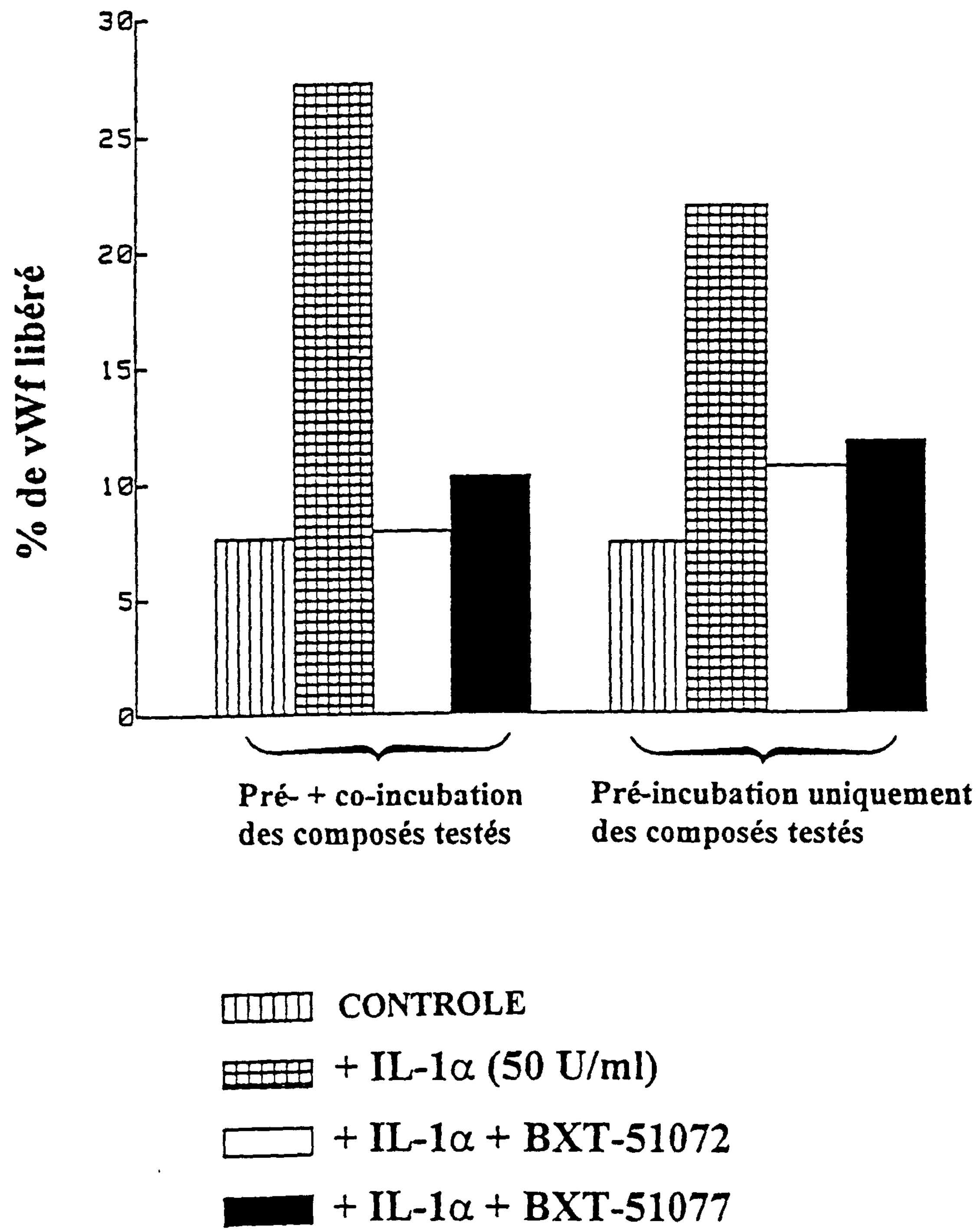
% de vWf libéré
30
25
20
15
10
5
0
Pré- + co-incubation des composés testés
Pré-incubation uniquement des composés testés
CONTROLE
+ IL-1α (50 U/ml)
+ IL-1α + BXT-51072
+ IL-1α + BXT-51077

FIG.5

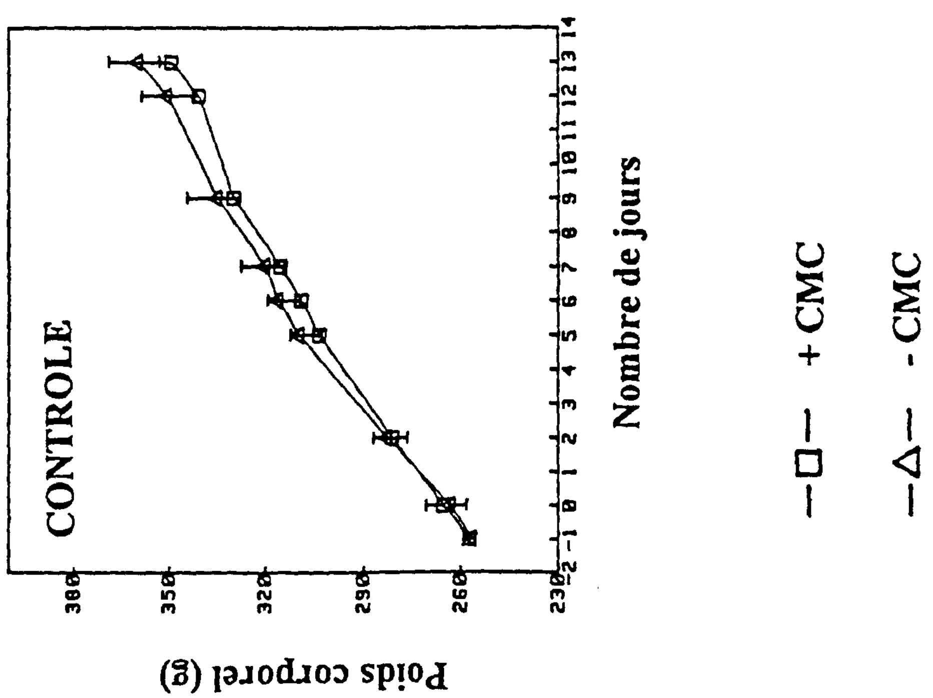
CONTROLE
Poids corporel (g)
380
350
320
290
260
230
-2 -1 0 1 2 3 4 5 6 7 8 9 10 11 12 13 14
Nombre de jours
+ CMC
- CMC

FIG.6

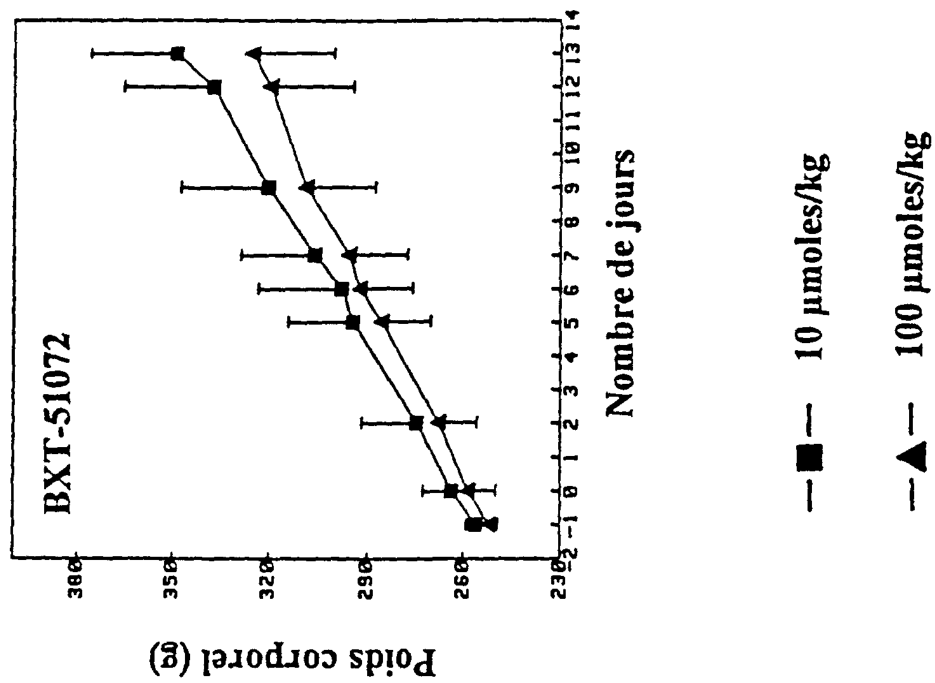
BXT-51072
Poids corporel (g)
380
350
320
290
260
230
-2 -1 0 1 2 3 4 5 6 7 8 9 10 11 12 13 14
Nombre de jours
10 µmoles/kg
100 µmoles/kg